# EUROPEAN PATENT APPLICATION

(11) **EP 4 074 732 A1**
(43) Date of publication of application: **19.10.2022**
(21) Application number: 22174660.5
(22) Date of filing: 07.05.2019
(51) Int. Cl.: C07K 16/28, A61K 39/395, A61P 35/00, A61P 37/00, A61P 31/00

(54) **FULLY HUMAN ANTIBODIES AGAINST OX40, METHOD FOR PREPARING THE SAME, AND USE THEREOF**

(30) Priority: 11.05.2018 WO PCT/CN2018/086574; 29.05.2018 CN 201810529840
(62) Divisional of application: 19799456.9
(71) Applicant: Wuxi Biologics (Shanghai) Co. Ltd., Shanghai 200131 (CN); Wuxi Biologics Ireland Limited, Dublin 1 (IE)
(72) Inventor: ZHENG, Yong, Shanghai, 200131 (CN); YANG, Baotian, Shanghai, 200131 (CN); LI, Jing, Shanghai, 200131 (CN)
(74) Representative: Arnold & Siedsma

(57) **Abstract**

The present application provides fully human monoclonal antibodies against tumor necrosis factor receptor superfamily, member 4 (TNFRSF4), also known as OX40 and CD134. It also provides the methods of hybridoma generation using a humanized transgenic rat, the nucleic acid molecules encoding the anti-OX40 antibodies, expression vectors and host cells used for the expression of anti-OX40 antibodies. The invention further provides the methods for validating the function of antibodies *in vitro* and the efficacy of antibodies *in vivo.* The antibodies of invention provide a very potent agent for the treatment of multiple cancers vial modulating human immune function.

## Description

### FIELD OF THE INVENTION

This application generally relates to antibodies. More specifically, the application relates to fully human monoclonal antibodies against OX40, a method for preparing the same, and the use thereof.

### BACKGROUND OF THE INVENTION

Increasing evidences from preclinical and clinical results have shown that targeting immune checkpoints is becoming the most promising approach to treat patients with cancers. Tumor necrosis factor receptor superfamily, member 4 (TNFRSF4, also known as OX40, CD134 and ACT35), one of the immune-checkpoint proteins, plays a major role in T cell function by potentiating T cell receptor signaling and leading to their activation.

OX40 is primarily expressed by activated CD4⁺ and CD8⁺ T cells, memory T cells, regulatory T (Treg) cells and nature killer (NK) cells. The interaction of OX40 expressed on activated T cells, and its ligand (OX40L) expressed on antigen presenting cells dramatically promotes T cell activation, proliferation and migration, increases survival of effector T cells, enhances the germinal center formation and dendritic cells maturation. In addition, OX40 signaling can inhibit differentiation and expansion of Tregs, antagonize generation of inducible Tregs and block Treg-suppressive function. It has been proved in a variety of preclinical mouse tumor models and clinical trials that an agonist of OX40 is quite a promising strategy for treating cancer and infectious diseases. Multiple agonistic agents targeting OX40 have been developed by pharmaceutical companies, such as Medlmmune, GlaxoSmithKline (GSK), Pfizer and Incyte. An agonistic murine antibody targeting OX40 (9B12, AgonOX), developed by Medlmmune, was used in Phase I clinical trial in patients with advanced cancer. Patients treated with one course of the antibody "9B12" showed an acceptable toxicity profile and regression of at least one metastatic lesion in 12 of 30 patients. Mechanistically, this treatment increased T and B cell response to reporter antigen immunizations (e.g. KLH), led to preferential up-regulation of OX40 on CD4+FoxP3+ Treg cells in tumor-infiltrating lymphocytes and increased the anti-tumor reactivity of T and B cells in patients with melanoma. GSK is also developing GSK-3174998, **a** humanized IgG1 monoclonal antibody that activates OX-40 on the surface of T cells, identified through a collaboration with MD Anderson Cancer Center, for the potential treatment of cancer including solid tumors and hematological malignancies. Other agents in clinical development that target OX40 include Pfizer's fully human IgG2 agonist antibody PF-04518600, which is currently in clinical development in a broad spectrum of malignancies; and Incyte's INCAGN-1949, which is an anti-OX40 human IgG1 antibody with optimal agonistic profile and the ability of selectively deplete intratumoral regulatory T cells, for the potential treatment of cancer.

WO 03106498A2 discloses agonistic binding molecules which bind to and stimulate human OX40 receptor. WO 2014148895A1 discloses humanized anti-CD134(OX40) antibodies and use thereof. WO 2017063162A1 discloses an anti-OX40 antibody and application thereof. WO 2016179517A1 discloses anti-OX40 antibodies and methods of use thereof. WO 2016185016A1 discloses bispecific polypeptides, such as bispecific antibodies, comprising a first binding domain capable of specifically binding to a first T cell target and a second binding domain capable of specifically binding to a second T cell target, wherein the first or second T cell target may be OX40. US 20180044427A1 discloses engineered anti-tumor necrosis factor receptor (TNFR) superfamily member antibodies and other Fc-containing molecules with enhanced agonism and effector functions.

There are some spaces for improvement for antibody against OX40 as a therapeutic agent. As an agonist against co-stimulatory receptors, toxicity may be the most concerned questions, such as cytokine storm, which limits the clinical applications. Moreover, the anti-OX40 antibodies currently tested in clinical trials are human-mouse chimeric or humanized antibodies, high immunogenicity diminishes efficacy owing to the mouse-derived protein sequences. Fully human antibody overcomes these shortages and showed higher efficiency and lower toxicity in vivo.

In this invention, we have generated fully human antibodies against OX40 utilizing our proprietary hybridoma technology. The antibodies of this invention have high binding affinity; specifically bind to both human and monkey OX40 protein; and potent modulating immune responses, including enhancing T cell proliferation and increasing cytokine IFN-γ and interleukin-2 production and impairing the suppressive function of Treg cells.

### SUMMARY OF THE INVENTION

These and other objectives are provided for by the present invention which, in a broad sense, is directed to compounds, methods, compositions and articles of manufacture that provide antibodies with improved efficacy. The benefits provided by the present invention are broadly applicable in the field of antibody therapeutics and diagnostics and may be used in conjunction with antibodies that react with a variety of targets. The present invention provides antibodies, preferably fully human monoclonal antibodies, that bind to human OX40. It also provides methods of hybridoma generation using humanized rats, nucleic acid molecules encoding the anti-OX40 antibodies, vectors and host cells used for the expression of anti-OX40 antibodies. The invention further provides the methods for validating the function of antibodies *in vitro* and *in vivo.* The antibodies of the invention provide a potent agent for the treatment of multiple diseases comprising cancer via modulating human immune function.

In some aspects, the invention comprises an isolated antibody, or an antigen-binding portion thereof.

In some embodiments, the isolated antibody or the antigen-binding portion thereof has one or more of the following properties:
(a) binding human OX40 with a K_{D} of 1 × 10⁻⁸ M or less;
(b) inducing production of a cytokine (e.g., IL-2 or IFN-γ) in CD4⁺T cells;
(c) enhancing proliferation of primary human CD4⁺ T cells;
(d) enhancing proliferation of primary human CD4⁺ T effector cells in the presence of Treg cells;
(e) binding human or rhesus monkey OX40 respectively; or
(f) having no cross-reactivity to human CD40, CD137 and CD271.

In some embodiments, the isolated antibody or the antigen-binding portion thereof binds to CRD2 and/or CRD3 domain of OX40.

In some embodiments, the isolated antibody or the antigen-binding portion thereof comprises:
A) one or more heavy chain CDRs (CDRHs) selected from at least one of the group consisting of:
   (i) a CDRH1 with at least 90% sequence identity to a CDRH1 as set forth in one of the sequences selected from the group consisting of SEQ ID NOs: 1, 7, 13, 15, 21 and 27;
   (ii) a CDRH2 with at least 90% sequence identity to a CDRH2 as set forth in one of the sequences selected from the group consisting of SEQ ID NOs: 3, 9, 17, 23 and 29; and
   (iii) a CDRH3 with at least 90%, sequence identity to a CDRH3 as set forth in one of the sequences selected from the group consisting of SEQ ID NOs: 5, 11, 19, 25 and 31;
B) one or more light chain CDRs (CDRLs) selected from at least one of the group consisting of:
   (i) a CDRL1 with at least 90% sequence identity to a CDRL1 as set forth in one of the sequences selected from the group consisting of SEQ ID NOs: 2, 8, 14, 16, 22 and 28;
   (ii) a CDRL2 with at least 90% sequence identity to a CDRL2 as set forth in one of the sequences selected from the group consisting of SEQ ID NOs: 4, 10, 18, 24 and 30; and
   (iii) a CDRL3 with at least 90% sequence identity to a CDRL3 as set forth in one of the sequences selected from the group consisting of SEQ ID NOs: 6, 12, 20, 26 and 32; or
C) one or more CDRHs of A) and one or more CDRLs of B).

In some embodiments, the isolated antibody or the antigen-binding portion thereof comprises:
A) one or more heavy chain CDRs (CDRHs) selected from at least one of the group consisting of:
   (i) a CDRH1 selected from the group consisting of SEQ ID NOs: 1, 7, 13, 15, 21 and 27, or a CDRH1 that differs in amino acid sequence from the CDRH1 by an amino acid addition, deletion or substitution of not more than 2 amino acids;
   (ii) a CDRH2 selected from the group consisting of SEQ ID NOs: 3, 9, 17, 23 and 29, or a CDRH2 that differs in amino acid sequence from the CDRH2 by an amino acid addition, deletion or substitution of not more than 2 amino acids; and
   (iii) a CDRH3 selected from the group consisting of SEQ ID NOs: 5, 11, 19, 25 and 31, or a CDRH3 that differs in amino acid sequence from the CDRH3 by an amino acid addition, deletion or substitution of not more than 2 amino acids;
B) one or more light chain CDRs (CDRLs) selected from at least one of the group consisting of:
   (i) a CDRL1 selected from the group consisting of SEQ ID NOs: 2, 8, 14, 16, 22 and 28, or a CDRL1 that differs in amino acid sequence from the CDRL1 by an amino acid addition, deletion or substitution of not more than 2 amino acids;
   (ii) a CDRL2 selected from the group consisting of SEQ ID NOs: 4, 10, 18, 24 and 30, or a CDRL2 that differs in amino acid sequence from the CDRL2 by an amino acid addition, deletion or substitution of not more than 2 amino acids; and
   (iii) a CDRL3 selected from the group consisting of SEQ ID NOs: 6, 12, 20, 26 and 32, or a CDRL3 that differs in amino acid sequence from the CDRL3 by an amino acid addition, deletion or substitution of not more than 2 amino acids; or
C) one or more CDRHs of A) and one or more CDRLs of B).

In some embodiments, the isolated antibody or the antigen-binding portion thereof comprises:
A) a CDRH3 comprising SEQ ID NO: 5, 11, 19, 25 or 31; or
B) a CDRH3 with at least 90% sequence identity to a CDRH3 as set forth in one of the sequences selected from the group consisting of SEQ ID NOs: 5, 11, 19, 25 and 31; or
C) a CDRH3 that differs in amino acid sequence from the CDRH3 of (A) by an amino acid addition, deletion or substitution of not more than 2 amino acids,
and wherein the isolated antibody or the antigen-binding portion thereof binds human OX40 with a K_{D} of 1 × 10⁻⁸ M or less.

In some embodiments, the isolated antibody or the antigen-binding portion thereof comprises:
(a) a CDRH1 comprising or consisting of SEQ ID NO: 1;
(b) a CDRH2 comprising or consisting of SEQ ID NO: 3;
(c) a CDRH3 comprising or consisting of SEQ ID NO: 5;
(d) a CDRL1 comprising or consisting of SEQ ID NO: 2;
(e) a CDRL2 comprising or consisting of SEQ ID NO: 4; and
(f) a CDRL3 comprising or consisting of SEQ ID NO: 6.

In some embodiments, the isolated antibody or the antigen-binding portion thereof comprises:
(a) a CDRH1 comprising or consisting of SEQ ID NO: 7;
(b) a CDRH2 comprising or consisting of SEQ ID NO: 9;
(c) a CDRH3 comprising or consisting of SEQ ID NO: 11;
(d) a CDRL1 comprising or consisting of SEQ ID NO: 8;
(e) a CDRL2 comprising or consisting of SEQ ID NO: 10; and
(f) a CDRL3 comprising or consisting of SEQ ID NO: 12.

In some embodiments, the isolated antibody or the antigen-binding portion thereof comprises:
(a) a CDRH1 comprising or consisting of SEQ ID NO: 13;
(b) a CDRH2 comprising or consisting of SEQ ID NO: 9;
(c) a CDRH3 comprising or consisting of SEQ ID NO: 11;
(d) a CDRL1 comprising or consisting of SEQ ID NO: 14;
(e) a CDRL2 comprising or consisting of SEQ ID NO: 10; and
(f) a CDRL3 comprising or consisting of SEQ ID NO: 12.

In some embodiments, the isolated antibody or the antigen-binding portion thereof comprises:
(a) a CDRH1 comprising or consisting of SEQ ID NO: 15;
(b) a CDRH2 comprising or consisting of SEQ ID NO: 17;
(c) a CDRH3 comprising or consisting of SEQ ID NO: 19;
(d) a CDRL1 comprising or consisting of SEQ ID NO: 16;
(e) a CDRL2 comprising or consisting of SEQ ID NO: 18; and
(f) a CDRL3 comprising or consisting of SEQ ID NO: 20.

In some embodiments, the isolated antibody or the antigen-binding portion thereof comprises:
(a) a CDRH1 comprising or consisting of SEQ ID NO: 21;
(b) a CDRH2 comprising or consisting of SEQ ID NO: 23;
(c) a CDRH3 comprising or consisting of SEQ ID NO: 25;
(d) a CDRL1 comprising or consisting of SEQ ID NO: 22;
(e) a CDRL2 comprising or consisting of SEQ ID NO: 24; and
(f) a CDRL3 comprising or consisting of SEQ ID NO: 26.

In some embodiments, the isolated antibody or the antigen-binding portion thereof comprises:
(a) a CDRH1 comprising or consisting of SEQ ID NO: 27;
(b) a CDRH2 comprising or consisting of SEQ ID NO: 29;
(c) a CDRH3 comprising or consisting of SEQ ID NO: 31;
(d) a CDRL1 comprising or consisting of SEQ ID NO: 28;
(e) a CDRL2 comprising or consisting of SEQ ID NO: 30; and
(f) a CDRL3 comprising or consisting of SEQ ID NO: 32.

In some embodiments, the isolated antibody or the antigen-binding portion thereof comprises:
(A) a heavy chain variable region (VH):
   (i) comprising the amino acid sequence selected from the group consisting of SEQ ID NO: 33, 35, 37, 39, 41 and 43;
   (ii) comprising an amino acid sequence at least 85%, 90%, or 95% identical to the amino acid sequence selected from the group consisting of SEQ ID NO: 33, 35, 37, 39, 41 and 43; or
   (iii) comprising an amino acid sequence with addition, deletion and/or substitution of one or more (such as 1-10, 1-5, 1-3, 1, 2, 3, 4, or 5) amino acids compared with the amino acid sequence selected from the group consisting of SEQ ID NO: 33, 35, 37, 39, 41 and 43; and/or
(B) a light chain variable region (VL):
   (i) comprising the amino acid sequence selected from the group consisting of SEQ ID NO: 34, 36, 38, 40, 42 and 44;
   (ii) comprising an amino acid sequence at least 85%, at least 90%, or at least 95% identical to the amino acid sequence selected from the group consisting of SEQ ID NO: 34, 36, 38, 40, 42 and 44; or
   (iii) comprising an amino acid sequence with addition, deletion and/or substitution of one or more (such as 1-10, 1-5, 1-3, 1, 2, 3, 4, or 5) amino acids compared with the amino acid sequence selected from the group consisting of SEQ ID NO: 34, 36, 38, 40, 42 and 44.

In some embodiments, the invention comprises an isolated antibody or the antigen-binding portion thereof which competes binding for the same epitope with the isolated antibody or the antigen-binding portion thereof as defined above.

In some aspects, the invention is directed to an isolated nucleic acid molecule, comprising a nucleic acid sequence encoding the heavy chain variable region and/or the light chain variable region of the isolated antibody as disclosed herein.

In some aspects, the invention is directed to a vector comprising the nucleic acid molecule encoding the antibody or antigen-binding portion thereof as disclosed herein.

In some aspects, the invention is directed to a host cell comprising the expression vector as disclosed herein.

In some aspects, the invention is directed to a pharmaceutical composition comprising at least one antibody or antigen-binding portion thereof as disclosed herein and a pharmaceutically acceptable carrier.

In some aspects, the invention is directed to a method for preparing an anti-OX40 antibody or antigen-binding portion thereof which comprises expressing the antibody or antigen-binding portion thereof in the host cell and isolating the antibody or antigen-binding portion thereof from the host cell.

In some aspects, the invention is directed to a method of modulating an immune response in a subject, comprising administering the antibody or antigen-binding portion thereof as disclosed herein to the subject such that an immune response in the subject is modulated.

In some aspects, the invention is directed to a method for treating abnormal cell growth in a subject, comprising administering an effective amount of the antibody or antigen-binding portion thereof or the pharmaceutical composition as disclosed herein to the subject.

In some aspects, the invention is directed to a method for inhibiting growth of tumor cells in a subject, comprising administering an effective amount of the antibody or antigen-binding portion thereof or the pharmaceutical composition as disclosed herein to the subject.

In some aspects, the invention is directed to a method for reducing tumor cell metastasis in a subject, comprising administering an effective amount of the antibody or antigen-binding portion thereof or the pharmaceutical composition as disclosed herein to the subject.

In some aspects, the invention is directed to a method for impairing the suppressive function of Treg cells in a subject, comprising administering an effective amount of the antibody or antigen-binding portion thereof or the pharmaceutical composition as disclosed herein to the subject.

In some aspects, the invention is directed to a method for treating or preventing diseases comprising proliferative disorders (such as cancers), autoimmune diseases, inflammatory disease or infectious diseases in a subject comprising administering an effective amount of the antibody or antigen-binding portion thereof or the pharmaceutical composition as disclosed herein to the subject.

In some aspects, the invention is directed to the use of the antibody or antigen-binding portion thereof as disclosed herein in the manufacture of a medicament for treating or preventing diseases comprising proliferative disorders (such as cancers), autoimmune diseases, inflammatory disease or infectious diseases.

In some aspects, the invention is directed to the use of the antibody or antigen-binding portion thereof as disclosed herein in the manufacture of a diagnostic agent for diagnosing proliferative diseases comprising proliferative disorders (such as cancers), autoimmune diseases, inflammatory disease or infectious diseases.

In some aspects, the invention is directed to the antibody or antigen-binding portion thereof as disclosed herein for use in treating or preventing diseases comprising proliferative disorders (such as cancers), autoimmune diseases, inflammatory disease or infectious diseases.

In some aspects, the invention is directed to kits or devices and associated methods that employ the antibody or antigen-binding portion thereof as disclosed herein, and pharmaceutical compositions as disclosed herein, which are useful for the treatment of diseases comprising proliferative disorders (such as cancers), autoimmune diseases, inflammatory disease or infectious diseases. To this end the present invention preferably provides an article of manufacture useful for treating such disorders comprising a receptacle containing the antibody or antigen-binding portion thereof as disclosed herein and instructional materials for using the antibody or antigen-binding portion thereof as disclosed herein to treat, ameliorate or prevent a proliferative disorder or progression or recurrence thereof.

The foregoing is a summary and thus contains, by necessity, simplifications, generalizations, and omissions of detail; consequently, those skilled in the art will appreciate that the summary is illustrative only and is not intended to be in any way limiting. Other aspects, features, and advantages of the methods, compositions and/or devices and/or other subject matter described herein will become apparent in the teachings set forth herein. The summary is provided to introduce a selection of concepts in a simplified form that are further described below in the Detailed Description. This summary is not intended to identify key features or essential features of the claimed subject matter, nor is it intended to be used as an aid in determining the scope of the claimed subject matter.

Embodiments shall be further described by the following clauses.
1. An isolated antibody or the antigen-binding portion thereof, wherein the isolated antibody or the antigen-binding portion thereof comprises:
   A) one or more heavy chain CDRs (CDRHs) selected from at least one of the group consisting of:
      (i) a CDRH1 with at least 90% sequence identity to a CDRH1 as set forth in one of the sequences selected from the group consisting of SEQ ID NOs: 1, 7, 13, 15, 21 and 27;
      (ii) a CDRH2 with at least 90% sequence identity to a CDRH2 as set forth in one of the sequences selected from the group consisting of SEQ ID NOs: 3, 9, 17, 23 and 29; and
      (iii) a CDRH3 with at least 90%, sequence identity to a CDRH3 as set forth in one of the sequences selected from the group consisting of SEQ ID NOs: 5, 11, 19, 25 and 31;
   B) one or more light chain CDRs (CDRLs) selected from at least one of the group consisting of:
      (i) a CDRL1 with at least 90% sequence identity to a CDRL1 as set forth in one of the sequences selected from the group consisting of SEQ ID NOs: 2, 8, 14, 16, 22 and 28;
      (ii) a CDRL2 with at least 90% sequence identity to a CDRL2 as set forth in one of the sequences selected from the group consisting of SEQ ID NOs: 4, 10, 18, 24 and 30; and
      (iii) a CDRL3 with at least 90% sequence identity to a CDRL3 as set forth in one of the sequences selected from the group consisting of SEQ ID NOs: 6, 12, 20, 26 and 32; or
   C) one or more CDRHs of A) and one or more CDRLs of B).
2. The isolated antibody or the antigen-binding portion thereof of clause 1, wherein the isolated antibody or the antigen-binding portion thereof comprises:
   A) one or more heavy chain CDRs (CDRHs) selected from at least one of the group consisting of:
      (i) a CDRH1 selected from the group consisting of SEQ ID NOs: 1, 7, 13, 15, 21 and 27, or a CDRH1 that differs in amino acid sequence from the CDRH1 by an amino acid addition, deletion or substitution of not more than 2 amino acids;
      (ii) a CDRH2 selected from the group consisting of SEQ ID NOs: 3, 9, 17, 23 and 29, or a CDRH2 that differs in amino acid sequence from the CDRH2 by an amino acid addition, deletion or substitution of not more than 2 amino acids; and
      (iii) a CDRH3 selected from the group consisting of SEQ ID NOs: 5, 11, 19, 25 and 31, or a CDRH3 that differs in amino acid sequence from the CDRH3 by an amino acid addition, deletion or substitution of not more than 2 amino acids;
   B) one or more light chain CDRs (CDRLs) selected from at least one of the group consisting of:
      (i) a CDRL1 selected from the group consisting of SEQ ID NOs: 2, 8, 14, 16, 22 and 28, or a CDRL1 that differs in amino acid sequence from the CDRL1 by an amino acid addition, deletion or substitution of not more than 2 amino acids;
      (ii) a CDRL2 selected from the group consisting of SEQ ID NOs: 4, 10, 18, 24 and 30, or a CDRL2 that differs in amino acid sequence from the CDRL2 by an amino acid addition, deletion or substitution of not more than 2 amino acids; and
      (iii) a CDRL3 selected from the group consisting of SEQ ID NOs: 6, 12, 20, 26 and 32, or a CDRL3 that differs in amino acid sequence from the CDRL3 by an amino acid addition, deletion or substitution of not more than 2 amino acids; or
   C) one or more CDRHs of A) and one or more CDRLs of B).
3. The isolated antibody or the antigen-binding portion thereof of clause 1 or 2, wherein the isolated antibody or the antigen-binding portion thereof comprises:
   (a) a CDRH1 comprising or consisting of SEQ ID NO: 1;
   (b) a CDRH2 comprising or consisting of SEQ ID NO: 3;
   (c) a CDRH3 comprising or consisting of SEQ ID NO: 5;
   (d) a CDRL1 comprising or consisting of SEQ ID NO: 2;
   (e) a CDRL2 comprising or consisting of SEQ ID NO: 4; and
   (f) a CDRL3 comprising or consisting of SEQ ID NO: 6.
4. The isolated antibody or the antigen-binding portion thereof of clause 1 or 2, wherein the isolated antibody or the antigen-binding portion thereof comprises:
   (a) a CDRH1 comprising or consisting of SEQ ID NO: 7;
   (b) a CDRH2 comprising or consisting of SEQ ID NO: 9;
   (c) a CDRH3 comprising or consisting of SEQ ID NO: 11;
   (d) a CDRL1 comprising or consisting of SEQ ID NO: 8;
   (e) a CDRL2 comprising or consisting of SEQ ID NO: 10; and
   (f) a CDRL3 comprising or consisting of SEQ ID NO: 12.
5. The isolated antibody or the antigen-binding portion thereof of clause 1 or 2, wherein the isolated antibody or the antigen-binding portion thereof comprises:
   (a) a CDRH1 comprising or consisting of SEQ ID NO: 13;
   (b) a CDRH2 comprising or consisting of SEQ ID NO: 9;
   (c) a CDRH3 comprising or consisting of SEQ ID NO: 11;
   (d) a CDRL1 comprising or consisting of SEQ ID NO: 14;
   (e) a CDRL2 comprising or consisting of SEQ ID NO: 10; and
   (f) a CDRL3 comprising or consisting of SEQ ID NO: 12.
6. The isolated antibody or the antigen-binding portion thereof of clause 1 or 2, wherein the isolated antibody or the antigen-binding portion thereof comprises:
   (a) a CDRH1 comprising or consisting of SEQ ID NO: 15;
   (b) a CDRH2 comprising or consisting of SEQ ID NO: 17;
   (c) a CDRH3 comprising or consisting of SEQ ID NO: 19;
   (d) a CDRL1 comprising or consisting of SEQ ID NO: 16;
   (e) a CDRL2 comprising or consisting of SEQ ID NO: 18; and
   (f) a CDRL3 comprising or consisting of SEQ ID NO: 20.
7. The isolated antibody or the antigen-binding portion thereof of clause 1 or 2, wherein the isolated antibody or the antigen-binding portion thereof comprises:
   (a) a CDRH1 comprising or consisting of SEQ ID NO: 21;
   (b) a CDRH2 comprising or consisting of SEQ ID NO: 23;
   (c) a CDRH3 comprising or consisting of SEQ ID NO: 25;
   (d) a CDRL1 comprising or consisting of SEQ ID NO: 22;
   (e) a CDRL2 comprising or consisting of SEQ ID NO: 24; and
   (f) a CDRL3 comprising or consisting of SEQ ID NO: 26.
8. The isolated antibody or the antigen-binding portion thereof of clause 1 or 2, wherein the isolated antibody or the antigen-binding portion thereof comprises:
   (a) a CDRH1 comprising or consisting of SEQ ID NO: 27;
   (b) a CDRH2 comprising or consisting of SEQ ID NO: 29;
   (c) a CDRH3 comprising or consisting of SEQ ID NO: 31;
   (d) a CDRL1 comprising or consisting of SEQ ID NO: 28;
   (e) a CDRL2 comprising or consisting of SEQ ID NO: 30; and
   (f) a CDRL3 comprising or consisting of SEQ ID NO: 32.
9. The isolated antibody or the antigen-binding portion thereof of clause 1 or 2, wherein the isolated antibody or the antigen-binding portion thereof comprises:
   (A) a heavy chain variable region:
      (i) comprising the amino acid sequence selected from the group consisting of SEQ ID NO: 33, 35, 37, 39, 41 and 43;
      (ii) comprising an amino acid sequence at least 85%, at least 90%, or at least 95% identical to the amino acid sequence selected from the group consisting of SEQ ID NO: 33, 35, 37, 39, 41 and 43; or
      (iii) comprising an amino acid sequence with addition, deletion and/or substitution of one or more amino acids compared with the amino acid sequence selected from the group consisting of SEQ ID NO: 33, 35, 37, 39, 41 and 43; and/or
   (B) a light chain variable region:
      (i) comprising the amino acid sequence selected from the group consisting of SEQ ID NO: 34, 36, 38, 40, 42 and 44;
      (ii) comprising an amino acid sequence at least 85%, at least 90%, or at least 95% identical to the amino acid sequence selected from the group consisting of SEQ ID NO: 34, 36, 38, 40, 42 and 44; or
      (iii) comprising an amino acid sequence with addition, deletion and/or substitution of one or more amino acids compared with the amino acid sequence selected from the group consisting of SEQ ID NO: 34, 36, 38, 40, 42 and 44.
10. The isolated antibody or the antigen-binding portion thereof of clause 9, wherein the isolated antibody or the antigen-binding portion thereof comprises:
   (a) a heavy chain variable region comprising the amino acid sequence of SEQ ID NO: 33 and a light chain variable region comprising the amino acid sequence of SEQ ID NO: 34; or
   (b) a heavy chain variable region comprising the amino acid sequence of SEQ ID NO: 35 and a light chain variable region comprising the amino acid sequence of SEQ ID NO: 36; or
   (c) a heavy chain variable region comprising the amino acid sequence of SEQ ID NO: 37 and a light chain variable region comprising the amino acid sequence of SEQ ID NO: 38; or
   (d) a heavy chain variable region comprising the amino acid sequence of SEQ ID NO: 39 and a light chain variable region comprising the amino acid sequence of SEQ ID NO: 40; or
   (e) a heavy chain variable region comprising the amino acid sequence of SEQ ID NO: 41 and a light chain variable region comprising the amino acid sequence of SEQ ID NO: 42; or
   (f) a heavy chain variable region comprising the amino acid sequence of SEQ ID NO: 43 and a light chain variable region comprising the amino acid sequence of SEQ ID NO: 44.
11. The isolated antibody or the antigen-binding portion thereof of any of the preceding clauses, having one or more of the following properties:
   (a) binding human OX40 with a K_{D} of 1 × 10⁻⁸ M or less;
   (b) inducing production of a cytokine (e.g., IL-2 or IFN-γ) in CD4⁺T cells;
   (c) enhancing proliferation of primary human CD4⁺ T cells;
   (d) enhancing proliferation of primary human CD4⁺ T effector cells in the presence of Treg cells;
   (e) binding human or rhesus monkey OX40 respectively; or
   (f) having no cross-reactivity to human CD40, CD137 and CD271.
12. The isolated antibody or the antigen-binding portion thereof of any of the preceding clauses, wherein the antibody is a monoclonal antibody, a chimeric antibody, or a humanized antibody.
13. The isolated antibody or the antigen-binding portion thereof of any of the preceding clauses, wherein the antibody is a fully human monoclonal antibody.
14. The isolated antibody or the antigen-binding portion thereof of any of the preceding clauses, wherein the antibody is a fully human monoclonal antibody produced by a transgenic mammalian animal.
15. The isolated antibody or the antigen-binding portion thereof of any of the preceding clauses, wherein the antibody is a fully human monoclonal antibody produced by a transgenic rat.
16. The isolated antibody or the antigen-binding portion thereof of any of the preceding clauses, wherein the antibody is a fully human monoclonal antibody produced by a transgenic rat with recombinant immunoglobulin loci.
17. The isolated antibody or the antigen-binding portion thereof of any of the preceding clauses, wherein the antibody is fused to a constant region of an IgG.
18. The isolated antibody or the antigen-binding portion thereof of any of the preceding clauses, wherein the antibody is fused to a constant region of a human IgG.
19. The isolated antibody or the antigen-binding portion thereof of any of the preceding clauses, wherein the antibody is fused to a constant region of a human IgG1 or human IgG4.
20. The isolated antibody or the antigen-binding portion thereof of any of the preceding clauses, wherein the antibody binds to CRD2 and/or CRD3 domain of OX40.
21. An isolated antibody or the antigen-binding portion thereof, which competes binding for the same epitope with the isolated antibody or the antigen-binding portion thereof of any of the preceding clauses.
22. An isolated nucleic acid molecule, comprising a nucleic acid sequence encoding the heavy chain variable region and/or the light chain variable region of the isolated antibody as defined in any of clauses 1-21.
23. The isolated nucleic acid molecule of clause 22, which encodes the heavy chain variable region of the isolated antibody as defined in any of clauses 1-21 and comprises a nucleic acid sequence selected from the group consisting of:
   (A) a nucleic acid sequence that encodes a heavy chain variable region as set forth in SEQ ID NO: 33, 35, 37, 39, 41 or 43;
   (B) a nucleic acid sequence as set forth in SEQ ID NO: 45, 47, 49, 51, 53 or 55; or
   (C) a nucleic acid sequence that hybridized under high stringency conditions to the complementary strand of the nucleic acid sequence of (A) or (B).
24. The isolated nucleic acid molecule of clause 22, which encodes the light chain variable region of the isolated antibody as defined in any of clauses 1-21 and comprises a nucleic acid sequence selected from the group consisting of:
   (A) a nucleic acid sequence that encodes a heavy chain variable region as set forth in SEQ ID NO: 34, 36, 38, 40, 42 or 44;
   (B) a nucleic acid sequence as set forth in SEQ ID NO: 46, 48, 50, 52, 54 or 56; or
   (C) a nucleic acid sequence that hybridized under high stringency conditions to the complementary strand of the nucleic acid sequence of (A) or (B).
25. A vector comprising the nucleic acid molecule of any of clauses 22-24.
26. A host cell comprising the vector of clause 25.
27. A pharmaceutical composition comprising at least one antibody or antigen-binding portion thereof as defined in any of clauses 1-21 and a pharmaceutically acceptable carrier.
28. A method for preparing antibody or antigen-binding portion thereof as defined in any of clauses 1-21 comprising the steps of:
   - expressing the antibody or antigen-binding portion thereof as defined in any of clauses 1-21 in the host cell of clause 26; and
   - isolating the antibody or antigen-binding portion thereof from the host cell.
29. A method of modulating an immune response in a subject, comprising administering to the subject the antibody or antigen-binding portion thereof as defined in any of clauses 1-21 such that an immune response is modulated in the subject.
30. The method of clause 24, wherein the T cell proliferation is enhanced in the subject.
31. The method of clause 24, wherein anti-CD3 induced proliferation of primary human CD4⁺ T cells is enhanced in the subject.
32. The method of clause 24, wherein the proliferation of primary human CD4⁺ T effector cells in the presence of Treg cells is enhanced in the subject.
33. The method of clause 24, wherein the cytokine IFN-γ production is increased.
34. The method of clause 24, wherein the cytokine IL-2 production is increased.
35. A method for treating abnormal cell growth in a subject, comprising administering an effective amount of the antibody or antigen-binding portion thereof as defined in any of clauses 1-21 or the pharmaceutical composition of clause 27 to the subject.
36. A method for impairing the suppressive function of Treg cells in a subject, comprising administering an effective amount of the antibody or antigen-binding portion thereof as defined in any of clauses 1-21 or the pharmaceutical composition of clause 27 to the subject.
37. A method for inhibiting growth of tumor cells in a subject, comprising administering an effective amount of the antibody or antigen-binding portion thereof as defined in any of clauses 1-21 or the pharmaceutical composition of clause 27 to the subject.
38. A method for reducing tumor cell metastasis in a subject, comprising administering an effective amount of the antibody or antigen-binding portion thereof as defined in any of clauses 1-21 or the pharmaceutical composition of clause 27 to the subject.
39. A method for treating or preventing diseases comprising proliferative disorders (such as cancers), autoimmune diseases, inflammatory disease or infectious diseases in a subject, comprising administering an effective amount of the antibody or antigen-binding portion thereof as defined in any of clauses 1-21 or the pharmaceutical composition of clause 27 to the subject.
40. The method of clause 39, wherein the cancer is solid cancer.
41. The method of clause 39 or 40, wherein the cancer is colon cancer.
42. Use of the antibody or antigen-binding portion thereof as defined in any of clauses 1-21 in the manufacture of a medicament for modulating an immune response in a subject.
43. Use of the antibody or antigen-binding portion thereof as defined in any of clauses 1-21 in the manufacture of a medicament for treating abnormal cell growth in a subject.
44. Use of the antibody or antigen-binding portion thereof as defined in any of clauses 1-21 in the manufacture of a medicament for inhibiting growth of tumor cells in a subject.
45. Use of the antibody or antigen-binding portion thereof as defined in any of clauses 1-21 in the manufacture of a medicament for reducing tumor cell metastasis in a subject.
46. Use of the antibody or antigen-binding portion thereof as defined in any of clauses 1-21 in the manufacture of a medicament for treating or preventing proliferative disorders (such as cancers), autoimmune diseases, inflammatory disease or infectious diseases.
47. Use of the antibody or antigen-binding portion thereof as defined in any of clauses 1-21 in the manufacture of a diagnostic agent for diagnosing proliferative disorders (such as cancers), autoimmune diseases, inflammatory disease or infectious diseases.
48. Antibody or antigen-binding portion thereof as defined in any of clauses 1-21 for use in treating or preventing proliferative disorders (such as cancers), autoimmune diseases, inflammatory disease or infectious diseases.
49. Antibody or antigen-binding portion thereof as defined in any of clauses 1-21 for use in diagnosing proliferative disorders (such as cancers), autoimmune diseases, inflammatory disease or infectious diseases.
50. A kit for treating or diagnosing proliferative disorders (such as cancers), autoimmune diseases, inflammatory disease or infectious diseases, comprising a container comprising at least one antibody or antigen-binding portion thereof as defined in any of clauses 1-21.

### BRIEF DESCRIPTION OF THE FIGURES

Figure 1 is a graph showing comparison between variants of the anti-OX40 antibody 1.62.3-ul-IgGlK after PTM mutation.
Figure 2 is a graph showing antibodies binding to human OX40 transfected CHO-K1 cells.
Figure 3 is a graph showing antibodies binding to activated human CD4⁺ T cells.
Figure 4 is a graph showing antibodies competitively binding to OX40 with OX40L.
Figure 5 is a graph showing antibodies binding to rhesus monkey OX40 transfected 293F cells.
Figure 6 is a graph showing results of cross family binding test of anti-OX40 antibodies to other TNFR family members including human CD40, CD137 and CD271 by ELISA.
Figures 7A, 7B and 7C are graphs showing epitope binning of the antibodies against benchmark antibodies BMK1 (Figure 7A), BMK7 (Figure 7B) and BMK10 (Figure 7C), respectively.
Figures 8A, 8B and 8C are graphs showing the effect of antibodies on OX40-stimulated NFkB luciferase activity in Jurkat cells using free antibodies or FcyR cross-linking by CD32b-expressing CHO-K1 cells or anti-human IgG Fc reagent. Reporter activity of (Figure 8A) free antibodies or cross-linked by (Figure 8B) F(ab')₂ goat anti-human IgG or (Figure 8C) CD32b-expressing CHO-K1 cells is shown, respectively.
Figure 9 is a graph showing the effect of antibodies on anti-CD3 induced IL-2 secretion by primary human CD4⁺ T cells.
Figure 10 is a graph showing the effect of antibodies on anti-CD3 induced IFN-γ secretion by primary human CD4⁺ T cells.
Figure 11 is a graph showing the effect of antibodies on anti-CD3 induced proliferation of primary human CD4⁺ T cells.
Figure 12 is a graph showing the effect of antibodies on CD3/CD28 Dynabeads induced proliferation of primary human CD4⁺ T effector cells in the presence of Treg cells.
Figure 13A is a graph showing OX40 expression on activated human CD4⁺ T cells, and Figure 13B is a graph showing OX40 expression on OX40 over-expressing Jurkat cells.
Figure 14A is a graph showing the ADCC effect of OX40 antibodies on OX40 over-expressing Jurkat cells, and Figure 14B is a graph showing the ADCC effect of OX40 antibodies on activated human CD4⁺ T cells.
Figure 15A is a graph showing the CDC effect of OX40 antibodies on OX40 over-expressing Jurkat cells, and Figure 15B is a graph showing the CDC effect of OX40 antibodies on activated human CD4⁺ T cells.
Figures 16A and 16B are graphs showing tumor growth of MC38 tumor-bearing mice post administration of the antibody 1.134.9-ul-IgGlL.
Figure 17 is a graph showing body weight change of MC38 tumor-bearing mice post administration of the antibody 1.134.9-u1-IgG1L.

### DETAILED DESCRIPTION OF THE INVENTION

While the present invention may be embodied in many different forms, disclosed herein are specific illustrative embodiments thereof that exemplify the principles of the invention. It should be emphasized that the present invention is not limited to the specific embodiments illustrated. Moreover, any section headings used herein are for organizational purposes only and are not to be construed as limiting the subject matter described.

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. More specifically, as used in this specification and the appended claims, the singular forms "a", "an" and "the" include plural referents unless the context clearly dictates otherwise. Thus, for example, reference to "a protein" includes a plurality of proteins; reference to "a cell" includes mixtures of cells, and the like. In this application, the use of "or" means "and/or" unless stated otherwise. Furthermore, the use of the term "comprising", as well as other forms, such as "comprises" and "comprised", is not limiting. In addition, ranges provided in the specification and appended claims include both end points and all points between the end points.

Generally, nomenclature used in connection with, and techniques of, cell and tissue culture, molecular biology, immunology, microbiology, genetics and protein and nucleic acid chemistry and hybridization described herein are those well-known and commonly used in the art. The methods and techniques of the present invention are generally performed according to conventional methods well known in the art and as described in various general and more specific references that are cited and discussed throughout the present specification unless otherwise indicated. See, e.g., Abbas et al., Cellular and Molecular Immunology, 6th ed., W.B. Saunders Company (2010); Sambrook J. & Russell D. Molecular Cloning: A Laboratory Manual, 3rd ed., Cold Spring Harbor Laboratory Press, Cold Spring Harbor, N.Y. (2000); Ausubel et al., Short Protocols in Molecular Biology: A Compendium of Methods from Current Protocols in Molecular Biology, Wiley, John & Sons, Inc. (2002); Harlow and Lane Using Antibodies: A Laboratory Manual, Cold Spring Harbor Laboratory Press, Cold Spring Harbor, N.Y. (1998); and Coligan et al., Short Protocols in Protein Science, Wiley, John & Sons, Inc. (2003). The nomenclature used in connection with, and the laboratory procedures and techniques of, analytical chemistry, synthetic organic chemistry, and medicinal and pharmaceutical chemistry described herein are those well-known and commonly used in the art. Moreover, any section headings used herein are for organizational purposes only and are not to be construed as limiting the subject matter described.

### Definitions

In order to better understand the invention, the definitions and explanations of the relevant terms are provided as follows.

The term "antibody" or "Ab", as used herein, generally refers to a Y-shaped tetrameric protein comprising two heavy (H) and two light (L) polypeptide chains held together by covalent disulfide bonds and non-covalent interactions. Light chains of an antibody may be classified into κ and λ light chain. Heavy chains may be classified into µ, δ, γ, α and ε, which define isotypes of an antibody as IgM, IgD, IgG, IgA and IgE, respectively. In a light chain and a heavh chain, a variable region is linked to a constant region via a "J" region of about 12 or more amino acids, and a heavy chain further comprises a "D" region of about 3 or more amino acids. Each heavy chain consists of a heavy chain variable region (V_{H}) and a heavy chain constant region (C_{H}). A heavy chain constant region consists of 3 domains (C_{H}1, C_{H}2 and C_{H}3). Each light chain consists of a light chain variable region (V_{L}) and a light chain constant region (C_{L}). V_{H} and V_{L} region can further be divided into hypervariable regions (called complementary determining regions (CDR)), which are interspaced by relatively conservative regions (called framework region (FR)). Each V_{H} and V_{L} consists of 3 CDRs and 4 FRs in the following order: FR1, CDR1, FR2, CDR2, FR3, CDR3, FR4 from N-terminal to C-terminal. The variable region (V_{H} and V_{L}) of each heavy/light chain pair forms antigen binding sites, respectively. Distribution of amino acids in various regions or domains follows the definition in Kabat Sequences of Proteins of Immunological Interest (National Institutes of Health, Bethesda, Md. (1987 and 1991)), or Chothia & Lesk (1987) J. Mol. Biol. 196:901-917; Chothia et al., (1989) Nature 342:878-883. Antibodies may be of different antibody isotypes, for example, IgG (e.g., IgG1, IgG2, IgG3 or IgG4 subtype), IgAl, IgA2, IgD, IgE or IgM antibody.

The term "antigen-binding portion" or "antigen-binding fragment" of an antibody, which can be interchangeably used in the context of the application, refers to polypeptides comprising fragments of a full-length antibody, which retain the ability of specifically binding to an antigen that the full-length antibody speificaly binds to, and/or compete with the full-length antibody for binding to the same antigen. Generally, see Fundamental Immunology, Ch. 7 (Paul, W., ed., the second edition, Raven Press, N.Y. (1989). Antigen binding fragments of an antibody may be produced by recombinant DNA techniques or by enzymatic or chemical cleavage of an intact antibody. Under some conditions, antigen binding fragments include Fab, Fab', F(ab')₂, Fd, Fv, dAb and complementary determining region (CDR) fragments, single chain antibody (e.g. scFv), chimeric antibody, diabody and such polypeptides that comprise at least part of antibody sufficient to confer the specific antigen binding ability on the polypeptides. Antigen binding fragments of an antibody may be obtained from a given antibody (e.g., the monoclonal anti-human OX40 antibody provided in the instant application) by conventional techniques known by a person skilled in the art (e.g., recombinant DNA technique or enzymatic or chemical cleavage methods), and may be screened for specificity in the same manner by which intact antibodies are screened.

The term "monoclonal antibody" or "mAb", as used herein, refer to a preparation of antibody molecules of single molecular composition. A monoclonal antibody displays a single binding specificity and affinity for a particular epitope.

The term "human antibody" or "fully human antibody", as used herein, is intended to include antibodies having variable regions in which both the framework and CDR regions are derived from human germline immunoglobulin sequences. Furthermore, if the antibody contains a constant region, the constant region also is derived from human germline immunoglobulin sequences. The human antibodies of the invention can include amino acid residues not encoded by human germline immunoglobulin sequences (e.g., mutations introduced by random or site- specific mutagenesis in vitro or by somatic mutation in vivo). However, the term "human antibody", as used herein, is not intended to include antibodies in which CDR sequences derived from the germline of another mammalian species, such as a mouse, have been grafted onto human framework sequences.

The term "human monoclonal antibody", as used herein, refers to antibodies displaying a single binding specificity, which have variable regions in which both the framework and CDR regions are derived from human germline immunoglobulin sequences.

The term "humanized antibody" is intended to refer to antibodies in which CDR sequences derived from the germline of another mammalian species, such as a mouse, have been grafted onto human framework sequences. Additional framework region modifications may be made within the human framework sequences.

The term "chimeric antibody", as used herein, refers to an antibody in which the variable region sequences are derived from one species and the constant region sequences are derived from another species, such as an antibody in which the variable region sequences are derived from a mouse antibody and the constant region sequences are derived from a human antibody.

The term "recombinant antibody", as used herein, refers to an antibody that is prepared, expressed, created or isolated by recombinant means, such as antibodies isolated from an animal that is transgenic for another species' immunoglobulin genes, antibodies expressed using a recombinant expression vector transfected into a host cell, antibodies isolated from a recombinant, combinatorial antibody library, or antibodies prepared, expressed, created or isolated by any other means that involves splicing of immunoglobulin gene sequences to other DNA sequences.

The term "anti-OX40 antibody" or "OX40 antibody, as used herein, refers to an antibody, as defined herein, capable of binding to an OX40 receptor, for example, a human OX40 receptor.

The terms "OX40", "OX40 receptor", "OX40 protein", "tumor necrosis factor receptor superfamily, member 4 (TNFRSF4)", or "CD134", which are used interchangeably herein, is a member of the tumor necrosis factor (TNF) receptor superfamily. The term "OX40" may include human OX40 receptor, as well as variants, isoforms, and species homologs thereof. Accordingly, an antibody or antigen-binding portion thereof, as defined and disclosed herein, may also bind OX40 from species other than human, for example cynomolgus OX40.

The term "human OX40", as used herein, refers to human sequence OX40, such as the complete amino acid sequence of human OX40 having Genbank Accession No. CAE11757.1. The human OX40 sequence may differ from human OX40 of Genbank Accession No. CAE11757.1 by having, e.g., conserved mutations or mutations in non-conserved regions and the OX40 has substantially the same biological function as the human OX40 of Genbank Accession No. CAE11757.1.

The term "mouse OX40", as used herein, refers to mouse sequence OX40, such as the complete amino acid sequence of mouse OX40 having Genbank Accession No. CAA59476.1.

The term "cynomolgus OX40", as used herein, refers to cynomolgus sequence OX40, such as the complete amino acid sequence of Rhesus macaque OX40 having Genbank Accession No. XP_001090870.1.

The term "Ka", as used herein, is intended to refer to the association rate of a particular antibody-antigen interaction, whereas the term "Kd" as used herein, is intended to refer to the dissociation rate of a particular antibody-antigen interaction. Kd values for antibodies can be determined using methods well established in the art. The term "K_{D}" as used herein, is intended to refer to the dissociation constant of a particular antibody-antigen interaction, which is obtained from the ratio of Kd to Ka (i.e., Kd/Ka) and is expressed as a molar concentration (M). A preferred method for determining the Kd of an antibody is by using surface plasmon resonance, preferably using a biosensor system such as a Biacore^{®} system.

The term "high affinity" for an IgG antibody, as used herein, refers to an antibody having a K_{D} of 1 × 10⁻⁷ M or less, more preferably 5 × 10⁻⁸ M or less, even more preferably 1×10⁻⁸ M or less, even more preferably 5 × 10⁻⁹ M or less and even more preferably 1 × 10⁻⁹ M or less for a target antigen, for example, an OX40 receptor.

The term "EC₅₀", as used herein, which is also termed as "half maximal effective concentration" refers to the concentration of a drug, antibody or toxicant which induces a response halfway between the baseline and maximum after a specified exposure time. In the context of the application, EC₅₀ is expressed in the unit of "nM".

The term "compete for binding", as used herein, refers to the interaction of two antibodies in their binding to a binding target. A first antibody competes for binding with a second antibody if binding of the first antibody with its cognate epitope is detectably decreased in the presence of the second antibody compared to the binding of the first antibody in the absence of the second antibody. The alternative, where the binding of the second antibody to its epitope is also detectably decreased in the presence of the first antibody, can, but need not, be the case. That is, a first antibody can inhibit the binding of a second antibody to its epitope without that second antibody inhibiting the binding of the first antibody to its respective epitope. However, where each antibody detectably inhibits the binding of the other antibody with its cognate epitope, whether to the same, greater, or lesser extent, the antibodies are said to "cross-compete" with each other for binding of their respective epitope(s).

The ability of "inhibit binding", as used herein, refers to the ability of an antibody or antigen-binding fragment thereof to inhibit the binding of two molecules (eg, human OX40 and human anti-OX40 antibody) to any detectable level. In certain embodiments, the binding of the two molecules can be inhibited at least 50% by the antibody or antigen-binding fragment thereof. In certain embodiments, such an inhibitory effect may be greater than 60%, greater than 70%, greater than 80%, or greater than 90%.

The term "epitope", as used herein, refers to a portion on antigen that an immunoglobulin or antibody specifically binds to. "Epitope" is also known as "antigenic determinant". Epitope or antigenic determinant generally consists of chemically active surface groups of a molecule such as amino acids, carbohydrates or sugar side chains, and generally has a specific three-dimensional structure and a specific charge characteristic. For example, an epitope generally comprises at least 3, 4, 5, 6, 7, 8, 9, 10, 11, 12, 13, 14 or 15 consecutive or non-consecutive amino acids in a unique steric conformation, which may be "linear" or "conformational". See, for example, Epitope Mapping Protocols in Methods in Molecular Biology, Vol. 66, G. E. Morris, Ed. (1996). In a linear epitope, all the interaction sites between a protein and an interaction molecule (e.g., an antibody) are present linearly along the primary amino acid sequence of the protein. In a conformational epitope, the interaction sites span over amino acid residues that are separate from each other in a protein. Antibodies may be screened depending on competitiveness of binding to the same epitope by conventional techniques known by a person skilled in the art. For example, study on competition or cross-competition may be conducted to obtain antibodies that compete or cross-compete with each other for binding to antigens (e.g. RSV fusion protein). High-throughput methods for obtaining antibodies binding to the same epitope, which are based on their cross-competition, are described in an international patent application WO 03/48731.

The term "isolated", as used herein, refers to a state obtained from natural state by artificial means. If a certain "isolated" substance or component is present in nature, it is possible because its natural environment changes, or the substance is isolated from natural environment, or both. For example, a certain un-isolated polynucleotide or polypeptide naturally exists in a certain living animal body, and the same polynucleotide or polypeptide with a high purity isolated from such a natural state is called isolated polynucleotide or polypeptide. The term "isolated" excludes neither the mixed artificial or synthesized substance nor other impure substances that do not affect the activity of the isolated substance.

The term "isolated antibody", as used herein, is intended to refer to an antibody that is substantially free of other antibodies having different antigenic specificities (e.g., an isolated antibody that specifically binds an OX40 protein is substantially free of antibodies that specifically bind antigens other than OX40 proteins). An isolated antibody that specifically binds a human OX40 protein may, however, have cross-reactivity to other antigens, such as OX40 proteins from other species. Moreover, an isolated antibody can be substantially free of other cellular material and/or chemicals.

The term "vector", as used herein, refers to a nucleic acid vehicle which can have a polynucleotide inserted therein. When the vector allows for the expression of the protein encoded by the polynucleotide inserted therein, the vector is called an expression vector. The vector can have the carried genetic material elements expressed in a host cell by transformation, transduction, or transfection into the host cell. Vectors are well known by a person skilled in the art, including, but not limited to plasmids, phages, cosmids, artificial chromosome such as yeast artificial chromosome (YAC), bacterial artificial chromosome (BAC) or P1-derived artificial chromosome (PAC); phage such as λ phage or M13 phage and animal virus. The animal viruses that can be used as vectors, include, but are not limited to, retrovirus (including lentivirus), adenovirus, adeno-associated virus, herpes virus (such as herpes simplex virus), pox virus, baculovirus, papillomavirus, papova virus (such as SV40). A vector may comprise multiple elements for controlling expression, including, but not limited to, a promoter sequence, a transcription initiation sequence, an enhancer sequence, a selection element and a reporter gene. In addition, a vector may comprise origin of replication.

The term "host cell", as used herein, refers to a cellular system which can be engineered to generate proteins, protein fragments, or peptides of interest. Host cells include, without limitation, cultured cells, e.g., mammalian cultured cells derived from rodents (rats, mice, guinea pigs, or hamsters) such as CHO, BHK, NSO, SP2/0, YB2/0; or human tissues or hybridoma cells, yeast cells, and insect cells, and cells comprised within a transgenic animal or cultured tissue. The term encompasses not only the particular subject cell but also the progeny of such a cell. Because certain modifications may occur in succeeding generations due to either mutation or environmental influences, such progeny may not be identical to the parent cell, but are still included within the scope of the term "host cell."

The term "identity", as used herein, refers to a relationship between the sequences of two or more polypeptide molecules or two or more nucleic acid molecules, as determined by aligning and comparing the sequences. "Percent identity" means the percent of identical residues between the amino acids or nucleotides in the compared molecules and is calculated based on the size of the smallest of the molecules being compared. For these calculations, gaps in alignments (if any) are preferably addressed by a particular mathematical model or computer program (i.e., an "algorithm"). Methods that can be used to calculate the identity of the aligned nucleic acids or polypeptides include those described in Computational Molecular Biology, (Lesk, A. M., ed.), 1988, New York: Oxford University Press; Biocomputing Informatics and Genome Projects, (Smith, D. W., ed.), 1993, New York: Academic Press; Computer Analysis of Sequence Data, Part I, (Griffin, A. M., and Griffin, H. G., eds.), 1994, New Jersey: Humana Press; von Heinje, G., 1987, Sequence Analysis in Molecular Biology, New York: Academic Press; Sequence Analysis Primer, (Gribskov, M. and Devereux, J., eds.), 1991, New York: M. Stockton Press; and Carillo et al, 1988, SIAMJ. Applied Math. 48:1073.

The term "immunogenicity", as used herein, refers to ability of stimulating the formation of specific antibodies or sensitized lymphocytes in organisms. It not only refers to the property of an antigen to stimulate a specific immunocyte to activate, proliferate and differentiate so as to finally generate immunologic effector substance such as antibody and sensitized lymphocyte, but also refers to the specific immune response that antibody or sensitized T lymphocyte can be formed in immune system of an organism after stimulating the organism with an antigen. Immunogenicity is the most important property of an antigen. Whether an antigen can successfully induce the generation of an immune response in a host depends on three factors, properties of an antigen, reactivity of a host, and immunization means.

The term "transfection", as used herein, refers to the process by which nucleic acids are introduced into eukaryotic cells, particularly mammalian cells. Protocols and techniques for transfection include but not limited to lipid transfection and chemical and physical methods such as electroporation. A number of transfection techniques are well known in the art and are disclosed herein. See, e.g., Graham et al., 1973, Virology 52:456; Sambrook et al., 2001, Molecular Cloning: A Laboratory Manual, supra; Davis et al., 1986, Basic Methods in Molecular Biology, Elsevier; Chu et al, 1981, Gene 13:197. In a specific embodiment of the invention, human OX40 gene was transfected into 293F cells.

The term "hybridoma" and the term "hybridoma cell line", as used herein, may be used interchangeably. When the term "hybridoma" and the term "hybridoma cell line" are mentioned, they also include subclone and progeny cell of hybridoma.

The term "SPR" or "surface plasmon resonance", as used herein, refers to and includes an optical phenomenon that allows for the analysis of real-time biospecific interactions by detection of alterations in protein concentrations within a biosensor matrix, for example using the BIAcore system (Pharmacia Biosensor AB, Uppsala, Sweden and Piscataway, N.J.). For further descriptions, see Example 5 and Jönsson, U., et al. (1993) Ann. Biol. Clin. 51:19-26; Jönsson, U., et al. (1991) Biotechniques 11:620-627; Johnsson, B., et al. (1995) J. Mol. Recognit. 8:125-131; and Johnnson, B., et al. (1991) Anal. Biochem. 198:268-277.

The term "fluorescence-activated cell sorting" or "FACS", as used herein, refers to a specialized type of flow cytometry. It provides a method for sorting a heterogeneous mixture of biological cells into two or more containers, one cell at a time, based upon the specific light scattering and fluorescent characteristics of each cell (FlowMetric. "Sorting Out Fluorescence Activated Cell Sorting". Retrieved 2017-11-09.). Instruments for carrying out FACS are known to those of skill in the art and are commercially available to the public. Examples of such instruments include FACS Star Plus, FACScan and FACSort instruments from Becton Dickinson (Foster City, Calif.) Epics C from Coulter Epics Division (Hialeah, Fla.) and MoFlo from Cytomation (Colorado Springs, Colo.).

The term "antibody-dependent cell-mediated cytotoxicity" or "ADCC", as used herein, refers to a form of cytotoxicity in which secreted Ig bound onto Fc receptors (FcRs) present on certain cytotoxic cells (e.g. Natural Killer (NK) cells, neutrophils, and macrophages) enable these cytotoxic effector cells to bind specifically to an antigen-bearing target cell and subsequently kill the target cell with cytotoxins. The antibodies "arm" the cytotoxic cells and are absolutely required for such killing. The primary cells for mediating ADCC, NK cells, express FcyRIII only, whereas monocytes express FcyRI, FcyRII and FcyRIII. FcR expression on hematopoietic cells is summarized in Table 3 on page 464 of Ravetch and Kinet, Annu. Rev. Immunol 9:457-92 (1991). To assess ADCC activity of a molecule of interest, an in vitro ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed in vivo, e.g., in an animal model such as that disclosed in Clynes et al. PNAS (USA) 95:652-656 (1998).

The term "complement dependent cytotoxicity" or "CDC" refers to the lysis of a target cell in the presence of complement. Activation of the classical complement pathway is initiated by the binding of the first component of the complement system (Clq) to antibodies (of the appropriate subclass) which are bound to their cognate antigen. To assess complement activation, a CDC assay, e.g. as described in Gazzano-Santoro et al., J. Immunol. Methods 202: 163 (1996), may be performed.

The term "subject" includes any human or nonhuman animal, preferably humans.

The term "cancer", as used herein, refers to any or a tumor or a malignant cell growth, proliferation or metastasis-mediated, solid tumors and non-solid tumors such as leukemia and initiate a medical condition.

The term "treatment", "treating" or "treated", as used herein in the context of treating a condition, pertains generally to treatment and therapy, whether of a human or an animal, in which some desired therapeutic effect is achieved, for example, the inhibition of the progress of the condition, and includes a reduction in the rate of progress, a halt in the rate of progress, regression of the condition, amelioration of the condition, and cure of the condition. Treatment as a prophylactic measure (i.e., prophylaxis, prevention) is also included. For cancer, "treating" may refer to dampen or slow the tumor or malignant cell growth, proliferation, or metastasis, or some combination thereof. For tumors, "treatment" includes removal of all or part of the tumor, inhibiting or slowing tumor growth and metastasis, preventing or delaying the development of a tumor, or some combination thereof.

The term "an effective amount", as used herein, pertains to that amount of an active compound, or a material, composition or dosage from comprising an active compound, which is effective for producing some desired therapeutic effect, commensurate with a reasonable benefit/risk ratio, when administered in accordance with a desired treatment regimen. For instance, the "an effective amount", when used in connection with treatment of OX40-related diseases or conditions, refers to an antibody or antigen-binding portion thereof in an amount or concentration effective to treat the said diseases or conditions.

The term "prevent", "prevention" or "preventing", as used herein, with reference to a certain disease condition in a mammal, refers to preventing or delaying the onset of the disease, or preventing the manifestation of clinical or subclinical symptoms thereof.

The term "pharmaceutically acceptable", as used herein, means that the vehicle, diluent, excipient and/or salts thereof, are chemically and/or physically is compatible with other ingredients in the formulation, and the physiologically compatible with the recipient.

As used herein, the term "a pharmaceutically acceptable carrier and/or excipient" refers to a carrier and/or excipient pharmacologically and/or physiologically compatible with a subject and an active agent, which is well known in the art (see, e.g., Remington's Pharmaceutical Sciences. Edited by Gennaro AR, 19th ed. Pennsylvania: Mack Publishing Company, 1995), and includes, but is not limited to pH adjuster, surfactant, adjuvant and ionic strength enhancer. For example, the pH adjuster includes, but is not limited to, phosphate buffer; the surfactant includes, but is not limited to, cationic, anionic, or non-ionic surfactant, e.g., Tween-80; the ionic strength enhancer includes, but is not limited to, sodium chloride.

As used herein, the term "adjuvant" refers to a non-specific immunopotentiator, which can enhance immune response to an antigen or change the type of immune response in an organism when it is delivered together with the antigen to the organism or is delivered to the organism in advance. There are a variety of adjuvants, including, but not limited to, aluminium adjuvants (for example, aluminum hydroxide), Freund's adjuvants (for example, Freund's complete adjuvant and Freund's incomplete adjuvant), coryne bacterium parvum, lipopolysaccharide, cytokines, and the like. Freund's adjuvant is the most commonly used adjuvant in animal experiments now. Aluminum hydroxide adjuvant is more commonly used in clinical trials.

### Anti-OX40 Antibodies

In some aspects, the invention comprises an isolated antibody or an antigen-binding portion thereof.

In the context of the application, the "antibody" may include polyclonal antibodies, multiclonal antibodies, monoclonal antibodies, chimeric antibodies, humanized and primatized antibodies, CDR grafted antibodies, human antibodies, recombinantly produced antibodies, intrabodies, multispecific antibodies, bispecific antibodies, monovalent antibodies, multivalent antibodies, anti-idiotypic antibodies, synthetic antibodies, including muteins and variants thereof,; and derivatives thereof including Fc fusions and other modifications, and any other immunoreactive molecule so long as it exhibits preferential association or binding with a OX40 protein. Moreover, unless dictated otherwise by contextual constraints the term further comprises all classes of antibodies (i.e. IgA, IgD, IgE, IgG, and IgM) and all subclasses (i.e., IgG1,IgG2, IgG3, IgG4, IgAl, and IgA2). In a preferred embodiment, the antibody is a monoclonal antibody. In a more preferred embodiment, the antibody is a human monoclonal antibody.

Human antibodies can be produced using various techniques known in the art. One technique is phage display in which a library of (preferably human) antibodies is synthesized on phages, the library is screened with the antigen of interest or an antibody-binding portion thereof, and the phage that binds the antigen is isolated, from which one may obtain the immune-reactive fragments. Methods for preparing and screening such libraries are well known in the art and kits for generating phage display libraries are commercially available (e.g., the Pharmacia Recombinant Phage Antibody System, catalog no. 27-9400-01; and the Stratagene SurfZAP^{™} phage display kit, catalog no. 240612). There also are other methods and reagents that can be used in generating and screening antibody display libraries (see, e.g., Barbas et al., Proc. Natl. Acad. Sci. USA 88:7978-7982 (1991)).

Human antibodies can also be made by introducing human immunoglobulin loci into transgenic animals, e.g., mice in which the endogenous immunoglobulin genes have been partially or completely inactivated and human immunoglobulin genes have been introduced. Upon challenge, human antibody production is observed, which closely resembles that seen in humans in all respects, including gene rearrangement, assembly, and antibody repertoire. This approach is described, for example, in U.S.P.Ns. 5,545,807; 5,545,806; 5,569,825; 5,625,126; 5,633,425; 5,661,016, and U.S.P.Ns. 6,075,181 and 6,150,584 regarding XenoMouse^{®} technology; and Lonberg and Huszar, Intern. Rev. Immunol. 13:65-93 (1995). Alternatively, the human antibody may be prepared via immortalization of human B lymphocytes producing an antibody directed against a target antigen (such B lymphocytes may be recovered from an individual suffering from a neoplastic disorder or may have been immunized *in vitro).* See, e.g., Cole et al., Monoclonal Antibodies and Cancer Therapy, Alan R. Liss, p. 77 (1985); Boerner et al., J. Immunol, 147 (1):86-95 (1991); and U.S.P.N. 5,750,373.

Monoclonal antibodies can be prepared using a wide variety of techniques known in the art including hybridoma techniques, recombinant techniques, phage display technologies, transgenic animals (e.g., a XenoMouse^{®}) or some combination thereof. For example, monoclonal antibodies can be produced using hybridoma and art-recognized biochemical and genetic engineering techniques such as described in more detail in An, Zhigiang (ed.) Therapeutic Monoclonal Antibodies: From Bench to Clinic, John Wiley and Sons, 1st ed. 2009; Shire et. al. (eds.) Current Trends in Monoclonal Antibody Development and Manufacturing, Springer Science + Business Media LLC, 1st ed. 2010; Harlow et al., Antibodies: A Laboratory Manual, Cold Spring Harbor Laboratory Press, 2nd ed. 1988; Hammerling, et al., in: Monoclonal Antibodies and T-Cell Hybridomas 563-681 (Elsevier, N.Y., 1981). It should be understood that a selected binding sequence can be further altered, for example, to improve affinity for the target, to humanize the target binding sequence, to improve its production in cell culture, to reduce its immunogenicity in vivo, to create a multispecific antibody, etc., and that an antibody comprising the altered target binding sequence is also an antibody of this invention. In a preferred embodiment, the anti-human OX40 monoclonal antibody is prepared by using hybridoma.

### Generation of Hybridomas Producing Human Monoclonal Antibodies of the Invention

To generate hybridomas producing the antibodies of the invention, for instance, human monoclonal antibodies of the invention, splenocytes and/or lymph node cells from immunized mice can be isolated and fused to an appropriate immortalized cell line, such as a mouse myeloma cell line. The resulting hybridomas can be screened for the production of antigen- specific antibodies. Generation of hybridomas is well-known in the art. See, e.g., Harlow and Lane (1988) Antibodies, A Laboratory Manual, Cold Spring Harbor Publications, New York.

### Generation of Transfectomas Producing Monoclonal Antibodies of the Invention

Antibodies of the invention also can be produced in a host cell transfectoma using, for example, a combination of recombinant DNA techniques and gene transfection methods as is well known in the art (e.g., Morrison, S. (1985) Science 229:1202). In one embodiment, DNA encoding partial or full-length light and heavy chains obtained by standard molecular biology techniques is inserted into one or more expression vectors such that the genes are operatively linked to transcriptional and translational regulatory sequences. In this context, the term "operatively linked" is intended to mean that an antibody gene is ligated into a vector such that transcriptional and translational control sequences within the vector serve their intended function of regulating the transcription and translation of the antibody gene.

The term "regulatory sequence" is intended to include promoters, enhancers and other expression control elements (e.g., polyadenylation signals) that control the transcription or translation of the antibody chain genes. Such regulatory sequences are described, e.g., in Goeddel (Gene Expression Technology. Methods in Enzymology 185, Academic Press, San Diego, CA (1990)). Exemplary regulatory sequences for mammalian host cell expression include viral elements that direct high levels of protein expression in mammalian cells, such as promoters and/or enhancers derived from cytomegalovirus (CMV), Simian Virus 40 (SV40), adenovirus, (e.g., the adenovirus major late promoter (AdMLP) and polyoma. Alternatively, nonviral regulatory sequences can be used, such as the ubiquitin promoter or β-globin promoter. Still further, regulatory elements composed of sequences from different sources, such as the SRa promoter system, which contains sequences from the SV40 early promoter and the long terminal repeat of human T cell leukemia virus type 1 (Takebe et al. (1988) Mol. Cell. Biol. 8:466-472). The expression vector and expression control sequences are chosen to be compatible with the expression host cell used.

The antibody light chain gene and the antibody heavy chain gene can be inserted into the same or separate expression vectors. In some embodiments, the variable regions are used to create full-length antibody genes of any antibody isotype by inserting them into expression vectors already encoding heavy chain constant and light chain constant regions of the desired isotype such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody chain genes and regulatory sequences, the recombinant expression vectors of the invention can carry additional sequences, such as sequences that regulate replication of the vector in host cells (e.g., origins of replication) and selectable marker genes. The selectable marker gene facilitates selection of host cells into which the vector has been introduced (see, e.g., U.S. Pat. Nos. 4,399,216; 4,634,665 and 5,179,017). For example, typically the selectable marker gene confers resistance to drugs, such as G418, hygromycin or methotrexate, on a host cell into which the vector has been introduced. Selectable marker genes may include the dihydrofolate reductase (DHFR) gene (for use in dhfr-host cells with methotrexate selection/amplification) and the neo gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, e.g., electroporation, calcium-phosphate precipitation, DEAE-dextran transfection and the like. It is possible to express the antibodies of the invention in either prokaryotic or eukaryotic host cells, for example, mammalian host cells, which can assemble and secrete a properly folded and immunologically active antibody.

Mammalian host cells for expressing the recombinant antibodies of the invention include Chinese Hamster Ovary (CHO cells) (including dhfr CHO cells, described in Urlaub and Chasin, (1980) Proc. Natl. Acad. ScL USA 77:4216-4220, used with a DHFR selectable marker, e.g., as described in R. J. Kaufman and P. A. Sharp (1982) J. Mol. Biol. 159:601-621), NSO myeloma cells, COS cells and SP2 cells. In particular, for use with NSO myeloma cells, another expression system is the GS gene expression system disclosed in WO 87/04462, WO 89/01036 and EP 338,841. When recombinant expression vectors encoding antibody genes are introduced into mammalian host cells, the antibodies are produced by culturing the host cells for a period of time sufficient to allow for expression of the antibody in the host cells or, secretion of the antibody into the culture medium in which the host cells are grown. Antibodies can be recovered from the culture medium using standard protein purification methods.

### Anti-OX40 antibodies with certain properties

The antibodies of the invention are characterized by particular functional features or properties of the antibodies. In some embodiments, the isolated antibody or the antigen-binding portion thereof has one or more of the following properties:
(a) binding human OX40 with a K_{D} of 1 × 10⁻⁸ M or less;
(b) inducing production of a cytokine (e.g., IL-2 or IFN-γ) in CD4⁺T cells;
(c) enhancing proliferation of primary human CD4⁺T cells;
(d) enhancing proliferation of primary human CD4⁺ T effector cells in the presence of Treg cells;
(e) binding human or rhesus monkey OX40 respectively; or
(f) having no cross-reactivity to human CD40, CD137 and CD271

The antibody of the invention binds to human OX40 with high affinity. The binding of an antibody of the invention to OX40 can be assessed using one or more techniques well established in the art, for instance, ELISA. The binding specificity of an antibody of the invention can also be determined by monitoring binding of the antibody to cells expressing an OX40 protein, e.g., flow cytometry. For example, an antibody can be tested by a flow cytometry assay in which the antibody is reacted with a cell line that expresses human OX40, such as CHO cells that have been transfected to express OX40 on their cell surface. Other suitable cells for use in flow cytometry assays include anti-CD3-stimulated CD4⁺ activated T cells, which express native OX40. Additionally or alternatively, the binding of the antibody, including the binding kinetics (e.g., K_{d} value) can be tested in BIAcore binding assays. Still other suitable binding assays include ELISA assays, for example using a recombinant OX40 protein. For instance, an antibody of the invention binds to a human OX40 with a K_{D} of 1 × 10⁻⁸ M or less, binds to a human OX40 with a K_{D} of 1 × 10⁻⁹ M or less, binds to a human OX40 with a K_{D} of 5 × 10⁻¹⁰ M or less, binds to a human OX40 with a K_{D} of 2 × 10⁻¹⁰ M or less, binds to a human OX40 protein with a K_{D} of 1 × 10⁻¹⁰ M or less, binds to a human OX40 protein with a K_{D} of 5 × 10⁻¹¹ M or less, binds to a human OX40 protein with a K_{D} of 3 × 10⁻¹¹ M or less, or binds to a human OX40 protein with a K_{D} of 2 × 10⁻¹¹ M or less.

### Anti-OX40 antibodies comprising CDRs with sequence identity to specific sequences

In some embodiments, the isolated antibody or the antigen-binding portion thereof comprises:
A) one or more heavy chain CDRs (CDRHs) selected from at least one of the group consisting of:
   (i) a CDRH1 with at least 90% sequence identity to a CDRH1 as set forth in one of the sequences selected from the group consisting of SEQ ID NOs: 1, 7, 13, 15, 21 and 27;
   (ii) a CDRH2 with at least 90% sequence identity to a CDRH2 as set forth in one of the sequences selected from the group consisting of SEQ ID NOs: 3, 9, 17, 23 and 29; and
   (iii) a CDRH3 with at least 90%, sequence identity to a CDRH3 as set forth in one of the sequences selected from the group consisting of SEQ ID NOs: 5, 11, 19, 25 and 31;
B) one or more light chain CDRs (CDRLs) selected from at least one of the group consisting of:
   (i) a CDRL1 with at least 90% sequence identity to a CDRL1 as set forth in one of the sequences selected from the group consisting of SEQ ID NOs: 2, 8, 14, 16, 22 and 28;
   (ii) a CDRL2 with at least 90% sequence identity to a CDRL2 as set forth in one of the sequences selected from the group consisting of SEQ ID NOs: 4, 10, 18, 24 and 30; and
   (iii) a CDRL3 with at least 90% sequence identity to a CDRL3 as set forth in one of the sequences selected from the group consisting of SEQ ID NOs: 6, 12, 20, 26 and 32; or
C) one or more CDRHs of A) and one or more CDRLs of B).

The assignment of amino acids to each CDR may be in accordance with one of the numbering schemes provided by Kabat et al. (1991) Sequences of Proteins of Immunological Interest (5th Ed.), US Dept. of Health and Human Services, PHS, NIH, NIH Publication no. 91-3242; Chothia *et al.,* 1987, PMID: 3681981; Chothia *et al.,* 1989, PMID: 2687698; MacCallum *et al*.,1996, PMID: 8876650; or Dubel, Ed. (2007) Handbook of Therapeutic Antibodies, 3rd Ed., Wily-VCH Verlag GmbH and Co. unless otherwise noted.

Variable regions and CDRs in an antibody sequence can be identified according to general rules that have been developed in the art (as set out above, such as, for example, the Kabat numbering system) or by aligning the sequences against a database of known variable regions. Methods for identifying these regions are described in Kontermann and Dubel, eds., Antibody Engineering, Springer, New York, NY, 2001 and Dinarello et al., Current Protocols in Immunology, John Wiley and Sons Inc., Hoboken, NJ, 2000. Exemplary databases of antibody sequences are described in, and can be accessed through, the "Abysis" website at www.bioinf.org.uk/abs (maintained by A.C. Martin in the Department of Biochemistry & Molecular Biology University College London, London, England) and the VBASE2 website at www.vbase2.org, as described in Retter et al., Nucl. Acids Res., 33 (Database issue): D671 -D674 (2005). Preferably sequences are analyzed using the Abysis database, which integrates sequence data from Kabat, IMGT and the Protein Data Bank (PDB) with structural data from the PDB. See Dr. Andrew C. R. Martin's book chapter Protein Sequence and Structure Analysis of Antibody Variable Domains. In: Antibody Engineering Lab Manual (Ed.: Duebel, S. and Kontermann, R., Springer-Verlag, Heidelberg, ISBN-13: 978-3540413547, also available on the website bioinforg.uk/abs). The Abysis database website further includes general rules that have been developed for identifying CDRs which can be used in accordance with the teachings herein. Unless otherwise indicated, all CDRs set forth herein are derived according to the Abysis database website as per Kabat.

The percent identity between two amino acid sequences can be determined using the algorithm of E. Meyers and W. Miller (Comput. Appl. Biosci., 4:11-17 (1988)) which has been incorporated into the ALIGN program (version 2.0), using a PAM120 weight residue table, a gap length penalty of 12 and a gap penalty of 4. In addition, the percentage of identity between two amino acid sequences can be determined by the algorithm of Needleman and Wunsch (J. Mol. Biol. 48:444-453 (1970)) which has been incorporated into the GAP program in the GCG software package (available at http://www.gcg.com), using either a Blossum 62 matrix or a PAM250 matrix, and a gap weight of 16, 14, 12, 10, 8, 6, or 4 and a length weight of 1, 2, 3, 4, 5, or 6.

Additionally or alternatively, the protein sequences of the present invention can further be used as a "query sequence" to perform a search against public databases to, for example, identify related sequences. Such searches can be performed using the XBLAST program (version 2.0) of Altschul, et al. (1990) J. Mol. Biol. 215:403-10. BLAST protein searches can be performed with the XBLAST program, score = 50, wordlength = 3 to obtain amino acid sequences homologous to the antibody molecules of the invention. To obtain gapped alignments for comparison purposes, Gapped BLAST can be utilized as described in Altschul et al, (1997) Nucleic Acids Res. 25(17):3389-3402. When utilizing BLAST and Gapped BLAST programs, the default parameters of the respective programs {e.g., XBLAST and NBLAST) can be used. See www.ncbi.nlm.nih.gov.

In other embodiments, the CDR amino acid sequences can be at least 90%, 91%, 92%, 93%, 94%, 95%, 96%, 97%, 98% or 99% identical to the respective sequences set forth above. As an illustrative example, the antibody may comprise a CDRH1 with at least 90%, 91%, 92%, 93%, 94%, 95%, 96%, 97%, 98% or 99% sequence identity to a CDRH1 as set forth in one of the sequences selected from the group consisting of SEQ ID NOs: 1, 7, 13, 15, 21 and 27.

### Anti-OX40 antibodies comprising CDRs with amino acid addition, deletion and/or substitution

In some embodiments, the isolated antibody or the antigen-binding portion thereof comprises:
A) one or more heavy chain CDRs (CDRHs) selected from at least one of the group consisting of:
   (i) a CDRH1 selected from the group consisting of SEQ ID NOs: 1, 7, 13, 15, 21 and 27, or a CDRH1 that differs in amino acid sequence from the CDRH1 by an amino acid addition, deletion or substitution of not more than 2 amino acids;
   (ii) a CDRH2 selected from the group consisting of SEQ ID NOs: 3, 9, 17, 23 and 29, or a CDRH2 that differs in amino acid sequence from the CDRH2 by an amino acid addition, deletion or substitution of not more than 2 amino acids; and
   (iii) a CDRH3 selected from the group consisting of SEQ ID NOs: 5, 11, 19, 25 and 31, or a CDRH3 that differs in amino acid sequence from the CDRH3 by an amino acid addition, deletion or substitution of not more than 2 amino acids;
B) one or more light chain CDRs (CDRLs) selected from at least one of the group consisting of:
   (i) a CDRL1 selected from the group consisting of SEQ ID NOs: 2, 8, 14, 16, 22 and 28, or a CDRL1 that differs in amino acid sequence from the CDRL1 by an amino acid addition, deletion or substitution of not more than 2 amino acids;
   (ii) a CDRL2 selected from the group consisting of SEQ ID NOs: 4, 10, 18, 24 and 30, or a CDRL2 that differs in amino acid sequence from the CDRL2 by an amino acid addition, deletion or substitution of not more than 2 amino acids; and
   (iii) a CDRL3 selected from the group consisting of SEQ ID NOs: 6, 12, 20, 26 and 32, or a CDRL3 that differs in amino acid sequence from the CDRL3 by an amino acid addition, deletion or substitution of not more than 2 amino acids; or
C) one or more CDRHs of A) and one or more CDRLs of B).

In some embodiments, the CDRs of the isolated antibody or the antigen-binding portion thereof contain a conservative substitution of not more than 1 amino acid. The term "conservative substitution", as used herein, refers to amino acid substitutions which would not disadvantageously affect or change the essential properties of a protein/polypeptide comprising the amino acid sequence. For example, a conservative substitution may be introduced by standard techniques known in the art such as site-directed mutagenesis and PCR-mediated mutagenesis. Conservative amino acid substitutions include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having alkaline side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, tryptophan), amino acids having nonpolar side chains (for example, alanine, valine, leucine, isoleucine, proline, phenylalanine, methionine), amino acids having β-branched side chains (such as threonine, valine, isoleucine) and amino acids having aromatic side chains (for example, tyrosine, phenylalanine, tryptophan, histidine). Therefore, a corresponding amino acid residue is preferably substituted with another amino acid residue from the same side-chain family. Methods for identifying amino acid conservative substitutions are well known in the art (see, for example, Brummell et al., Biochem. 32: 1180-1187 (1993); Kobayashi et al., Protein Eng. 12(10): 879-884 (1999); and Burks et al., Proc. Natl. Acad. Sci. USA 94: 412-417 (1997)).

### Anti-OX40 antibodies comprising CDRs

In some embodiments, the isolated antibody or the antigen-binding portion thereof comprises:
(a) a CDRH1 comprising SEQ ID NO: 1;
(b) a CDRH2 comprising SEQ ID NO: 3;
(c) a CDRH3 comprising SEQ ID NO: 5;
(d) a CDRL1 comprising SEQ ID NO: 2;
(e) a CDRL2 comprising SEQ ID NO: 4; and
(f) a CDRL3 comprising SEQ ID NO: 6.

In a specific embodiment, the isolated antibody or the antigen-binding portion thereof comprises:
(a) a CDRH1 consisting of SEQ ID NO: 1;
(b) a CDRH2 consisting of SEQ ID NO: 3;
(c) a CDRH3 consisting of SEQ ID NO: 5;
(d) a CDRL1 consisting of SEQ ID NO: 2;
(e) a CDRL2 consisting of SEQ ID NO: 4; and
(f) a CDRL3 consisting of SEQ ID NO: 6.

In some embodiments, the isolated antibody or the antigen-binding portion thereof comprises:
(a) a CDRH1 comprising SEQ ID NO: 7;
(b) a CDRH2 comprising SEQ ID NO: 9;
(c) a CDRH3 comprising SEQ ID NO: 11;
(d) a CDRL1 comprising SEQ ID NO: 8;
(e) a CDRL2 comprising SEQ ID NO: 10; and
(f) a CDRL3 comprising SEQ ID NO: 12.

In a specific embodiment, the isolated antibody or the antigen-binding portion thereof comprises:
(a) a CDRH1 consisting of SEQ ID NO: 7;
(b) a CDRH2 consisting of SEQ ID NO: 9;
(c) a CDRH3 consisting of SEQ ID NO: 11;
(d) a CDRL1 consisting of SEQ ID NO: 8;
(e) a CDRL2 consisting of SEQ ID NO: 10; and
(f) a CDRL3 consisting of SEQ ID NO: 12.

In some embodiments, the isolated antibody or the antigen-binding portion thereof comprises:
(a) a CDRH1 comprising SEQ ID NO: 13;
(b) a CDRH2 comprising SEQ ID NO: 9;
(c) a CDRH3 comprising SEQ ID NO: 11;
(d) a CDRL1 comprising SEQ ID NO: 14;
(e) a CDRL2 comprising SEQ ID NO: 10; and
(f) a CDRL3 comprising SEQ ID NO: 12.

In a specific embodiment, the isolated antibody or the antigen-binding portion thereof comprises:
(a) a CDRH1 consisting of SEQ ID NO: 13;
(b) a CDRH2 consisting of SEQ ID NO: 9;
(c) a CDRH3 consisting of SEQ ID NO: 11;
(d) a CDRL1 consisting of SEQ ID NO: 14;
(e) a CDRL2 consisting of SEQ ID NO: 10; and
(f) a CDRL3 consisting of SEQ ID NO: 12.

In some embodiments, the isolated antibody or the antigen-binding portion thereof comprises:
(a) a CDRH1 comprising SEQ ID NO: 15;
(b) a CDRH2 comprising SEQ ID NO: 17;
(c) a CDRH3 comprising SEQ ID NO: 19;
(d) a CDRL1 comprising SEQ ID NO: 16;
(e) a CDRL2 comprising SEQ ID NO: 18; and
(f) a CDRL3 comprising SEQ ID NO: 20.

In a specific embodiment, the isolated antibody or the antigen-binding portion thereof comprises:
(a) a CDRH1 consisting of SEQ ID NO: 15;
(b) a CDRH2 consisting of SEQ ID NO: 17;
(c) a CDRH3 consisting of SEQ ID NO: 19;
(d) a CDRL1 consisting of SEQ ID NO: 16;
(e) a CDRL2 consisting of SEQ ID NO: 18; and
(f) a CDRL3 consisting of SEQ ID NO: 20.

In some embodiments, the isolated antibody or the antigen-binding portion thereof comprises:
(a) a CDRH1 comprising SEQ ID NO: 21;
(b) a CDRH2 comprising SEQ ID NO: 23;
(c) a CDRH3 comprising SEQ ID NO: 25;
(d) a CDRL1 comprising SEQ ID NO: 22;
(e) a CDRL2 comprising SEQ ID NO: 24; and
(f) a CDRL3 comprising SEQ ID NO: 26.

In a specific embodiment, the isolated antibody or the antigen-binding portion thereof comprises:
(a) a CDRH1 consisting of SEQ ID NO: 21;
(b) a CDRH2 consisting of SEQ ID NO: 23;
(c) a CDRH3 consisting of SEQ ID NO: 25;
(d) a CDRL1 consisting of SEQ ID NO: 22;
(e) a CDRL2 consisting of SEQ ID NO: 24; and
(f) a CDRL3 consisting of SEQ ID NO: 26.

In some embodiments, the isolated antibody or the antigen-binding portion thereof comprises:
(a) a CDRH1 comprising SEQ ID NO: 27;
(b) a CDRH2 comprising SEQ ID NO: 29;
(c) a CDRH3 comprising SEQ ID NO: 31;
(d) a CDRL1 comprising SEQ ID NO: 28;
(e) a CDRL2 comprising SEQ ID NO: 30; and
(f) a CDRL3 comprising SEQ ID NO: 32.

In a specific embodiment, the isolated antibody or the antigen-binding portion thereof comprises:
(a) a CDRH1 consisting of SEQ ID NO: 27;
(b) a CDRH2 consisting of SEQ ID NO: 29;
(c) a CDRH3 consisting of SEQ ID NO: 31;
(d) a CDRL1 consisting of SEQ ID NO: 28;
(e) a CDRL2 consisting of SEQ ID NO: 30; and
(f) a CDRL3 consisting of SEQ ID NO: 32.

### Anti-OX40 antibodies comprising α heavy chain variable region and a light chain variable region

In some embodiments, the isolated antibody or the antigen-binding portion thereof comprises:
(A) a heavy chain variable region:
   (i) comprising the amino acid sequence selected from the group consisting of SEQ ID NO: 33, 35, 37, 39, 41 and 43;
   (ii) comprising an amino acid sequence at least 85%, 90%, or 95% identical to the amino acid sequence selected from the group consisting of SEQ ID NO: 33, 35, 37, 39, 41 and 43; or
   (iii) comprising an amino acid sequence with addition, deletion and/or substitution of one or more amino acids compared with the amino acid sequence selected from the group consisting of SEQ ID NO: 33, 35, 37, 39, 41 and 43; and/or
(B) a light chain variable region:
   (i) comprising the amino acid sequence selected from the group consisting of SEQ ID NO: 34, 36, 38, 40, 42 and 44;
   (ii) comprising an amino acid sequence at least 85%, at least 90%, or at least 95% identical to the amino acid sequence selected from the group consisting of SEQ ID NO: 34, 36, 38, 40, 42 and 44; or
   (iii) comprising an amino acid sequence with addition, deletion and/or substitution of one or more amino acids compared with the amino acid sequence selected from the group consisting of SEQ ID NO: 34, 36, 38, 40, 42 and 44.

In a specific embodiment, the isolated antibody or the antigen-binding portion thereof comprises a heavy chain variable region consisting of the amino acid sequence of SEQ ID NO: 33 and a light chain variable region consisting of the amino acid sequence of SEQ ID NO: 34.

In a specific embodiment, the isolated antibody or the antigen-binding portion thereof comprises a heavy chain variable region consisting of the amino acid sequence of SEQ ID NO: 35 and a light chain variable region consisting of the amino acid sequence of SEQ ID NO: 36.

In a specific embodiment, the isolated antibody or the antigen-binding portion thereof comprises a heavy chain variable region consisting of the amino acid sequence of SEQ ID NO: 37 and a light chain variable region consisting of the amino acid sequence of SEQ ID NO: 38.

In a specific embodiment, the isolated antibody or the antigen-binding portion thereof comprises a heavy chain variable region consisting of the amino acid sequence of SEQ ID NO: 39 and a light chain variable region consisting of the amino acid sequence of SEQ ID NO: 40.

In a specific embodiment, the isolated antibody or the antigen-binding portion thereof comprises a heavy chain variable region consisting of the amino acid sequence of SEQ ID NO: 41 and a light chain variable region consisting of the amino acid sequence of SEQ ID NO: 42.

In a specific embodiment, the isolated antibody or the antigen-binding portion thereof comprises a heavy chain variable region consisting of the amino acid sequence of SEQ ID NO: 43 and a light chain variable region consisting of the amino acid sequence of SEQ ID NO: 44.

In other embodiments, the amino acid sequences of the heavy chain variable region and/or the light chain variable region can be at least 85%, 86%, 87%, 88%, 89%, 90%, 91%, 92%, 93%, 94%, 95%, 96%, 97%, 98% or 99% identical to the respective sequences set forth above. As an illustrative example, the antibody may comprise a heavy chain variable region with at least 85%, 86%, 87%, 88%, 89%, 90%, 91%, 92%, 93%, 94%, 95%, 96%, 97%, 98% or 99% sequence identity to a heavy chain variable region consisting of the amino acid sequence of SEQ ID NO: 33.

In some further embodiments, the isolated antibody or the antigen-binding portion thereof may contain conservative substitution or modification of amino acids in the variable regions of the heavy chain and/or light chain. It is understood in the art that certain conservative sequence modification can be made which do not remove antigen binding. See, e.g., Brummell et al. (1993) Biochem 32:1180-8; de Wildt et al. (1997) Prot. Eng. 10:835-41; Komissarov et al. (1997) J. Biol. Chem. 272:26864- 26870; Hall et al. (1992) J. Immunol. 149:1605-12; Kelley and O' Connell (1993) Biochem. 32:6862-35; Adib-Conquy et al. (1998) Int. Immunol. 10:341-6 and Beers et al. (2000) Clin. Can. Res. 6:2835-43.

As described above, the term "conservative substitution", as used herein, refers to amino acid substitutions which would not disadvantageously affect or change the essential properties of a protein/polypeptide comprising the amino acid sequence. For example, a conservative substitution may be introduced by standard techniques known in the art such as site-directed mutagenesis and PCR-mediated mutagenesis. Conservative amino acid substitutions include substitutions wherein an amino acid residue is substituted with another amino acid residue having a similar side chain, for example, a residue physically or functionally similar (such as, having similar size, shape, charge, chemical property including the capability of forming covalent bond or hydrogen bond, etc.) to the corresponding amino acid residue. The families of amino acid residues having similar side chains have been defined in the art. These families include amino acids having alkaline side chains (for example, lysine, arginine and histidine), amino acids having acidic side chains (for example, aspartic acid and glutamic acid), amino acids having uncharged polar side chains (for example, glycine, asparagine, glutamine, serine, threonine, tyrosine, cysteine, tryptophan), amino acids having nonpolar side chains (for example, alanine, valine, leucine, isoleucine, proline, phenylalanine, methionine), amino acids having β-branched side chains (such as threonine, valine, isoleucine) and amino acids having aromatic side chains (for example, tyrosine, phenylalanine, tryptophan, histidine). Therefore, a corresponding amino acid residue is preferably substituted with another amino acid residue from the same side-chain family. Methods for identifying amino acid conservative substitutions are well known in the art (see, for example, Brummell et al., Biochem. 32: 1180-1187 (1993); Kobayashi et al., Protein Eng. 12(10): 879-884 (1999); and Burks et al., Proc. Natl. Acad. Sci. USA 94: 412-417 (1997)).

### Binning and epitope mapping

It will further be appreciated the disclosed antibodies will associate with, or bind to, discrete epitopes or immunogenic determinants presented by the selected target or fragment thereof. In some embodiments, epitope or immunogenic determinants include chemically active surface groupings of molecules such as amino acids, sugar side chains, phosphoryl groups, or sulfonyl groups. In some embodiments, epitopes may have specific three-dimensional structural characteristics, and/or specific charge characteristics. Thus, as used herein the term "epitope" includes any protein determinant capable of specific binding to an immunoglobulin or T-cell receptor or otherwise interacting with a molecule. In some embodiments, an antibody is said to specifically bind (or immune-specifically bind or react) an antigen when it preferentially recognizes its target antigen in a complex mixture of proteins and/or macromolecules. In some embodiments, an antibody is said to specifically bind an antigen when the equilibrium dissociation constant (K_{D}) is less than or equal to 10⁻⁶ M or less than or equal to 10⁻⁷ M, more preferably when the e K_{D} is less than or equal to 10⁻⁸ M, and even more preferably when the K_{D} is less than or equal to 10⁻⁹ M.

Epitopes formed from contiguous amino acids (sometimes referred to as "linear" or "continuous" epitopes) are typically retained upon protein denaturing, whereas epitopes formed by tertiary folding are typically lost upon protein denaturing. In any event an antibody epitope typically includes at least 3, and more usually, at least 5 or 8-10 amino acids in a unique spatial conformation.

In this respect, it will be appreciated that, in some embodiments, an epitope may be associated with, or reside in, one or more regions, domains or motifs of, for example, the PD-1 protein. Similarly, the art-recognized term "motif' will be used in accordance with its common meaning and shall generally refer to a short, conserved region of a protein that is typically ten to twenty contiguous amino acid residues.

In any event once a desired epitope on an antigen is determined, it is possible to generate antibodies to that epitope, e.g., by immunizing with a peptide comprising the epitope using techniques described in the present invention. Alternatively, during the discovery process, the generation and characterization of antibodies may elucidate information about desirable epitopes located in specific domains or motifs. From this information, it is then possible to competitively screen antibodies for binding to the same epitope. An approach to achieve this is to conduct competition studies to find antibodies that competitively bind with one another, i.e. the antibodies compete for binding to the antigen. A high throughput process for binning antibodies based upon their cross-competition is described in WO 03/48731. Other methods of binning or domain level or epitope mapping comprising antibody competition or antigen fragment expression on yeast are well known in the art.

As used herein, the term "binning" refers to methods used to group or classify antibodies based on their antigen binding characteristics and competition. While the techniques are useful for defining and categorizing the antibodies of the instant invention, the bins do not always directly correlate with epitopes and such initial determinations of epitope binding may be further refined and confirmed by other art-recognized methodology in the art and as described herein. However, it will be appreciated that empirical assignment of the antibodies to individual bins provides information that may be indicative of the therapeutic potential of the disclosed antibodies.

More specifically, one can determine whether a selected reference antibody (or fragment thereof) binds to the same epitope or cross competes for binding with a second test antibody (i.e., is in the same bin) by using methods known in the art and set forth in the Examples herein.

Other compatible epitope mapping techniques include alanine scanning mutants, peptide blots (Reineke (2004) Methods Mol Biol 248:443-63), or peptide cleavage analysis. In addition, methods such as epitope excision, epitope extraction and chemical modification of antigens can be employed (Tomer (2000) Protein Science 9: 487-496).

### Nucleic Acid Molecules Encoding Antibodies of the Invention

In some aspects, the invention is directed to an isolated nucleic acid molecule, comprising a nucleic acid sequence encoding the heavy chain variable region and/or the light chain variable region of the isolated antibody as disclosed herein.

Nucleic acids of the invention can be obtained using standard molecular biology techniques. For antibodies expressed by hybridomas (e.g., hybridomas prepared from transgenic mice carrying human immunoglobulin genes as described further below), cDNAs encoding the light and heavy chains of the antibody made by the hybridoma can be obtained by standard PCR amplification or cDNA cloning techniques. For antibodies obtained from an immunoglobulin gene library (e.g., using phage display techniques), a nucleic acid encoding such antibodies can be recovered from the gene library.

The isolated nucleic acid encoding the VH region can be converted to a full-length heavy chain gene by operatively linking the VH-encoding nucleic acid to another DNA molecule encoding heavy chain constant regions (CH1, CH2 and CH3). The sequences of human heavy chain constant region genes are known in the art (see e.g., Kabat et al. (1991), supra) and DNA fragments encompassing these regions can be obtained by standard PCR amplification. The heavy chain constant region can be an IgG1,IgG2, IgG3, IgG4, IgA, IgE, IgM or IgD constant region, but more preferably is an IgG1 or IgG4 constant region.

The isolated nucleic acid encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see e.g., Kabat et al., supra) and DNA fragments encompassing these regions can be obtained by standard PCR amplification. In preferred embodiments, the light chain constant region can be a kappa or lambda constant region.

Once DNA fragments encoding VH and VL segments are obtained, these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

In some embodiments, the invention is directed to an isolated nucleic acid molecule, comprising a nucleic acid sequence encoding the heavy chain variable region of the isolated antibody as disclosed herein.

In some specific embodiments, the isolated nucleic acid molecule encodes the heavy chain variable region of the isolated antibody and comprises a nucleic acid sequence selected from the group consisting of:
(A) a nucleic acid sequence that encodes a heavy chain variable region as set forth in SEQ ID NO: 33, 35, 37, 39, 41 and 43;
(B) a nucleic acid sequence as set forth in SEQ ID NO: 45, 47, 49, 51, 53 or 55; or
(C) a nucleic acid sequence that hybridized under high stringency conditions to the complementary strand of the nucleic acid sequence of (A) or (B).

For example, the nucleic acid molecule is consisted of SEQ ID NO: SEQ ID NO: 45, 47, 49, 51, 53 or 55. Alternatively, the nucleic acid molecule share an at least 80% (e.g. at least 85%, 86%, 87%, 88%, 89%, 90%, 91 %, 92%, 93%, 94%, 95%, 96%, 97%, 98%, or 99%) sequence identity to SEQ ID NO: SEQ ID NO: 45, 47, 49, 51, 53 or 55. In some specific embodiments, the percentage of identity is derived from the degeneracy of the genetic code, and the encoded protein sequences remain unchanged.

In some embodiments, the invention is directed to an isolated nucleic acid molecule, comprising a nucleic acid sequence encoding the light chain variable region of the isolated antibody as disclosed herein.

In some specific embodiments, the isolated nucleic acid molecule encodes the light chain variable region of the isolated antibody comprises a nucleic acid sequence selected from the group consisting of:
(A) a nucleic acid sequence that encodes a heavy chain variable region as set forth in SEQ ID NO: 34, 36, 38, 40, 42 or 44;
(B) a nucleic acid sequence as set forth in SEQ ID NO: 46, 48, 50, 52, 54 or 56; or
(C) a nucleic acid sequence that hybridized under high stringency conditions to the complementary strand of the nucleic acid sequence of (A) or (B).

For example, the nucleic acid molecule is consisted of SEQ ID NO: 46, 48, 50, 52, 54 or 56. Alternatively, the nucleic acid molecule share an at least 80% (e.g. at least 85%, 86%, 87%, 88%, 89%, 90%, 91%, 92%, 93%, 94%, 95%, 96%, 97%, 98%, or 99%) sequence identity to SEQ ID NO: 46, 48, 50, 52, 54 or 56. In some specific embodiments, the percentage of identity is derived from the degeneracy of the genetic code, and the encoded protein sequences remain unchanged.

Exemplary high stringency conditions include hybridization at 45°C in 5X SSPE and 45% formamide, and a final wash at 65°C in 0.1 X SSC. It is understood in the art that conditions of equivalent stringency can be achieved through variation of temperature and buffer, or salt concentration as described Ausubel, et al. (Eds.), Protocols in Molecular Biology, John Wiley & Sons (1994), pp. 6.0.3 to 6.4.10. Modifications in hybridization conditions can be empirically determined or precisely calculated based on the length and the percentage of guanosine/cytosine (GC) base pairing of the probe. The hybridization conditions can be calculated as described in Sambrook, et al, (Eds.), Molecular Cloning: A laboratory Manual. Cold Spring Harbor Laboratory Press: Cold Spring Harbor, New York (1989), pp. 9.47 to 9.51.

### Pharmaceutical Compositions

In some aspects, the invention is directed to a pharmaceutical composition comprising at least one antibody or antigen-binding portion thereof as disclosed herein and a pharmaceutically acceptable carrier.

### Components of the compositions

The pharmaceutical composition may optionally contain one or more additional pharmaceutically active ingredients, such as another antibody or a drug. The pharmaceutical compositions of the invention also can be administered in a combination therapy with, for example, another immune-stimulatory agent, anti-cancer agent, an antiviral agent, or a vaccine, such that the anti-OX40 antibody enhances the immune response against the vaccine. A pharmaceutically acceptable carrier can include, for example, a pharmaceutically acceptable liquid, gel or solid carriers, an aqueous medium, a non-aqueous medium, an anti-microbial agent, isotonic agents, buffers, antioxidants, anesthetics, suspending/dispersing agent, a chelating agent, a diluent, adjuvant, excipient or a nontoxic auxiliary substance, other known in the art various combinations of components or more.

Suitable components may include, for example, antioxidants, fillers, binders, disintegrating agents, buffers, preservatives, lubricants, flavorings, thickening agents, coloring agents, emulsifiers or stabilizers such as sugars and cyclodextrin. Suitable anti-oxidants may include, for example, methionine, ascorbic acid, EDTA, sodium thiosulfate, platinum, catalase, citric acid, cysteine, mercapto glycerol, thioglycolic acid, Mercapto sorbitol, butyl methyl anisole, butylated hydroxy toluene and/or propylgalacte. As disclosed in the present invention, in a solvent containing an antibody or an antigen-binding fragment of the present invention discloses compositions include one or more anti-oxidants such as methionine, reducing antibody or antigen binding fragment thereof may be oxidized. The oxidation reduction may prevent or reduce a decrease in binding affinity, thereby enhancing antibody stability and extended shelf life. Thus, in some embodiments, the present invention provides a composition comprising one or more antibodies or antigen binding fragment thereof and one or more anti-oxidants such as methionine. The present invention further provides a variety of methods, wherein an antibody or antigen binding fragment thereof is mixed with one or more anti-oxidants, such as methionine, so that the antibody or antigen binding fragment thereof can be prevented from oxidation, to extend their shelf life and/or increased activity.

To further illustrate, pharmaceutical acceptable carriers may include, for example, aqueous vehicles such as sodium chloride injection, Ringer's injection, isotonic dextrose injection, sterile water injection, or dextrose and lactated Ringer's injection, nonaqueous vehicles such as fixed oils of vegetable origin, cottonseed oil, corn oil, sesame oil, or peanut oil, antimicrobial agents at bacteriostatic or fungistatic concentrations, isotonic agents such as sodium chloride or dextrose, buffers such as phosphate or citrate buffers, antioxidants such as sodium bisulfate, local anesthetics such as procaine hydrochloride, suspending and dispersing agents such as sodium carboxymethylcelluose, hydroxypropyl methylcellulose, or polyvinylpyrrolidone, emulsifying agents such as Polysorbate 80 (TWEEN-80), sequestering or chelating agents such as EDTA (ethylenediaminetetraacetic acid) or EGTA (ethylene glycol tetraacetic acid), ethyl alcohol, polyethylene glycol, propylene glycol, sodium hydroxide, hydrochloric acid, citric acid, or lactic acid. Antimicrobial agents utilized as carriers may be added to pharmaceutical compositions in multiple-dose containers that include phenols or cresols, mercurials, benzyl alcohol, chlorobutanol, methyl and propyl p-hydroxybenzoic acid esters, thimerosal, benzalkonium chloride and benzethonium chloride. Suitable excipients may include, for example, water, saline, dextrose, glycerol, or ethanol. Suitable non-toxic auxiliary substances may include, for example, wetting or emulsifying agents, pH buffering agents, stabilizers, solubility enhancers, or agents such as sodium acetate, sorbitan monolaurate, triethanolamine oleate, or cyclodextrin.

### Administration, Formulation and Dosage

The pharmaceutical composition of the invention may be administered *in vivo,* to a subject in need thereof, by various routes, including, but not limited to, oral, intravenous, intra-arterial, subcutaneous, parenteral, intranasal, intramuscular, intracranial, intracardiac, intraventricular, intratracheal, buccal, rectal, intraperitoneal, intradermal, topical, transdermal, and intrathecal, or otherwise by implantation or inhalation. The subject compositions may be formulated into preparations in solid, semi-solid, liquid, or gaseous forms; including, but not limited to, tablets, capsules, powders, granules, ointments, solutions, suppositories, enemas, injections, inhalants, and aerosols. The appropriate formulation and route of administration may be selected according to the intended application and therapeutic regimen.

Suitable formulations for enteral administration include hard or soft gelatin capsules, pills, tablets, including coated tablets, elixirs, suspensions, syrups or inhalations and controlled release forms thereof.

Formulations suitable for parenteral administration (e.g., by injection), include aqueous or non-aqueous, isotonic, pyrogen-free, sterile liquids (e.g., solutions, suspensions), in which the active ingredient is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid) of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. Similarly, the particular dosage regimen, including dose, timing and repetition, will depend on the particular individual and that individual's medical history, as well as empirical considerations such as pharmacokinetics (e.g., half-life, clearance rate, etc.).

Frequency of administration may be determined and adjusted over the course of therapy, and is based on reducing the number of proliferative or tumorigenic cells, maintaining the reduction of such neoplastic cells, reducing the proliferation of neoplastic cells, or delaying the development of metastasis. In some embodiments, the dosage administered may be adjusted or attenuated to manage potential side effects and/or toxicity. Alternatively, sustained continuous release formulations of a subject therapeutic composition may be appropriate.

It will be appreciated by one of skill in the art that appropriate dosages can vary from patient to patient. Determining the optimal dosage will generally involve the balancing of the level of therapeutic benefit against any risk or deleterious side effects. The selected dosage level will depend on a variety of factors including, but not limited to, the activity of the particular compound, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound and route of administration will ultimately be at the discretion of the physician, veterinarian, or clinician, although generally the dosage will be selected to achieve local concentrations at the site of action that achieve the desired effect without causing substantial harmful or deleterious side-effects.

In general, the antibody or the antigen binding portion thereof of the invention may be administered in various ranges. These include about 5 µg/kg body weight to about 100 mg/kg body weight per dose; about 50 µg/kg body weight to about 5 mg/kg body weight per dose; about 100 µg/kg body weight to about 10 mg/kg body weight per dose. Other ranges include about 100 µg/kg body weight to about 20 mg/kg body weight per dose and about 0.5 mg/kg body weight to about 20 mg/kg body weight per dose. In certain embodiments, the dosage is at least about 100 µg/kg body weight, at least about 250 µg/kg body weight, at least about 750 µg/kg body weight, at least about 3 mg/kg body weight, at least about 5 mg/kg body weight, at least about 10 mg/kg body weight.

In any event, the antibody or the antigen binding portion thereof of the invention is preferably administered as needed to subjects in need thereof. Determination of the frequency of administration may be made by persons skilled in the art, such as an attending physician based on considerations of the condition being treated, age of the subject being treated, severity of the condition being treated, general state of health of the subject being treated and the like.

In certain preferred embodiments, the course of treatment involving the antibody or the antigen-binding portion thereof of the instant invention will comprise multiple doses of the selected drug product over a period of weeks or months. More specifically, the antibody or the antigen-binding portion thereof of the instant invention may be administered once every day, every two days, every four days, every week, every ten days, every two weeks, every three weeks, every month, every six weeks, every two months, every ten weeks or every three months. In this regard, it will be appreciated that the dosages may be altered or the interval may be adjusted based on patient response and clinical practices.

Dosages and regimens may also be determined empirically for the disclosed therapeutic compositions in individuals who have been given one or more administration(s). For example, individuals may be given incremental dosages of a therapeutic composition produced as described herein. In selected embodiments, the dosage may be gradually increased or reduced or attenuated based respectively on empirically determined or observed side effects or toxicity. To assess efficacy of the selected composition, a marker of the specific disease, disorder or condition can be followed as described previously. For cancer, these include direct measurements of tumor size via palpation or visual observation, indirect measurement of tumor size by x-ray or other imaging techniques; an improvement as assessed by direct tumor biopsy and microscopic examination of the tumor sample; the measurement of an indirect tumor marker (e.g., PSA for prostate cancer) or a tumorigenic antigen identified according to the methods described herein, a decrease in pain or paralysis; improved speech, vision, breathing or other disability associated with the tumor; increased appetite; or an increase in quality of life as measured by accepted tests or prolongation of survival. It will be apparent to one of skill in the art that the dosage will vary depending on the individual, the type of neoplastic condition, the stage of neoplastic condition, whether the neoplastic condition has begun to metastasize to other location in the individual, and the past and concurrent treatments being used.

Compatible formulations for parenteral administration (e.g., intravenous injection) will comprise the antibody or antigen-binding portion thereof as disclosed herein in concentrations of from about 10 µg/ml to about 100 mg/ml. In certain selected embodiments, the concentrations of the antibody or the antigen binding portion thereof will comprise 20 µg/ml, 40 µg/ml, 60 µg/ml, 80 µg/ml, 100 µg/ml, 200 µg/ml, 300, ng/ml, 400 ng/ml, 500 ng/ml, 600 µg/ml, 700 µg/ml, 800 µg/ml, 900 µg/ml or 1 mg/ml. In other preferred embodiments ADC concentrations will comprise 2 mg/ml, 3 mg/ml, 4 mg/ml, 5 mg/ml, 6 mg/ml, 8 mg/ml, 10 mg/ml, 12 mg/ml, 14 mg/ml, 16 mg/ml, 18 mg/ml, 20 mg/ml, 25 mg/ml, 30 mg/ml, 35 mg/ml, 40 mg/ml, 45 mg/ml, 50 mg/ml, 60 mg/ml, 70 mg/ml, 80 mg/ml, 90 mg/ml or 100 mg/ml

### Applications of the Invention

The antibodies, antibody compositions and methods of the present invention have numerous in vitro and in vivo utilities involving, for example, detection of OX40 or enhancement of immune response. For example, these molecules can be administered to cells in culture, in vitro or ex vivo, or to human subjects, e.g., in vivo, to enhance immunity in a variety of situations. The immune response can be modulated, for instance, augmented, stimulated or up-regulated.

Preferred subjects include human patients in need of enhancement of an immune response. The methods are particularly suitable for treating human patients having a disorder that can be treated by augmenting an immune response (e.g., the T-cell mediated immune response). In a particular embodiment, the methods are particularly suitable for treatment of cancer in vivo. To achieve antigen- specific enhancement of immunity, the anti-OX40 antibodies can be administered together with an antigen of interest or the antigen may already be present in the subject to be treated (e.g., a tumor-bearing or virus-bearing subject). When antibodies to OX40 are administered together with another agent, the two can be administered in either order or simultaneously.

The invention further provides methods for detecting the presence of human OX40 antigen in a sample, or measuring the amount of human OX40 antigen, comprising contacting the sample, and a control sample, with a human monoclonal antibody, or an antigen binding portion thereof, which specifically binds to human OX40, under conditions that allow for formation of a complex between the antibody or portion thereof and human OX40. The formation of a complex is then detected, wherein a difference complex formation between the sample compared to the control sample is indicative of the presence of human OX40 antigen in the sample. Moreover, the anti-OX40 antibodies of the invention can be used to purify human OX40 via immunoaffinity purification.

### Treatment of disorders including cancers

In some aspects, the present invention provides a method of treating a disorder in a mammal, which comprises administering to the subject (for example, a human) in need of treatment a therapeutically effective amount of the antibody or antigen-binding portion thereof as disclosed herein. For example, the disorder is a cancer.

A variety of cancers where OX40 is implicated, whether malignant or benign and whether primary or secondary, may be treated or prevented with a method provided by the disclosure. The cancers may be solid cancers or hematologic malignancies. Examples of such cancers include lung cancers such as bronchogenic carcinoma (e.g., squamous cell carcinoma, small cell carcinoma, large cell carcinoma, and adenocarcinoma), alveolar cell carcinoma, bronchial adenoma, chondromatous hamartoma (noncancerous), and sarcoma (cancerous); heart cancer such as myxoma, fibromas, and rhabdomyomas; bone cancers such as osteochondromas, condromas, chondroblastomas, chondromyxoid fibromas, osteoid osteomas, giant cell tumors, chondrosarcoma, multiple myeloma, osteosarcoma, fibrosarcomas, malignant fibrous histiocytomas, Ewing's tumor (Ewing's sarcoma), and reticulum cell sarcoma; brain cancer such as gliomas (e.g., glioblastoma multiforme), anaplastic astrocytomas, astrocytomas, oligodendrogliomas, medulloblastomas, chordoma, Schwannomas, ependymomas, meningiomas, pituitary adenoma, pinealoma, osteomas, hemangioblastomas, craniopharyngiomas, chordomas, germinomas, teratomas, dermoid cysts, and angiomas; cancers in digestive system such as colon cancer, leiomyoma, epidermoid carcinoma, adenocarcinoma, leiomyosarcoma, stomach adenocarcinomas, intestinal lipomas, intestinal neurofibromas, intestinal fibromas, polyps in large intestine, and colorectal cancers; liver cancers such as hepatocellular adenomas, hemangioma, hepatocellular carcinoma, fibrolamellar carcinoma, cholangiocarcinoma, hepatoblastoma, and angiosarcoma; kidney cancers such as kidney adenocarcinoma, renal cell carcinoma, hypernephroma, and transitional cell carcinoma of the renal pelvis; bladder cancers; hematological cancers such as acute lymphocytic (lymphoblastic) leukemia, acute myeloid (myelocytic, myelogenous, myeloblasts, myelomonocytic) leukemia, chronic lymphocytic leukemia (e.g., Sezary syndrome and hairy cell leukemia), chronic myelocytic (myeloid, myelogenous, granulocytic) leukemia, Hodgkin's lymphoma, non-Hodgkin's lymphoma, B cell lymphoma, mycosis fungoides, and myeloproliferative disorders (including myeloproliferative disorders such as polycythemia vera, myelofibrosis, thrombocythemia, and chronic myelocytic leukemia); skin cancers such as basal cell carcinoma, squamous cell carcinoma, melanoma, Kaposi's sarcoma, and Paget's disease; head and neck cancers; eye-related cancers such as retinoblastoma and intraoccular melanocarcinoma; male reproductive system cancers such as benign prostatic hyperplasia, prostate cancer, and testicular cancers (e.g., seminoma, teratoma, embryonal carcinoma, and choriocarcinoma); breast cancer; female reproductive system cancers such as uterine cancer (endometrial carcinoma), cervical cancer (cervical carcinoma), cancer of the ovaries (ovarian carcinoma), vulvar carcinoma, vaginal carcinoma, fallopian tube cancer, and hydatidiform mole; thyroid cancer (including papillary, follicular, anaplastic, or medullary cancer); pheochromocytomas (adrenal gland); noncancerous growths of the parathyroid glands; pancreatic cancers; and hematological cancers such as leukemias, myelomas, non-Hodgkin's lymphomas, and Hodgkin's lymphomas. In a specific embodiment, the cancer is colon cancer.

In some embodiments, examples of cancer include but not limited to B-cell lymphoma (including low grade/follicular non-Hodgkin's lymphoma (NHL); small lymphocytic (SL) NHL; intermediate grade/follicular NHL; intermediate grade diffuse NHL; high grade immunoblastic NHL; high grade lymphoblastic NHL; high grade small non-cleaved cell NHL; bulky disease NHL; mantle cell lymphoma; AIDS-related lymphoma; and Waldenstrom's Macroglobulinemia; chronic lymphocytic leukemia (CLL); acute lymphoblastic leukemia (ALL); Hairy cell leukemia; chronic myeloblastic leukemia; and post-transplant lymphoproliierative disorder (PTLD), as well as abnormal vascular proliferation associated with phakomatoses, edema (such as that associated with brain tumors), B-cell proliferative disorders, and Meigs' syndrome. More specific examples include, but are not limited to, relapsed or refractory NHL, front line low grade NHL, Stage III/IV NHL, chemotherapy resistant NHL, precursor B lymphoblastic leukemia and/or lymphoma, small lymphocytic lymphoma, B-cell chronic lymphocytic leukemia and/or prolymphocytic leukemia and/or small lymphocytic lymphoma, B-cell prolymphocytic lymphoma, immunocytoma and/or lymphoplasmacytic lymphoma, lymphoplasmacytic lymphoma, marginal zone B-cell lymphoma, splenic marginal zone lymphoma, extranodal marginal zone-MALT lymphoma, nodal marginal zone lymphoma, hairy cell leukemia, plasmacytoma and/or plasma cell myeloma, low grade/follicular lymphoma, intermediate grade/follicular NHL, mantle cell lymphoma, follicle center lymphoma (follicular), intermediate grade diffuse NHL, diffuse large B-cell lymphoma, aggressive NHL (including aggressive front-line NHL and aggressive relapsed NHL), NHL relapsing after or refractory to autologous stem cell transplantation, primary mediastinal large B-cell lymphoma, primary effusion lymphoma, high grade immunoblastic NHL, high grade lymphoblastic NHL, high grade small non-cleaved cell NHL, bulky disease NHL, Burkitt's lymphoma, precursor (peripheral) large granular lymphocytic leukemia, mycosis fungoides and/or Sezary syndrome, skin (cutaneous) lymphomas, anaplastic large cell lymphoma, angiocentric lymphoma.

In some embodiments, examples of cancer further include, but are not limited to, B-cell proliferative disorders, which further include, but are not limited to, lymphomas (e.g., B-Cell Non- Hodgkin's lymphomas (NHL)) and lymphocytic leukemias. Such lymphomas and lymphocytic leukemias include e.g. a) follicular lymphomas, b) Small Non-Cleaved Cell Lymphomas/ Burkitt's lymphoma (including endemic Burkitt's lymphoma, sporadic Burkitt's lymphoma and Non-Burkitt's lymphoma), c) marginal zone lymphomas (including extranodal marginal zone B-cell lymphoma (Mucosa-associated lymphatic tissue lymphomas, MALT), nodal marginal zone B-cell lymphoma and splenic marginal zone lymphoma), d) Mantle cell lymphoma (MCL), e) Large Cell Lymphoma (including B-cell diffuse large cell lymphoma (DLCL), Diffuse Mixed Cell Lymphoma, Immunoblastic Lymphoma, Primary Mediastinal B-Cell Lymphoma, Angiocentric Lymphoma- Pulmonary B-Cell Lymphoma), f) hairy cell leukemia, g ) lymphocytic lymphoma, Waldenstrom's macroglobulinemia, h) acute lymphocytic leukemia (ALL), chronic lymphocytic leukemia (CLL)/ small lymphocytic lymphoma (SLL), B cell prolymphocytic leukemia, i) plasma cell neoplasms, plasma cell myeloma, multiple myeloma, plasmacytoma, and/or j) Hodgkin's disease.

In some other embodiments, the disorder is an autoimmune disease. Examples of autoimmune diseases that may be treated with the antibody or antigen-binding portion thereof include autoimmune encephalomyelitis, lupus erythematosus, and rheumatoid arthritis. The antibody or the antigen-binding portion thereof may also be used to treat or prevent infectious disease, inflammatory disease (such as allergic asthma) and chronic graft-versus-host disease.

### Stimulation of an immune response

In some aspects, the invention also provides a method of enhancing (for example, stimulating) an immune response in a subject comprising administering an antibody or an antigen binding portion thereof of the invention to the subject such that an immune response in the subject is enhanced. For example, the subject is a mammal. In a specific embodiment, the subject is a human.

The term "enhancing an immune response" or its grammatical variations, means stimulating, evoking, increasing, improving, or augmenting any response of a mammal's immune system. The immune response may be a cellular response (i.e. cell-mediated, such as cytotoxic T lymphocyte mediated) or a humoral response (i.e. antibody mediated response), and may be a primary or secondary immune response. Examples of enhancement of immune response include increased CD4⁺ helper T cell activity and generation of cytolytic T cells. The enhancement of immune response can be assessed using a number of in vitro or in vivo measurements known to those skilled in the art, including, but not limited to, cytotoxic T lymphocyte assays, release of cytokines (for example IL-2 production or IFN-γ production), regression of tumors, survival of tumor bearing animals, antibody production, immune cell proliferation, expression of cell surface markers, and cytotoxicity. Typically, methods of the disclosure enhance the immune response by a mammal when compared to the immune response by an untreated mammal or a mammal not treated using the methods as disclosed herein. In one embodiment, the antibody or an antigen binding portion thereof is used to enhance the immune response of a human to a microbial pathogen (such as a virus). In another embodiment, the antibody or an antigen binding portion thereof is used to enhance the immune response of a human to a vaccine. In one embodiment, the method enhances a cellular immune response, particularly a cytotoxic T cell response. In another embodiment, the cellular immune response is a T helper cell response. In still another embodiment, the immune response is a cytokine production, particularly IFN-γ production or IL-2 production. The antibody or an antigen binding portion thereof may be used to enhance the immune response of a human to a microbial pathogen (such as a virus) or to a vaccine.

The antibody or the antigen-binding portion thereof may be used alone as a monotherapy, or may be used in combination with chemical therapies or radiotherapies.

### Combined use with chemotherapies

The antibody or the antigen-binding portion thereof may be used in combination with an anti-cancer agent, a cytotoxic agent or chemotherapeutic agent.

The term "anti-cancer agent" or "anti-proliferative agent" means any agent that can be used to treat a cell proliferative disorder such as cancer, and includes, but is not limited to, cytotoxic agents, cytostatic agents, anti-angiogenic agents, debulking agents, chemotherapeutic agents, radiotherapy and radiotherapeutic agents, targeted anti-cancer agents, BRMs, therapeutic antibodies, cancer vaccines, cytokines, hormone therapies, radiation therapy and anti-metastatic agents and immunotherapeutic agents. It will be appreciated that, in selected embodiments as discussed above, such anti-cancer agents may comprise conjugates and may be associated with the disclosed site-specific antibodies prior to administration. More specifically, in certain embodiments selected anti-cancer agents will be linked to the unpaired cysteines of the engineered antibodies to provide engineered conjugates as set forth herein. Accordingly, such engineered conjugates are expressly contemplated as being within the scope of the instant invention. In other embodiments, the disclosed anti-cancer agents will be given in combination with site-specific conjugates comprising a different therapeutic agent as set forth above.

As used herein the term "cytotoxic agent" means a substance that is toxic to the cells and decreases or inhibits the function of cells and/or causes destruction of cells. In certain embodiments, the substance is a naturally occurring molecule derived from a living organism. Examples of cytotoxic agents include, but are not limited to, small molecule toxins or enzymatically active toxins of bacteria (e.g., Diptheria toxin, Pseudomonas endotoxin and exotoxin, Staphylococcal enterotoxin A), fungal (e.g., α-sarcin, restrictocin), plants (e.g., abrin, ricin, modeccin, viscumin, pokeweed anti-viral protein, saporin, gelonin, momoridin, trichosanthin, barley toxin, Aleurites fordii proteins, dianthin proteins, Phytolacca mericana proteins (PAPI, PAPII, and PAP-S), Momordica charantia inhibitor, curcin, crotin, saponaria officinalis inhibitor, gelonin, mitegellin, restrictocin, phenomycin, neomycin, and the tricothecenes) or animals, (e.g., cytotoxic RNases, such as extracellular pancreatic RNases; DNase I, including fragments and/or variants thereof).

For the purposes of the instant invention a "chemotherapeutic agent" comprises a chemical compound that non-specifically decreases or inhibits the growth, proliferation, and/or survival of cancer cells (e.g., cytotoxic or cytostatic agents). Such chemical agents are often directed to intracellular processes necessary for cell growth or division, and are thus particularly effective against cancerous cells, which generally grow and divide rapidly. For example, vincristine depolymerizes microtubules, and thus inhibits cells from entering mitosis. In general, chemotherapeutic agents can include any chemical agent that inhibits, or is designed to inhibit, a cancerous cell or a cell likely to become cancerous or generate tumorigenic progeny (e.g., TIC). Such agents are often administered, and are often most effective, in combination, e.g., in regimens such as CHOP or FOLFIRI.

Examples of anti-cancer agents that may be used in combination with the site-specific constructs of the present invention (either as a component of a site specific conjugate or in an unconjugated state) include, but are not limited to, alkylating agents, alkyl sulfonates, aziridines, ethylenimines and methylamelamines, acetogenins, a camptothecin, bryostatin, callystatin, CC-1065, cryptophycins, dolastatin, duocarmycin, eleutherobin, pancratistatin, a sarcodictyin, spongistatin, nitrogen mustards, antibiotics, enediyne antibiotics, dynemicin, bisphosphonates, esperamicin, chromoprotein enediyne antiobiotic chromophores, aclacinomysins, actinomycin, authramycin, azaserine, bleomycins, cactinomycin, carabicin, carminomycin, carzinophilin, chromomycinis, dactinomycin, daunorubicin, detorubicin, 6-diazo-5-oxo-L-norleucine, ADRIAMYCIN^{®} doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites, erlotinib, vemurafenib, crizotinib,sorafenib, ibrutinib, enzalutamide, folic acid analogues, purine analogs, androgens, anti-adrenals, folic acid replenisher such as frolinic acid, aceglatone, aldophosphamide glycoside, aminolevulinic acid, eniluracil, amsacrine, bestrabucil, bisantrene, edatraxate, defofamine, demecolcine, diaziquone, elfornithine, elliptinium acetate, an epothilone, etoglucid, gallium nitrate, hydroxyurea, lentinan, lonidainine, maytansinoids, mitoguazone, mitoxantrone, mopidanmol, nitraerine, pentostatin, phenamet, pirarubicin, losoxantrone, podophyllinic acid, 2-ethylhydrazide, procarbazine, PSK^{®} polysaccharide complex (JHS Natural Products, Eugene, OR), razoxane; rhizoxin; sizofiran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, chloranbucil; GEMZAR^{®} gemcitabine; 6-thioguanine; mercaptopurine; methotrexate; platinum analogs, vinblastine; platinum; etoposide (VP-16); ifosfamide; mitoxantrone; vincristine; NAVELBINE^{®} vinorelbine; novantrone; teniposide; edatrexate; daunomycin; aminopterin; xeloda; ibandronate; irinotecan (Camptosar, CPT-11), topoisomerase inhibitor RFS 2000; difluorometlhylornithine; retinoids; capecitabine; combretastatin; leucovorin; oxaliplatin; inhibitors of PKC-alpha, Raf, H-Ras, EGFR and VEGF-A that reduce cell proliferation and pharmaceutically acceptable salts, acids or derivatives of any of the above. Also included in this definition are anti-hormonal agents that act to regulate or inhibit hormone action on tumors such as anti-estrogens and selective estrogen receptor modulators, aromatase inhibitors that inhibit the enzyme aromatase, which regulates estrogen production in the adrenal glands, and anti-androgens; as well as troxacitabine (a 1,3- dioxolane nucleoside cytosine analog); antisense oligonucleotides, ribozymes such as a VEGF expression inhibitor and a HER2 expression inhibitor; vaccines, PROLEUKIN^{®} rIL-2; LURTOTECAN^{®} topoisomerase 1 inhibitor; AbARELIX^{®} rmRH; Vinorelbine and Esperamicins and pharmaceutically acceptable salts, acids or derivatives of any of the above.

### Combined use with radiotherapies

The present invention also provides for the combination of the antibody or the antigen-binding portion thereof with radiotherapy (i.e., any mechanism for inducing DNA damage locally within tumor cells such as gamma-irradiation, X-rays, UV-irradiation, microwaves, electronic emissions and the like). Combination therapy using the directed delivery of radioisotopes to tumor cells is also contemplated, and the disclosed conjugates may be used in connection with a targeted anti-cancer agent or other targeting means. Typically, radiation therapy is administered in pulses over a period of time from about 1 to about 2 weeks. The radiation therapy may be administered to subjects having head and neck cancer for about 6 to 7 weeks. Optionally, the radiation therapy may be administered as a single dose or as multiple, sequential doses.

### Diagnosis

The invention provides *in vitro* and *in vivo* methods for detecting, diagnosing or monitoring proliferative disorders and methods of screening cells from a patient to identify tumor cells including tumorigenic cells. Such methods include identifying an individual having cancer for treatment or monitoring progression of a cancer, comprising contacting the patient or a sample obtained from a patient (either *in vivo* or *in vitro)* with an antibody as described herein and detecting presence or absence, or level of association, of the antibody to bound or free target molecules in the sample. In some embodiments, the antibody will comprise a detectable label or reporter molecule as described herein.

In some embodiments, the association of the antibody with particular cells in the sample can denote that the sample may contain tumorigenic cells, thereby indicating that the individual having cancer may be effectively treated with an antibody as described herein.

Samples can be analyzed by numerous assays, for example, radioimmunoassays, enzyme immunoassays (e.g. ELISA), competitive-binding assays, fluorescent immunoassays, immunoblot assays, Western Blot analysis and flow cytometry assays. Compatible *in vivo* theragnostic or diagnostic assays can comprise art recognized imaging or monitoring techniques, for example, magnetic resonance imaging, computerized tomography (e.g. CAT scan), positron tomography (e.g., PET scan), radiography, ultrasound, etc., as would be known by those skilled in the art.

### Pharmaceutical packs and kits

Pharmaceutical packs and kits comprising one or more containers, comprising one or more doses of the antibody or the antigen-binding portion thereof are also provided. In certain embodiments, a unit dosage is provided wherein the unit dosage contains a predetermined amount of a composition comprising, for example, the antibody or the antigen-binding portion thereof, with or without one or more additional agents. For other embodiments, such a unit dosage is supplied in single-use prefilled syringe for injection. In still other embodiments, the composition contained in the unit dosage may comprise saline, sucrose, or the like; a buffer, such as phosphate, or the like; and/or be formulated within a stable and effective pH range. Alternatively, in certain embodiments, the conjugate composition may be provided as a lyophilized powder that may be reconstituted upon addition of an appropriate liquid, for example, sterile water or saline solution. In certain preferred embodiments, the composition comprises one or more substances that inhibit protein aggregation, including, but not limited to, sucrose and arginine. Any label on, or associated with, the container(s) indicates that the enclosed conjugate composition is used for treating the neoplastic disease condition of choice.

The present invention also provides kits for producing single-dose or multi-dose administration units of site-specific conjugates and, optionally, one or more anti-cancer agents. The kit comprises a container and a label or package insert on or associated with the container. Suitable containers include, for example, bottles, vials, syringes, etc. The containers may be formed from a variety of materials such as glass or plastic and contain a pharmaceutically effective amount of the disclosed conjugates in a conjugated or unconjugated form. In other preferred embodiments, the container(s) comprise a sterile access port (for example the container may be an intravenous solution bag or a vial having a stopper pierceable by a hypodermic injection needle). Such kits will generally contain in a suitable container a pharmaceutically acceptable formulation of the engineered conjugate and, optionally, one or more anti-cancer agents in the same or different containers. The kits may also contain other pharmaceutically acceptable formulations, either for diagnosis or combined therapy. For example, in addition to the antibody or the antigen-binding portion thereof of the invention such kits may contain any one or more of a range of anti-cancer agents such as chemotherapeutic or radiotherapeutic drugs; anti-angiogenic agents; anti-metastatic agents; targeted anti-cancer agents; cytotoxic agents; and/or other anti-cancer agents.

More specifically the kits may have a single container that contains the disclosed the antibody or the antigen-binding portion thereof, with or without additional components, or they may have distinct containers for each desired agent. Where combined therapeutics are provided for conjugation, a single solution may be pre-mixed, either in a molar equivalent combination, or with one component in excess of the other. Alternatively, the conjugates and any optional anti-cancer agent of the kit may be maintained separately within distinct containers prior to administration to a patient. The kits may also comprise a second/third container means for containing a sterile, pharmaceutically acceptable buffer or other diluent such as bacteriostatic water for injection (BWFI), phosphate-buffered saline (PBS), Ringer's solution and dextrose solution.

When the components of the kit are provided in one or more liquid solutions, the liquid solution is preferably an aqueous solution, with a sterile aqueous or saline solution being particularly preferred. However, the components of the kit may be provided as dried powder(s). When reagents or components are provided as a dry powder, the powder can be reconstituted by the addition of a suitable solvent. It is envisioned that the solvent may also be provided in another container.

As indicated briefly above the kits may also contain a means by which to administer the antibody or the antigen-binding portion thereof and any optional components to a patient, e.g., one or more needles, I.V. bags or syringes, or even an eye dropper, pipette, or other such like apparatus, from which the formulation may be injected or introduced into the animal or applied to a diseased area of the body. The kits of the present invention will also typically include a means for containing the vials, or such like, and other component in close confinement for commercial sale, such as, e.g., injection or blow-molded plastic containers into which the desired vials and other apparatus are placed and retained.

### Sequence Listing Summary

Appended to the instant application is a sequence listing comprising a number of nucleic acid and amino acid sequences. The following Table A, B and C provides a summary of the included sequences.

Six illustrative antibodies as disclosed herein, which are fully human anti-OX40 monoclonal antibodies, are designated as "1.62.3-ul-IgGlK", "1.62.3-ul-3-IgGlK", "1.7.10-ul-IgGlK", "1.134.9-ul-IgGlL", "1.186.19-ul-IgGlK" and "1.214.23-ul-IgGlK", respectively.

**Table A**

| **CDR amino acid sequences** | | | | |
|---|---|---|---|---|
| Antibodv | | **CDR1** | **CDR2** | **CDR3** |
| 1.7.10-u1-IgG1K | **CDRH** | SEQ ID NO: 1 | SEQ ID NO: 3 | SEQ ID NO: 5 |
| | | GFTFSDYYMS | YISGSGNTIYYADSVKG | ERGAAGTGWFDP |
| | **CDRL** | SEQ ID NO: 2 | SEQ ID NO: 4 | SEQ ID NO: 6 |
| | | RASQGISSWLA | AASSLQG | QQVNSFPWT |
| 1.62.3-u1-IgGlK | **CDRH** | SEQ ID NO: 7 | SEQ ID NO: 9 | SEQ IDNO: 11 |
| | | GGSISNGGYYWS | YIYYSGSTYYNPSLKS | DEWELRGFDY |
| | **CDRL** | SEQ ID NO: 8 | SEQ ID NO: 10 | SEQ ID NO: 12 |
| | | KSSQSVVFSSNNKICLA | WSSTRES | QQYYSSPWT |
| 1.62.3-u1-3-IgG1K | **CDRH** | SEQ ID NO: 13 | SEQ ID NO: 9 | SEQ ID NO: 11 |
| | | GGSISNAGYYWS | YIYYSGSTYYNPSLKS | DEWELRGFDY |
| | **CDRL** | SEQ ID NO: 14 | SEQ ID NO: 10 | SEQ ID NO: 12 |
| | | KSSQSVVFSSNNKISLA | WSSTRES | QQYYSSPWT |
| 1.134.9-u1-IgG1L | **CDRH** | SEQ ID NO: 15 | SEQ ID NO: 17 | SEQ ID No: 19 |
| | | GGSISSYNWWS | EIYHGGNTNYNPSLKS | APGDWGGSPYFDF |
| | **CDRL** | SEQ ID NO: 16 | SEQ ID NO: 18 | SEQ ID NO: 20 |
| | | QGDNLRTYYAS | GRNKRPS | NSRDSSGNPVV |
| 1.186.19-u1-IgG1K | **CDRH** | SEQ ID NO: 21 | SEQ ID NO: 23 | SEQ ID NO: 25 |
| | | GFTFSDYYMG | YISGSGNTIYYADSVKG | ERGAAGAGWFDP |
| | **CDRL** | SEQ ID NO: 22 | SEQ ID NO: 24 | SEQ ID NO: 26 |
| | | RASQGISSWLA | AASSLQG | QQVNSFPWT |
| 1.214 23-u1-IgG1K | **CDRH** | SEQ ID NO: 27 | SEQ ID NO: 29 | SEQ ID NO: 31 |
| | | GGSISNRNWWS | EIFHSGNTNYNPSLKS | SFAVALDS |
| | **CDRL** | SEQ ID NO: 28 | SEQ ID NO: 30 | SEQ ID NO: 32 |
| | | RASQDINSYLA | AASSLQS | QQLFSYPIT |

**Table B**

| **Variable region amino acid sequences** | | |
|---|---|---|
| Antibody | **VH** | **VL** |
| 1.7.10-u1-IgG1K | SEQ ID NO:33 | SEQ ID NO: 34 |
| | QVHLVESGGGLVKPGGSLRLSCAASGFTFSDYYMSWIRQA PGKGLEWVSYISGSGNTIYYADSVKGRFTISRDNAKNSLYL QMNSLRADDTAVYFCARERGAAGTGWFDPWGQGTLVTVS S | DIQMTQSPSSVSASVGDRVTITCRASQGISSWLAWYQQK PGKAPKLLIYAASSLQGGVPSRFSGSGSGTDFTLTISSLQP EDFATYYCQQVNSFPWTFGQGTKVEIK |
| 1.62.3-u1-IgG1K | SEQ ID NO: 35 | SEQ ID NO: 36 |
| | QVQLQESGPGLVKPSQTLSLTCTVSGGSISNGGYYWSWIRQ HPGKGLEWIGYIYYSGSTYYNPSLKSRVTISVDTSKNQFSLK LSSVTAADTAVYYCARDEWELRGFDYWGQGTLVTVSS | DIVMTQSPDSLAVSLGERATINCKSSQSVVFSSNNKICLA WYQQKPGQPPKLLIYWSSTRESGVPDRFSGSGSGTDFTL TISSLQAEDVAVYYCQQYYSSPWTFGQGTKVEIK |
| 1.623-u1-3-IgG1K | SEQ ID NO: 37 | SEQ ID NO: 38 |
| | QVQLQESGPGLVKPSQTLSLTCTVSGGSISNAGYYWSWIRQ HPGKGLEWIGYIYYSGSTYYNPSLKSRVTISVDTSKNQFSLK LSSVTAADTAVYYCARDEWELRGFDYWGQGTLVTVSS | DIVMTQSPDSLAVSLGERATINCKSSQSVVFSSNNKISLA WYQQKPGQPPKLLIYWSSTRESGVPDRFSGSGSGTDFTL TISSLQAEDVAVYYCQQYYSSPWTFGQGTKVEIK |
| 1.134.9-u1-IgG1L | SEQ ID NO: 39 | SEQ ID NO: 40 |
| | QVQLQESGPGLVKPSGTLSLTCAVSGGSISSYNWWSWVRQ PPGKGLEWIGEIYHGGNTNYNPSLKSRVTMSVDNSKNQFSL KLSSVTAADTAVYYCARAPGDWGGSPYFDFWGQGTLVTV SS | SSELTQDPAVSVALGQTVRITCQGDNLRTYYASWYQQK PGQAPILLIYGRNKRPSGIPDRFSGSSSGNTASLTITGAQA EDEAAYYCNSRDSSGNPVVFGGGTKLTVL |
| 1.186.19-u1-IgG1K | SEQ ID NO: 41 | SEQ ID NO: 42 |
| | QVHLVESGGGLVKPGGSLRLSCAASGFTFSDYYMGWIRQA PGQGLEWVSYISGSGNTIYYADSVKGRFTISRDNAKNSLYL QMNSLRADDTAVYFCAKERGAAGAGWFDPWGQGTLVTV SS | DIQMTQSPSSVSASVGDRVTITCRASQGISSWLAWYQQK PGKAPKLLIYAASSLQGGVPSRFSGSGSGTDFTLTISSLQP EDFATYHCQQVNSFPWTFGQGTKVEIK |
| 1.214.23-u1-IgG1K | SEQ ID NO: 43 | SEQ ID NO: 44 |
| | QVQLQESGPGLVKPSGTLSLTCVVSGGSISNRNWWSWVRQ PPGKGLEWIGEIFHSGNTNYNPSLKSRVTISVDKSKNQFSLK VNSVTAADTAVYYCAKSFAVALDSWGQGTLVTVSS | DIQLTQSPSFLSASVGDRVTITCRASQDINSYLAWCQQKP GKAPKLLIYAASSLQSGVPSRFSGTGSGTEFTLTISSLQPE DFATYYCQQLFSYPITFGQGTRLEIK |

**Table C**

| **Variable region nucleotide sequences** | | |
|---|---|---|
| Antibody | **VHnu (heavy chain variable region nucleotide sequences)** | **VLnu (light chain variable region nucleotide sequences)** |
| 1.7.10-u1-IgG1K | SEQ ID NO: 45 | SEQ ID NO: 46 |
| | CAG GTG CAC CTG GTG GAG TCT GGG GGA GGC TTG GTC AAG CCT GGA GGG TCC CTG AGA CTC TCC TGT GCA GCC TCT GGA TTC ACC TTC AGT GAC TAC TAC ATG AGC TGG ATC CGC CAG GCT CCA GGG AAG GGG CTG GAG TGG GTT TCA TAC ATT AGT GGT AGT GGT AAC ACC ATT TAC TAC GCA GAC TCT GTG AAG GGC CGA TTC ACC ATC TCC AGG GAC AAC GCC AAG AAC TCA CTG TAT CTG CAA ATG AAC AGC CTG AGA GCC GAC GAC ACG GCC GTA TAT TTC TGT GCG AGA GAG AGA GGA GCA GCT GGT ACA GGG TGG TTC GAC CCC TGG GGC CAG GGA ACC CTG GTC ACC GTC TCC TCA | GAC ATC CAG ATG ACC CAG TCT CCA TCT TCC GTG TCT GCA TCT GTA GGA GAC AGA GTC ACC ATC ACT TGT CGG GCG AGT CAG GGT ATT AGC AGC TGG TTA GCC TGG TAT CAG CAG AAA CCA GGG AAA GCC CCT AAG CTC CTG ATC TAT GCT GCA TCC AGT TTG CAA GGT GGG GTC CCA TCA AGG TTC AGC GGC AGT GGA TCT GGG ACA GAT TTC ACT CTC ACC ATC AGC AGC CTG CAG CCT GAA GAT TTT GCA ACT TAC TAT TGT CAA CAG GTT AAC AGT TTC CCG TGG ACG TTC GGC CAA GGG ACC AAG GTG GAA ATC AAA |
| 1.62.3-u1-IgG1K | SEQ ID NO: 47 | SEQ ID NO: 48 |
| | CAG GTG CAG CTG CAG GAG TCG GGC CCA GGA CTG GTG AAG CCT TCA CAG ACC CTG TCC CTC ACC TGC ACT GTC TCT GGT GGC TCC ATC AGT AAT GGT GGT TAC TAC TGG AGC TGG ATC CGC CAG CAC CCA GGG AAG GGC CTG GAG TGG ATT GGG TAC ATC TAT TAC | GAC ATC GTG ATG ACC CAG TCT CCA GAC TCC CTG GCT GTG TCT CTG GGC GAG AGG GCC ACC ATC AAC TGC AAG TCC AGC CAG AGT GTT GTA TTC AGC TCC AAC AAT AAG ATC TGC TTA GCT TGG TAC CAG CAG AAA CCA GGA CAG CCT CCT AAG CTG CTC ATT TAC |
| | AGT GGG AGC ACC TAC TAC AAC CCG TCC CTC AAG AGT CGA GTT ACC ATA TCA GTA GAC ACG TCT AAG AAC CAG TTC TCC CTG AAA CTG AGC TCT GTG ACT GCC GCG GAC ACG GCC GTG TAT TAC TGT GCG AGA GAT GAG TGG GAG CTA CGG GGG TTT GAC TAC TGG GGC CAG GGA ACC CTG GTC ACC GTC TCC TCA | TGG TCA TCT ACC CGG GAA TCC GGG GTC CCT GAC CGA TTC AGT GGC AGC GGG TCT GGG ACA GAT TTC ACT CTC ACC ATC AGC AGC CTG CAG GCT GAA GAT GTG GCA GTT TAT TAC TGT CAG CAA TAT TAT AGT TCT CCG TGG ACG TTC GGC CAA GGG ACC AAG GTG GAA ATC AAA |
| 1.623-u1-3-IgG1K | SEQ ID NO: 49 | SEQ ID NO: 50 |
| | CAG GTG CAG CTG CAG GAG TCG GGC CCA GGA CTG GTG AAG CCT TCA CAG ACC CTG TCC CTC ACC TGC ACT GTC TCT GGT GGC TCC ATC AGT AAT GCC GGT TAC TAC TGG AGC TGG ATC CGC CAG CAC CCA GGG AAG GGC CTG GAG TGG ATT GGG TAC ATC TAT TAC AGT GGG AGC ACC TAC TAC AAC CCG TCC CTC AAG AGT CGA GTT ACC ATA TCA GTA GAC ACG TCT AAG AAC CAG TTC TCC CTG AAA CTG AGC TCT GTG ACT GCC GCG GAC ACG GCC GTG TAT TAC TGT GCG AGA GAT GAG TGG GAG CTA CGG GGG TTT GAC TAC TGG GGC CAG GGA ACC CTG GTC ACC GTC TCC TCA | GAC ATC GTG ATG ACC CAG TCT CCA GAC TCC CTG GCT GTG TCT CTG GGC GAG AGG GCC ACC ATC AAC TGC AAG TCC AGC CAG AGT GTT GTA TTC AGC TCC AAC AAT AAG ATC AGC TTA GCT TGG TAC CAG CAG AAA CCA GGA CAG CCT CCT AAG CTG CTC ATT TAC TGG TCA TCT ACC CGG GAA TCC GGG GTC CCT GAC CGA TTC AGT GGC AGC GGG TCT GGG ACA GAT TTC ACT CTC ACC ATC AGC AGC CTG CAG GCT GAA GAT GTG GCA GTT TAT TAC TGT CAG CAA TAT TAT AGT TCT CCG TGG ACG TTC GGC CAA GGG ACC AAG GTG GAA ATC AAA |
| 1.134.9-u1-IgG1L | SEQ ID NO: 51 | SEQ ID NO: 52 |
| | CAG GTG CAG CTG CAG GAG TCG GGC CCA GGA CTG GTG AAG CCT TCG GGG ACC CTG TCC CTC ACC TGC GCT GTC TCT GGT GGC TCC ATC AGT AGT TAT AAC TGG TGG AGT TGG GTC CGC CAG CCC CCA GGG AAG GGA CTG GAG TGG ATT GGG GAA ATC TAT CAT GGT GGG AAC ACC AAC TAC AAC CCG TCC CTC AAG AGT CGA GTC ACC ATG TCA GTA GAC AAC TCC AAG AAC CAG TTC TCC CTG AAG CTG AGC TCT GTG ACC GCC GCG GAC ACG GCC GTA TAT TAC TGT GCG AGA GCC CCC GGG GAC TGG GGA GGT TCC CCC TAT TTT GAC TTC TGG GGC CAG GGA ACC CTG GTC ACC GTC TCC TCA | GGT TCT GTG GTT TCT TCT GAA CTG ACT CAG GAC CCT GCT GTG TCT GTG GCC TTG GGA CAG ACA GTC AGG ATC ACA TGC CAG GGA GAC AAC CTC AGA ACC TAT TAT GCA AGC TGG TAC CAG CAG AAG CCA GGA CAG GCC CCT ATA CTT CTC ATC TAT GGT AGA AAC AAG CGG CCC TCA GGG ATC CCA GAC CGA TTC TCT GGC TCC AGC TCG GGA AAC ACA GCT TCC TTG ACC ATC ACT GGG GCT CAG GCG GAA GAT GAG GCT GCG TAC TAC TGT AAC TCC CGG GAC AGC AGT GGT AAT CCT GTG GTA TTC GGC GGA GGG ACC AAG CTG ACC GTC CTA |
| 1.186.19-u1-IgG1K | SEQ ID NO: 53 | SEQ ID NO: 54 |
| | CAG GTG CAC CTG GTG GAG TCT GGG GGA GGC TTG GTC AAG CCT GGA GGG TCC CTG AGA CTC TCC TGT GCA GCC TCT GGA TTC ACC TTC AGT GAC TAC TAC ATG GGC TGG ATC CGC CAG GCT CCA GGG CAG GGG CTG GAG TGG GTT TCA TAC ATT AGT GGT AGT GGT AAC ACC ATT TAC TAC GCA GAC TCT GTG AAG GGC CGA TTC ACC ATC TCC AGG GAC AAC GCC AAG AAC TCA CTG TAT CTG CAA ATG AAC AGC CTG AGA GCC GAC GAC ACG GCC GTT TAT TTC TGT GCG AAA GAG AGA GGA GCA GCT GGT GCA GGG TGG TTC GAC CCC TGG GGC CAG GGA ACC CTG GTC ACC GTC TCC TCA | GAC ATC CAG ATG ACC CAG TCT CCA TCT TCC GTG TCT GCA TCT GTA GGA GAC AGA GTC ACC ATC ACT TGT CGG GCG AGT CAG GGT ATT AGC AGC TGG TTA GCC TGG TAT CAG CAG AAA CCA GGG AAA GCC CCT AAG CTC CTG ATC TAT GCT GCA TCC AGT TTG CAA GGT GGG GTC CCA TCA AGG TTC AGC GGC AGT GGA TCT GGG ACA GAT TTC ACT CTC ACC ATC AGC AGC CTG CAG CCT GAA GAT TTT GCA ACT TAC CAT TGT CAA CAG GTT AAC AGT TTC CCG TGG ACG TTC GGC CAA GGG ACC AAG GTG GAA ATC AAA |
| 1.214.23-u1-IgG1K | SEQ ID NO: 55 | SEQ ID NO: 56 |
| | CAG GTG CAG CTG CAG GAG TCG GGC CCA GGA CTG GTG AAG CCT TCG GGG ACC CTG TCC CTC ACC TGT GTT GTC TCC GGT GGC TCC ATC AGC AAT AGA AAC TGG TGG AGT TGG GTC CGC CAG CCC CCA GGG AAG GGG CTG GAG TGG ATT GGG GAA ATC TTT CAT AGT GGG AAC ACC AAC TAC AAC CCG TCC CTC AAG AGT CGC GTC ACC ATA TCA GTA GAC AAG TCC AAG AAC CAG TTC TCC CTG AAG GTG AAC TCT GTG ACC GCC GCG GAC ACG GCC GTG TAT TAC TGT GCG AAA TCC TTT GCA GTG GCC CTT GAC TCC TGG GGC CAG GGA ACC CTG GTC ACC GTC TCC TCA | GAC ATC CAG TTG ACC CAG TCT CCA TCC TTC CTG TCT GCA TCT GTA GGA GAC AGA GTC ACC ATC ACT TGC CGG GCC AGT CAG GAC ATT AAC AGT TAT TTA GCC TGG TGT CAG CAA AAA CCA GGG AAA GCC CCT AAG CTC CTG ATC TAT GCT GCA TCC TCT TTG CAA AGT GGG GTC CCA TCA AGG TTC AGC GGC ACT GGA TCT GGG ACA GAG TTC ACT CTC ACA ATC AGC AGC CTG CAG CCT GAA GAT TTT GCA ACT TAT TAC TGT CAA CAG CTT TTT AGT TAC CCG ATC ACC TTC GGC CAA GGG ACA CGA CTG GAG ATT AAA |

### EXAMPLES

The present invention, thus generally described, will be understood more readily by reference to the following Examples, which are provided by way of illustration and are not intended to be limiting of the instant invention. The Examples are not intended to represent that the experiments below are all or the only experiments performed.

### EXAMPLE 1

### Preparation of Materials

### 1.1 Immunogen generation

cDNA encoding the extracellular domain (ECD) of OX40 protein (GenBank ref CAB96543.1) were synthesized by Sangon Biotech and inserted into a modified expression vector pcDNA3.3 (ThermoFisher). Max-prep the plasmid DNAs and the inserted DNA sequences were verified by sequencing. Fusion proteins OX40 ECD conjugated with human Fc or His tag were obtained by transfection of human OX40 ECD gene into Freestyle 293F (ThermoFisher) or Expi-293F cells (ThermoFisher). After 5 days, supernatants were harvested from the cultures of transient transfected cells. The fusion proteins were purified and quantitated for usage of immunization and screening.

### 1.2 Production of Benchmark Antibodies

Four benchmark antibodies, namely, BMK1, BMK5, BMK7 and BMK10, are applied as positive controls in the examples. BMK1 was synthesized according to the clone of 11D4 from U.S. patent No. US8236930B2 (Pfizer). BMK5 was synthesized according to the clone of 106-22 from U.S. patent application No. US20140308276 (University of Texas System). BMK7 was synthesized according to the clone of OX40mAb24 from PCT publication No. WO2016057667 (MedImmune). BMK10 was synthesized according to the clone of lA7.grl from PCT publication No. WO2015153513 (Genentech).

### 1.3 Establishment of Stable Cell Lines

In order to obtain tools for antibody screening and validation, we generated OX40 transfectant cell lines. Briefly, CHO-K1 or 293F cells were transfected with the modified expression vector pcDNA3.3 containing full-length OX40 using Lipofectamine 2000 or PlasFect transfection kit according to manufacturer's protocol. At 48-72 hours post transfection, the transfected cells were cultured in medium containing Blasticidin for selection. Overtime this will select the cells that have the expression plasmid stably incorporated into their genomic DNAs. Meanwhile the cells were checked for OX40 expression. Once the expression verified, single clones of interested were picked by limited dilution and scaled up to large volumes. The established monoclonal cell lines were then maintained in medium containing Blasticidin.

### EXAMPLE 2

### Antibody Hybridoma Generation

### 2.1 Immunization and cell fusion

Fully human monoclonal antibodies against OX40 were prepared using OMT rats, which comprise chimeric polynucleotides useful for optimal production of functional immunoglobulins with human idiotypes. The rat strain carries human heavy and light chain transgene as described in PCT Publication WO 2014/093908. To generate fully human monoclonal antibodies against OX40, OMT rats, 6-8 weeks of age, were immunized with 20 µg of human OX40 ECD protein in aluminium phosphate (Alum-Phos) in footpad and 20 µg of human OX40 ECD protein in TiterMax subcutaneously for first boost, and the immunization was repeated every two weeks with human OX40 ECD protein in Alum-Phos and TiterMax. The serum antibody titers were measured by enzyme-linked immunosorbent assay (ELISA) every one or two weeks. When the serum antibody titer was sufficiently high, rats were given a final boost with 40 µg of human OX40 ECD protein in DPBS without adjuvant. The cell fusion was performed as following: preparing myeloma cells SP2/0, myeloma cells were thawed the week before the fusion, and were split 1:2 each day until the day before the fusion to keep the cells in logarithmic growth phase. B lymphocytes isolated from lymph node of immunized OMT rats were combined with myeloma cells (at 1:1.1 ratio). Cell mixture was washed and re-suspended in ECF solution at 2×10⁶ cells/mL. The cells are ready for ECF. After electronic cell fusion, cell suspension from the fusion chamber was immediately transferred into a sterile tube containing more medium, and incubated for at least 24 hours in a 37 °C incubator. The cell suspension was mixed and transferred into 96-well plates (1×10⁴ cells/well). The 96-well plates were cultured at 37°C, 5% CO₂, and were monitored periodically. When the clones were big enough, 100 uL of supernatant were transferred from the tissue culture plates to 96-well assay plates for antibody screening.

### 2.2 High throughput screening of hybridoma supernatants

ELISA was used as first screening method to test the binding of hybridoma supernatants to human and monkey OX40 protein. Briefly, plates (Nunc) were coated with soluble protein of human or rhesus monkey OX40 ECD at 1 ug/mL overnight at 4 °C. After blocking and washing, the hybridoma supernatants were transferred to the coated plates and incubated at room temperature for 2 hours. The plates were then washed and subsequently incubated with secondary antibody, goat anti-rat IgG HRP (Bethyl), for 1 hour. After washing, TMB substrate was added and the interaction was stopped by 2M HCl. The absorbance at 450 nm was read using a microplate reader (Molecular Device).

In order to confirm the native binding of anti-OX40 antibodies on conformational OX40 molecules expressed on cell membrane, flow cytometry (FACS) analysis was performed on OX40 transfected cell lines. 293F cells expressing human OX40 were transferred into 96-well U-bottom plates (Corning) at a density of 1×10⁵ cells/well. The hybridoma supernatants were then loaded to the cells and incubated for 1 h at 4 °C. After washing with 1×PBS/1%BSA, the secondary antibody goat anti-rat Alexa647 (Jackson ImmunoResearch Lab) was applied and incubated with cells at 4 °C in the dark for half an hour. The cells were then washed and resuspended in 1×PBS/1%BSA or fixed with 4% paraformaldehyde, and analyzed by flow cytometry (BD). Antibody binding to parental 293F cell line was used as negative control. Testing the bioactivity of antibodies using Jurkat NFkB-luciferase Reporter T cells was used as second screening method. Briefly, human OX40/CD40 fusion protein-overexpressing Jurkat NFkB-luciferase reporter cell was constructed as described above. The cells were cultured in complete RPMI1640 medium containing 10% FBS, and 0.5 mg/mL of Hygromycin B as selection. OX40 Jurkat reporter cells were collected and added to a 96-well plate at 4×10⁴ cells/well. Cross linking antibodies F(ab')₂ goat anti-rat IgG (JacksonImmunoResearch Lab) and RPMI 1640 complete medium diluted hybridoma supernatants were added to the cells, and then the cells were incubated at 37 °C, 5% CO₂ overnight. The second day, reconstituted luciferase substrate (Promega) was added to each well (50 µL/well) and mixed well. The luciferase intensity was read using a microplate reader (Molecular Device).

### 2.3 Hybridoma sub-cloning:

Once specific binding and bioactivity were verified through first and confirmation screening, the positive hybridoma cell lines were used for subcloning. Briefly, for each hybridoma cell line, cells were counted and diluted to give 1 cell per 200 µL cloning medium. The cell suspension was plated 200 µL/well into two 96-well plates. Plates were cultured at 37 °C, 5% CO₂, until they were ready to be checked by ELISA assay. The exhausted supernatant (ESN) of selected single clones were collected, and the antibodies were purified for further characterization.

### EXAMPLE 3

### Fully human antibody molecules construction and purification

### 3.1 Hybridoma sequencing

RNAs were isolated from monoclonal hybridoma cells using RNeasy Plus Mini Kit (Qiagen) with Trizol reagent. The heavy chain variable region (VH) and heavy chain variable region (VL) of OX40 chimeric antibodies were amplified as follows. Briefly, RNA is first reverse transcribed into cDNA using a reverse transcriptase as described here.

**Table 1. cDNA amplification reaction (20 µL)**

| **Component** | **Amount** |
|---|---|
| Up to 5 µg total RNA | 5 µL |
| Primer (50 µM oligo(dT)₂₀/50 ng/µL random hexamers) | 1 µL/1 µL |
| Annealing Buffer | 1 µL |
| RNase/DNase-free water | to 8 µL |
| 65°C for 5min, then immediately place on ice for at least 1 minute | |
| 2×First-Strand Reaction Mix | 10 µl |
| SuperScript^{™} III/RNaseOUT^{™} Enzyme Mix | 2 µL |

**Table 2. cDNA amplification reaction condition**

| | **Step1** | **Step 2** | **Step3** | **Step4** |
|---|---|---|---|---|
| Temperature | 25 | 50 | 85 | 4 |
| Time | 10 min | 50 min | 5 min | ∞ |

The resulting cDNA was used as template for subsequent PCR amplification using primers specific for interested genes. The PCR reaction was done as follows.

**Table 3. PCR Reaction system (50 µL)**

| **Component** | **Amount** |
|---|---|
| cDNA | 2.0 µL |
| Premix Ex Taq | 25 µl |
| 5'- degenerated primer sets (10 pM) | 2.5 µl |
| 3'- constant region degenerated primer (10 pM) | 1.0 µl |
| ddH₂O | 19.5 µl |

**Table 4. PCR Reaction condition**

| | **Step 1** | **Step 2** | **Step 3** | **Step 4** | **Step 5** |
|---|---|---|---|---|---|
| Temperature (°C) | 95 | 94 | 58 | 72 | 72 |
| Time | 4 min | 45 sec | 45 sec | 1 min | 10 min |
| Cycles | NA | 30 | | NA | NA |

The PCR product (10 µL) was inserted into the pMD18-T vector; and 10 µL of the ligation product was transformed into the Top 10 competent cells. Transformed cells were plated on 2-YT+Cab plates and incubated overnight. Positive clones were checked by PCR using M13-48 and M13-47 primers followed by sequencing.

Hybridoma clones 1.7.10, 1.62.3, 1.134.9, 1.186.19 and 1.214.23 were selected for sequence optimization and further evaluation.

### 3.2 Antibody sequence optimization

Antibody sequence optimization was carried out by introducing appropriate modification at specific site into the nucleotide sequence encoding an antibody. PTM (post-translational modification) site removing mutations were introduced by site directed mutagenesis using QuickChange mutagenesis kit according to the manufacturer's protocol.

The amino acid NGG in CDR1 of hybridoma clone 1.62.3 heavy chain was identified as a deamidation site, so antisense mutagenic nucleotides were designed to introduce following mutations into "1.62.3-ul-IgGlK" heavy chain: N to Q (NGG-QGG), N to S (NGG-SGG) or G to A (NGG-NAG). The amino acid C in CDR1 of clone 1.62.3 light chain was identified as cysteine residue, so serine was substituted with cysteine (C to S). All mutations were verified by sequencing.

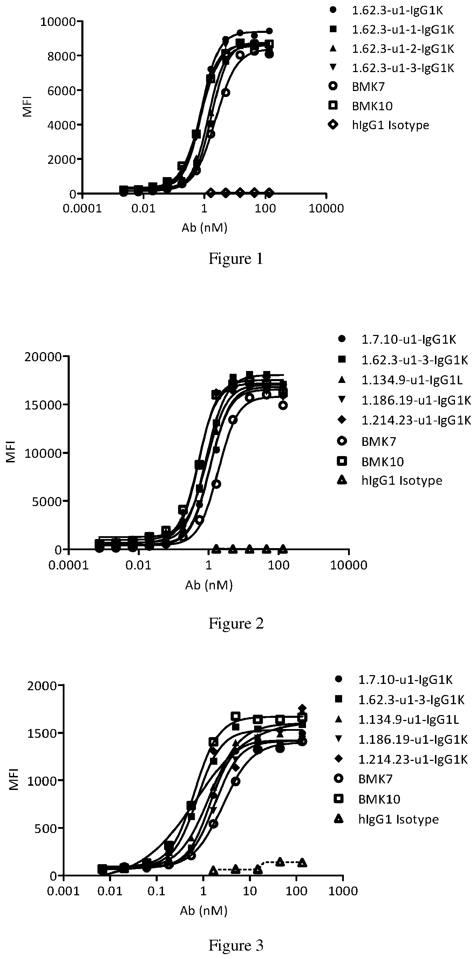

The comparison between variants after PTM mutation is shown in Figure 1. The variants are named as antibodies "1.62.3-u1-1-IgG1K", "1.62.3-ul-2-IgGlK", and "1.62.3-ul-2-IgGlK", respectively. Antibody "1.62.3-u1-1-IgG1K" contains the mutation N to Q, "1.62.3-ul-2-IgGlK" contains the mutation N to S, and "1.62.3-ul-3-IgGlK" contains the mutation G to A.

### 3.3 Fully human antibody molecule construction and purification

The VH and VL of OX40 hybridoma antibodies were amplified as described above. Synthetic genes were recloned into the modified human IgG1 expression vector pcDNA3.4 (ThermoFisher) to express fully human antibodies. Expi-293F cells were transiently transfected with the vector for antibody expression. The culture supernatant containing antibodies was harvested and purified using Protein A chromatography.

The fully human monoclonal antibodies 1.7.10-ul-IgGlK, 1.62.3-ul-IgGlK (sequence optimized clone named as "1.62.3-ul-3-IgGlK"), 1.134.9-ul-IgGlL, 1.186.19-ul-IgGlK and 1.214.23-ul-IgGlK were obtained from the hybridoma clones 1.7.10, 1.62.3, 1.134.9, 1.186.19 and 1.214.23 hybridomas, respectively. Sequences thereof are summarized in Table A, B and C.

### EXAMPLE 4

### Antibody characterization

### 4.1 Full kinetic binding affinity test by surface plasmon resonance (SPR)

Antibodies were characterized for affinity and binding kinetics to OX40 by SPR assay using Biacore T200 (GE). Anti-human IgG antibody was pre-immobilized to a sensor chip (CM5), and anti-OX40 antibodies in running buffer (1×HBS-EP+, GE) were captured when injected to the chip. Then various concentrations of human or monkey OX40 and running buffer were flowed through the sensor chip at a flow rate of 30 µL/min for an association phase of 180 s, followed by dissociation. The association and dissociation curve was fit by 1:1 Langmuir binding model using the BIAevaluation T200 software.

Experimental results are shown in Table 5 below.

**Table 5. Full kinetic binding affinity to human OX40 by SPR**

| **Abs** | **ka (l/Ms)** | **kd (l/s)** | **K_{D} (M)** |
|---|---|---|---|
| **1.7.10-u1-IgG1K** | 8.60×10⁵ | 1.28×10⁻³ | 1.49×10⁻⁹ |
| **1.62.3-u1-IgG1K** | 5.26×10⁵ | 2.43×10⁻⁴ | 4.61×10⁻¹⁰ |
| **1.62.3-u1-3-IgG1K** | 6.97×10⁵ | 1.45×10⁻³ | 2.08×10⁻⁹ |
| **1.134.9-u1-IgG1L** | 2.07×10⁵ | 3.70×10⁻⁵ | 1.79×10⁻¹⁰ |
| **1.186.19-u1-IgG1K** | 3.90×10⁵ | 4.51×10⁻⁴ | 1.16×10⁻⁹ |
| **1.214.23-u1-IgG1K** | 4.26×10⁵ | 2.14×10⁻⁴ | 5.02×10⁻¹⁰ |
| **BMK7** | 2.44×10⁵ | 1.54×10⁻³ | 6.30×10⁻⁹ |
| **BMK10** | 4.42×10⁵ | 2.25×10⁻⁵ | 5.10×10⁻¹⁰ |

As shown in Table 5, the illustrative antibodies of the invention, including 1.7.10-ul-IgGlK, 1.62.3-ul-IgGlK, 1.62.3-ul-3-IgGlK, 1.134.9-ul-IgGlL, 1.186.19-ul-IgGlK and 1.214.23-ul-IgGlK bind to human OX40 with high specificity, with a K_{D} from 1.79×10⁻¹⁰ M to 2.08×10⁻⁹ M.

### 4.2 Binding affinity analysis by flow cytometry

CHO-K1 cells expressing human OX40 were transferred in to 96-well U-bottom plates (Corning) at a density of 5×10⁴ cells/mL. Testing antibodies were 1:2 serially diluted in wash buffer (1×PBS/ 1%BSA) and incubated with cells at 4 °C for 1 h. The secondary antibody goat anti-human IgG Fc FITC (3.2 moles FITC per mole IgG, Jackson Immunoresearch Lab) was added and incubated with cells at 4 °C in the dark for half an hour. The cells were then washed once, resuspended in 1×PBS/1%BSA and analyzed by flow cytometry (BD). Fluorescence intensity was converted to bound molecules/cell based on the quantitative beads Quantum^{™} MESF Kits (Bangs Laboratories, Inc.). The K_{D} value of each antibody was calculated using Graphpad Prism5.

The data for binding of anti-human OX40 antibodies to CHO-K1 cells expressing human OX40 by Flow Cytometry are shown in Table 6 and Figure 2. The data demonstrate that the illustrative antibodies 1.7.10-ul-IgGlK, 1.62.3-ul-3-IgGlK, 1.134.9-ul-IgGlL, 1.186.19-ul-IgGlK and 1.214.23-ul-IgGlK show well binding efficiency to CHO-K1 cells expressing human OX40.

**Table 6. Binding affinity of anti-OX40 antibodies to cell surface human OX40 tested by flow cytometry**

| **Abs** | **Bmax (M)** | **K_{D} (M)** |
|---|---|---|
| **1.134.9-u1-IgG1L** | 2.1×10⁻¹⁰ | 5.3×10⁻¹⁰ |
| **1.214.23-u1-IgG1K** | 1.7×10⁻¹⁰ | 6.7×10⁻¹¹ |
| **BMK7** | 2.0×10⁻¹⁰ | 1.5×10⁻⁹ |
| **BMK10** | 1.8×10⁻¹⁰ | 2.3×10⁻¹⁰ |

As demonstrated in Table 6 and Figure 2, the antibodies 1.134.9-ul-IgGlL and 1.214.23-ul-IgGlK bind to cell surface human OX40 with high affinity which is comparable or even higher than BMK7 and BMK10.

### 4.3 Binding of anti-OX40 antibodies to OX40

Cell-based FACS was used for testing the binding activity of anti-OX40 antibodies to OX40. Briefly, human OX40-expressing CHO-K1 cells or activated human CD4⁺ T cells were transferred into 96-well U-bottom plates (Corning) at a density of 1×10⁵ cells/well. Testing antibodies were serially diluted in wash buffer (1×PBS/1%BSA) and incubated with cells at 4 °C for 1 h. After washing with 1×PBS/1%BSA, the secondary antibody goat anti-human IgG Fc-PE (Jackson ImmunoResearch Lab) was applied and incubated with cells at 4 °C in the dark for 1 h. The cells were then washed and resuspended in 1×PBS/1%BSA or fixed with 4% paraformaldehyde, and then analyzed by flow cytometry (BD).

The data for binding of anti-OX40 antibodies to activated human CD4⁺ T cells by flow cytometry are shown in Figure 3. The data show that the illustrative antibodies 1.7.10-ul-IgGlK, 1.62.3-ul-IgGlK, 1.134.9-ul-IgGlL, 1.186.19-ul-IgGlK and 1.214.23-ul-IgGlK bind to cell surface human OX40 in a dose-dependent manner.

### 4.4 Competition of ligand binding to OX40

ELISA based competition assay was used to test whether anti-OX40 antibodies could competitively block the binding of OX40 to OX40 ligand (OX40L). Briefly, plates (Nunc) were coated with human OX40 ECD at 1 µg/mL overnight at 4 °C. Antibodies were serially diluted in blocking buffer and mixed with constant concentration of OX40L. After blocking and washing, the antibody/OX40L mixture were added to the plates, and then incubated at room temperature for 1 h. The plates were then washed and subsequently incubated with HRP conjugated secondary antibody for 1 h to detect the binding of OX40L to OX40 ECD. After washing, TMB substrate was added and the interaction was stopped by 2M HCl. The absorbance at 450 nm and 540 nm was read using a microplate reader (Molecular Device).

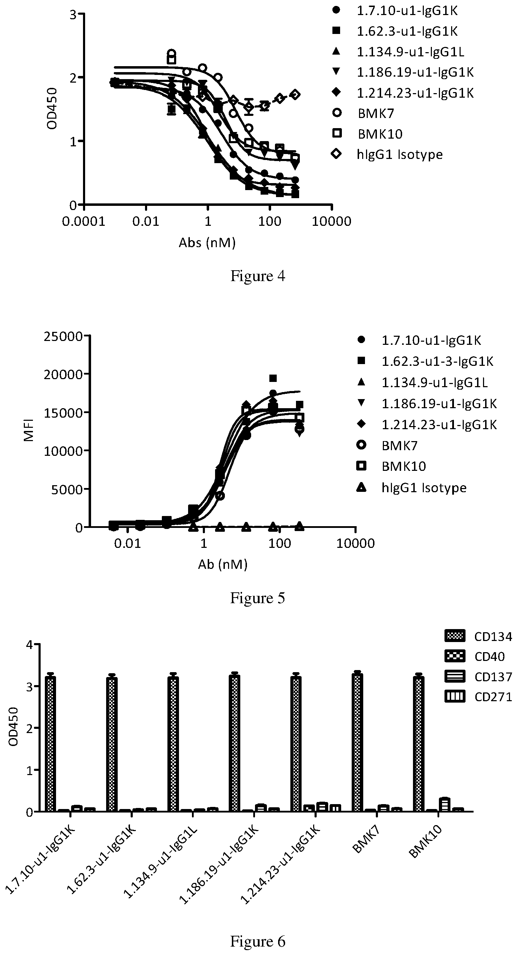

As shown in Figure 4, the illustrative antibodies 1.7.10-ul-IgGlK, 1.62.3-ul-IgGlK, 1.134.9-ul-IgGlL, 1.186.19-ul-IgGlK and 1.214.23-ul-IgGlK competitively binding to human OX40 with OX40L.

### 4.5 Orthologue (cross-species) test

Cross-reactivity of anti-OX40 antibodies to rhesus monkey OX40 was measured by cell-based FACS. Briefly, rhesus monkey OX40-expressing 293F cells were transferred into 96-well U-bottom plates (Corning) at a density of 2×10⁵ cells/well. Testing antibodies were serially diluted in wash buffer (1×PBS/1%BSA) and incubated with cells at 4 °C for 1 h. After washing with 1×PBS/1%BSA, the secondary antibody goat anti-human IgG Fc-PE (Jackson ImmunoResearch Lab) was applied and incubated with cells at 4 °C in the dark for 1 h. The cells were then washed and resuspended in 1×PBS/1%BSA or fixed with 4% paraformaldehyde, and then analyzed by flow cytometry (BD).

As demonstrated in Figure 5, the illustrative antibodies 1.7.10-ul-IgGlK, 1.62.3-ul-3-IgGlK, 1.134.9-ul-IgGlL, 1.186.19-ul-IgGlK and 1.214.23-ul-IgGlK have cross-reactive binding to rhesus monkey OX40 transfected 293F cells.

### 4.6 Homologue (cross-family) binding

Human OX40, CD40, 4-1BB (CD137) and CD271 ECD were coated on plates (Nunc) overnight at 4 °C. After blocking and washing, testing antibodies were diluted in blocking buffer and added to the plates and incubated at room temperature for 1 h. The plates were then washed and subsequently incubated with secondary antibody goat anti-human IgG Fc-HRP (Bethyl) for 1 h. After washing, TMB substrate was added and the interaction was stopped by 2M HCl. The absorbance at 450nm and 540nm was read using a microplate reader (Molecular Device).

Results on cross family binding test of anti-OX40 antibodies to human CD40, 4-1BB (CD137) and CD271 ECD by ELISA are shown in Figure 6. The result demonstrates that OX40 antibodies 1.7.10-ul-IgGlK, 1.62.3-ul-IgGlK, 1.134.9-ul-IgGlL, 1.186.19-ul-IgGlK and 1.214.23-ul-IgGlK specifically bind to OX40 (i.e., CD134), and do not bind to human CD40, CD137 and CD271.

### 4.7 Epitope binning test against BMK antibodies

The binding epitope of anti-OX40 antibodies was binned against benchmark antibodies by ELISA. The testing antibodies were coated on plates (Nunc) overnight at 4 °C. After blocking and washing, constant concentration of human OX40 protein diluted in blocking buffer was added to the plates and incubated at room temperature for 1 h. Then the biotinylated benchmarks were serially diluted and added to each well and incubated for another 1 h. The plates were then washed and subsequently incubated with secondary antibody streptavidin-HRP (Life Technology) for 1 h. After washing, TMB substrate was added and the interaction was stopped by 2M HCl. The absorbance at 450nm and 540nm was read using a microplate reader (Molecular Device).

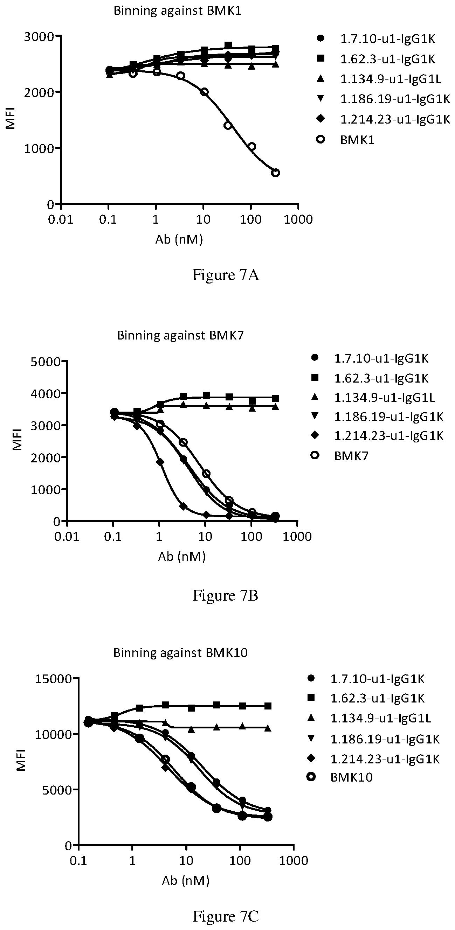

Figures 7A, 7B and 7C show epitope binning of the antibodies 1.7.10-ul-IgGlK, 1.62.3-ul-IgGlK, 1.134.9-ul-IgG1L, 1.186.19-ul-IgGlK and 1.214.23-ul-IgGlK against benchmark antibodies BMK1 (Figure 7A), BMK7 (Figure 7B) and BMK10 (Figure 7C), respectively. As shown in Figure 7A, the antibodies 1.7.10-ul-IgGlK, 1.62.3-ul-IgGlK, 1.134.9-ul-IgGlL, 1.186.19-ul-IgGlK and 1.214.23-ul-IgGlK share different bins from BMK1. As shown in Figures 7B and 7C, the antibodies 1.62.3-ul-IgGlK and 1.134.9-ul-IgGlL share different bins from BMK7 and BMK10, but the antibodies 1.7.10-ul-IgGlK, 1.186.19-ul-IgGlK and 1.214.23-ul-IgGlK share similar or close bins with BMK7 and BMK10.

### 4.8 Bioactivity assay using Jurkat NFkB-luciferase Reporter T cells

The ability of anti-OX40 antibodies to signal through human OX40 was assessed using an engineered Jurkat cell line expressing OX40/CD40 fusion protein and NFkB-luciferase reporter gene. Bioactivity of anti-OX40 antibodies cross-linked by using an anti-human IgG Fc reagent or cells expressing human Fcγ receptor complements were measured. The Jurkat NFkB-luciferase Reporter cells were cultured in complete RPMI 1640 medium containing 10% FBS, and 0.5 mg/mL of Hygromycin B as selection.

To determine the bioactivity of anti-OX40 antibodies in complexed condition, CD32b-expressing CHO-K1 cells or F(ab')₂ goat anti-human IgG (Jackson ImmunoResearch Lab) was used to mediate antibodies cross-linking, which clusters and activates OX40 on the Jurkat report cells. OX40 Jurkat reporter cells were collected and added to a 96-well plate. OX40 antibodies serially diluted in complete medium were added to the cells in the presence of CD32b-expressing CHO-K1 cells, parental CHO-K1 cells or cross-linker antibodies, and incubated the plates at 37 °C, 5% CO₂ for 6 hours or overnight. Reconstituted luciferase substrate (Promega) was added to each well and mixed well. The luciferase intensity was read using a microplate reader (Molecular Device). Anti-OX40 antibodies were also tested for bioactivity in soluble condition.

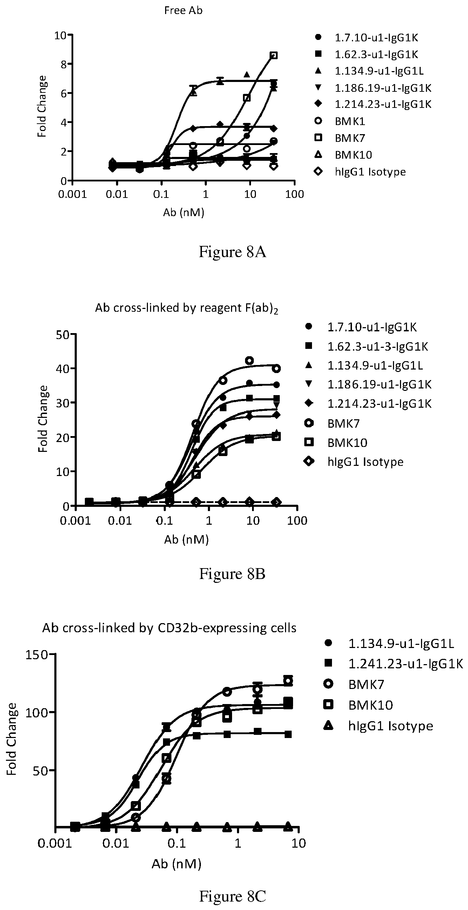

Figures 8A, 8B and 8C show the effect of testing antibodies on OX40-stimulated NFkB luciferase activity in Jurkat cells using free antibodies or FcyR cross-linking by CD32b-expressing CHO-K1 cells or anti-human IgG Fc reagent. Reporter activity of (Figure 8A) free antibodies or cross-linked by (Figure 8B) F(ab')₂ goat anti-human IgG or (Figure 8C) CD32b-expressing CHO-K1 cells is shown, respectively.

As shown in Figures 8A, 8B and 8C, cross-linked antibodies can effectively activate OX40 signaling.

### 4.9 In vitro function of anti-OX40 antibodies tested by cell-based assays

Human CD4⁺ T cells used in this example were isolated from human PBMCs using Human CD4⁺ T Cell Enrichment Kit (StemCell) according to the manufacturer's protocol. The cells were resuspended in complete RPMI 1640 medium.

### 4.9.1 Effects of anti-OX40 antibodies on interleukin 2 (IL-2) production in vitro

In this assay, non-tissue culture treated flat-bottom 96-well plates (Corning) were pre-coated with anti-CD3 overnight at 4 °C. On the day of assay, the plates were washed with complete RPMI 1640 medium to remove un-bound antibodies. Freshly isolated human CD4⁺ T cells were added to each well at a density of 1×10⁵ cells/well in a volume of 100 µL. Then constant concentration of cross linking antibody F(ab')₂ goat anti-human IgG and serially diluted OX40 antibodies were mixed in 100 µL and were also added to each well of the plates. The plates were incubated at 37 °C, 5% CO₂ for 3 days and then the supernatants were harvested for IL-2 measurement by ELISA.

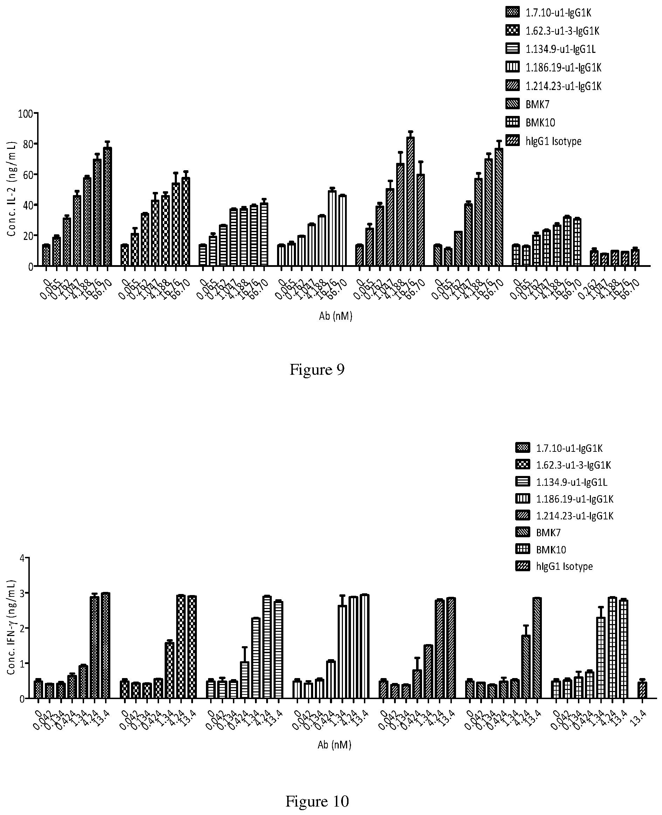

Figure 9 shows the effect of antibodies on anti-CD3 induced IL-2 secretion by primary human CD4⁺ T cells. It is demonstrated that the illustrative antibodies (including 1.7.10-ul-IgGlK, 1.62.3-ul-IgGlK, 1.134.9-ul-IgGlL, 1.186.19-ul-IgGlK and 1.214.23-ul-IgGlK) enhanced IL-2 secretion by primary human CD4⁺ T cells.

### 4.9.2 Effects of anti-OX40 antibodies on cytokine IFNγ secretion and CD4⁺ T cell proliferation in vitro

To directly assess the effect of anti-OX40 antibodies on enhancing IFNγ production and CD4⁺ T cell proliferation, we performed an assay to co-stimulate human CD4⁺ T cells through OX40 signal in combination with CD3/T cell receptor (TCR) complex. Briefly, non-tissue culture treated flat-bottom 96-well plates (Corning) were pre-coated with 100 µL of mixture of constant concentration of anti-CD3 and different concentration of anti-OX40 antibodies. The plates were incubated overnight at 4 °C, and then washed with complete RPMI 1640 medium to remove un-bound antibodies. Freshly isolated human CD4⁺ T cells were added to each well at a density of 1×10⁵ cells/well in a volume of 200 µL. The plates were incubated at 37 °C, 5% CO₂ for 3 days and then the supernatants were harvested for IFNγ measurement by ELISA. The cell pellets were harvested to measure CD4⁺ T cell proliferation by 3H-thymidin as follows: 3H-thymidine (PerkinElmer) was added to the cell culture plates at 0.5 µCi/well. The plates were cultured in 5% CO₂ at 37 °C for 16 to 18 hours, before the incorporation of ³H-thymidine into the proliferating cells was determined using Topcount NXT Scintillation Counter (Perkin Elmer).

Figure 10 shows the effect of antibodies on anti-CD3 induced IFN-γ secretion by primary human CD4⁺ T cells. It is demonstrated that the illustrative antibodies 1.7.10-ul-IgGlK, 1.62.3-ul-3-IgGlK, 1.134.9-ul-IgGlL, 1.186.19-ul-IgG1K and 1.214.23-ul-IgGlK enhanced IFN-γ secretion by primary human CD4⁺ T cells.

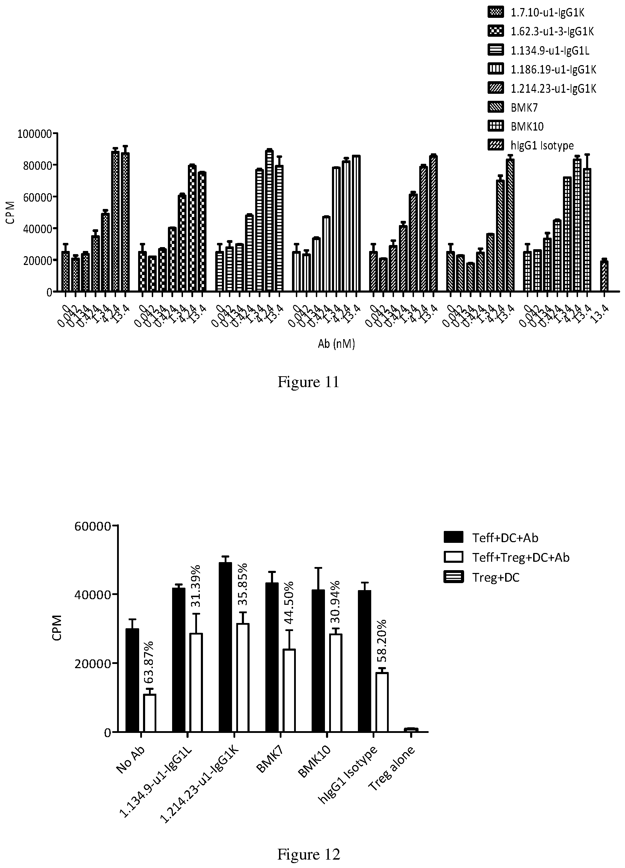

Figure 11 shows the effect of antibodies on anti-CD3 induced proliferation of primary human CD4⁺ T cells. It is demonstrated that the illustrative antibodies 1.7.10-ul-IgGlK, 1.62.3-ul-3-IgGlK, 1.134.9-ul-IgGlL, 1.186.19-ul-IgG1K and 1.214.23-ul-IgGlK enhanced proliferation of primary human CD4⁺ T cells.

### 4.9.3 Effect of human anti-OX40 antibodies on Tregs suppressive function

Tregs, a subpopulation of T cells, are a key immune modulator and play essential roles in maintaining self-tolerance. CD4⁺CD25⁺ regulatory T cells are suggested to be associated with tumor growth, as increased numbers of Tregs were found in patients with multiple cancers and were associated with poor prognosis. To directly assess the effect of human anti-OX40 antibodies on immune suppressive response, we compared the function of Tregs in the presence and absence of anti-OX40 antibodies. CD4⁺CD25⁺ Treg and CD4⁺CD25⁻ effector T (Teff) cells were separated using specific anti-CD25 microbeads (StemCell). Teff cells were seeded at 1×10⁵ cells/50 µL/well and co-cultured with 1×10⁵ Tregs/50 µL/well in 96-well round-bottom plates (BD). The cells were then stimulated with human allogeneic dendritic cells (DCs) induced from monocytes at 1 DC/10 Teff cells in the presence of cross linking antibody, as well as anti-OX40 antibodies. Either no antibody or isotype antibody was used as negative control. The co-cultures were incubated at 37 °C, 5% CO₂ for 5 days. The cell pellets were collected on day 5 to determine the Teff proliferation measured by ³H-thymidine incorporation.

Figure 12 shows the effect of antibodies on dendritic cells induced proliferation of primary human CD4⁺ T effector cells in the presence of Treg cells. The antibodies 1.134.9-ul-IgGlL and 1.214.23-ul-IgGlK can restore CD4⁺CD25⁻ T cell proliferation by reversing the suppressive function of regulatory T cells.

### 4.10 ADCC and CDC test:

OX40 is expressed on variety of cell types. In order to assess their ability to trigger Fc effector function, the anti-OX40 antibodies were evaluated whether they could induce ADCC and CDC effect on OX40 expressing cells.

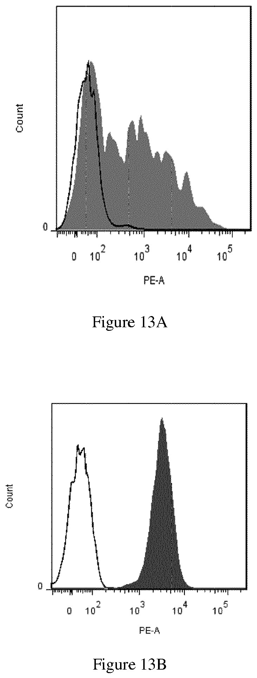

Figure 13A shows the expression of OX40 on activated human CD4⁺ T cells and Figure 13B shows the expression of OX40 on OX40 over-expressing Jurkat cells.

### 4.10.1 ADCC test:

Jurkat cells expressing OX40 or activated human CD4⁺ T cells, as target, and various concentrations of anti-OX40 antibodies were pre-incubated in 96-well round-bottom plate (BD) for 30 minutes; and then allogeneic PBMCs, as effector, were added at effector/target ratio of 50:1. The plate was kept at 37 °C, 5% CO₂ for 4 hours. Target cell lysis was determined by LDH-based Cytotoxicity Detection Kit (Roche). The absorbance at 492nm was read using a microplate reader (Molecular Device).

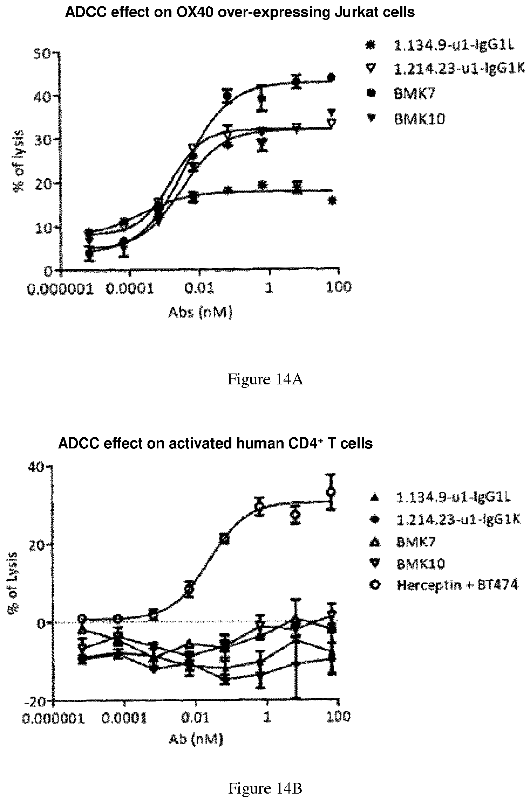

Figures 14A and 14B show the ADCC effect of OX40 antibodies on OX40 over-expressing Jurkat cells (Figure 14A) or activated human CD4⁺ T cells (Figure 14B), respectively. As shown in Figures 14A and 14B, the illustrative antibodies of the present disclosure, i.e., 1.134.9-ul-IgGlL and 1.214.23-ul-IgGlK, have low ADCC effect on OX40 over-expressing Jurkat cells and activated human CD4⁺ T cells.

### 4.10.2 CDC test:

Jurkat cells expressing OX40 or activated human CD4⁺ T cells, as target, and various concentrations of anti-OX40 antibodies were mixed in 96-well round-bottom plate (BD). Human complement was added at a final dilution of 1:50. The plate was kept at 37 °C, 5% CO₂ for 2 hours. Target cell lysis was determined by CellTiter-Glo (Promega). The luminescence was read using a microplate reader (Molecular Device).

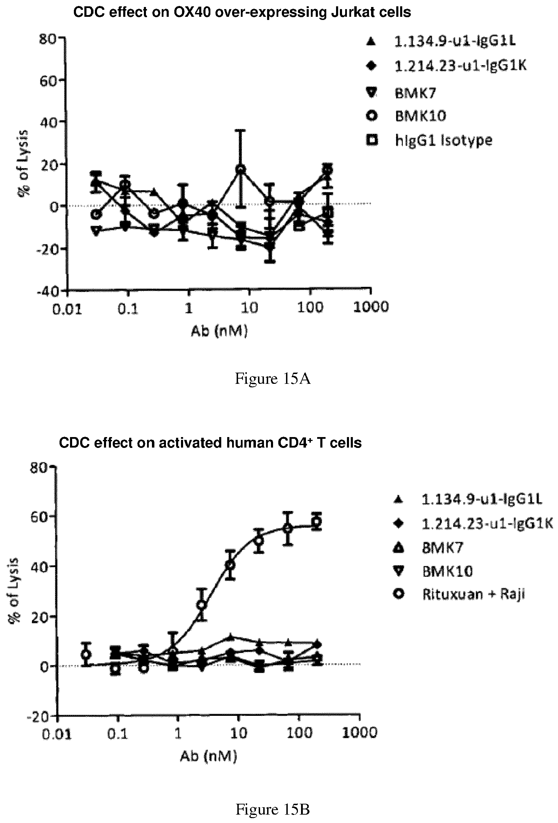

Figures 15A and 15B show the CDC effect of OX40 antibodies on OX40 over-expressing Jurkat cells (Figurel5A) or activated human CD4⁺ T cells (Figurel5B), respectively. As shown in Figures 15A and 15B, the illustrative antibodies of the present disclosure, i.e., 1.134.9-ul-IgGlL and 1.214.23-ul-IgGlK, have low CDC effect on OX40 over-expressing Jurkat cells and activated human CD4⁺ T cells.

### 4.11 Domain mapping

In order to examine the binding domain of OX40 antibodies, a series of human/mouse OX40 chimeric variants were used. OX40 antibodies specifically bind to human OX40, without cross-reactivity to mouse OX40 and human CD40, despite sharing 60% and 23% identity in their amino acid (alternatively, referred as "aa" herein) sequence respectively. Briefly, twenty-two variants (named as variant "xl", "x2" ..."x22") were constructed by replacing the following residues of the extracellular domain of human OX40 (hProl) with the corresponding mouse OX40 (mProl) amino acids or human CD40 amino acids (hPro40).
- Variant xl: xProl.FL-xl: CRDmox40_1 (Human OX40 aa 29 to 65 replace with the mouse counterparts)
- Variant x2: xProl.FL.x2: CRDmox40_2 (Human OX40 aa 66 to 107 replace with the mouse counterparts)
- Variant x3: xProl.FL-x3: CRDmox40_3 (Human OX40 aa 108 to 146 replace with the mouse counterparts)
- Variant x4: xProl.FL-x4: CRDmox40_4 (Human OX40 aa 147 to 214 replace with the mouse counterparts)
- Variant x5: xProl.FL-x5: CRDmox40_1-2 (Human OX40 aa 29 to 107 replace with the mouse counterparts)
- Variant x6: xProl.FL-x6: CRDmox40_2-3 (Human OX40 aa 66 to 146 replace with the mouse counterparts)
- Variant x7: xProl.FL-x7: CRDmox40_3-4 (Human OX40 aa 108 to 214 replace with the mouse counterparts)
- Variant x8: xProl.FL-x8: CRDmox40_1-3 (Human OX40 aa 29 to 146 replace with the mouse counterparts)
- Variant x9: xProl.FL-x9: CRDmox40_2-4 (Human OX40 aa 66 to 214 replace with the mouse counterparts)
- Variant x10: xPro1.FL-x10: CRDmox40_1,2,4 (Human OX40 aa 29 to 107 and 147 to 214 replace with the mouse counterparts)
- Variant x11: xPro1.FL-x11: CRDmox40_1,3,4 (Human OX40 aa 29 to 65 and 108 to 214 replace with the mouse counterparts)
- Variant x12: xPro1.FL-x12: CRDhcd40_1 (Human OX40 aa 29 to 65 replace with the human CD40 aa counterparts)
- Variant x13: xProl.FL-xl3: CRDhcd40_2 (Human OX40 aa 66 to 107 replace with the human CD40 aa counterparts)
- Variant xl4: xProl.FL-xl4: CRDhcd40_3 (Human OX40 aa 108 to 146 replace with the human CD40 aa counterparts)
- Variant x15: xPro1.FL-x15: CRD hcd40_4 (Human OX40 aa 147 to 214 replace with the human CD40 aa counterparts)
- Variant x16: xPro1.FL-x16: CRDhcd40_1-2 (Human OX40 aa 29 to 107 replace with the human CD40 aa counterparts)
- Variant x17: xPro1.FL-x17: CRDhcd40_2-3 (Human OX40 aa 66 to 146 replace with the human CD40 aa counterparts)
- Variant xl8: xProl.FL-xl8: CRDhcd40_3-4 (Human OX40 aa 108 to 214 replace with the human CD40 aa counterparts)
- Variant x19: xPro1.FL-x19: CRDhcd40_1-3 (Human OX40 aa 29 to 146 replace with the human CD40 aa counterparts)
- Variant x20: xProl.FL-x20: CRDhcd40_2-4 (Human OX40 aa 66 to 214 replace with the human CD40 aa counterparts)
- Variant x21: xPro1.FL-x21: CRDhcd40_1,2,4 (Human OX40 aa 29 to 107 and 147 to 214 replace with the human CD40 aa counterparts)
- Variant x22: xProl.FL-x22: CRDhcd40_1,3,4 (Human OX40 aa 29 to 65 and 108 to 214 replace with the human CD40 aa counterparts)

The twenty-two variants from "xl" to "x22" were cloned into pcDNA3.0 vector, and used for 293F cells transfection. Briefly, 293F cells were diluted to a density of 1×10⁶ cells/mL with FreeStyle 293F medium and aliquots of 3 mL per well were added to 24-well plate. Transfections were performed using 293fectin reagent (Life Technologies). For each transfection, 3 µg of DNA were diluted in 150 µL Opti-MEMI reduced serum medium (life Technologies), and then combined with 6 µL 293fectin reagent pre-diluted in 150 µL Opti-MEMI reduced serum medium. The DNA/Lipofectamine mixture was allowed to stand at 25 °C for 20 min before being added to the culture. The transfected cells were analyzed by flow cytometry 48h post-transfection.

Binding of antibodies to chimeric OX40 variants was analyzed by flow cytometry. Briefly, 1 µg/mL antibodies, except BMK10 is 2 µg/mL, were incubated with chimeric OX40 expressed transfected 293F cells for 1 hour at 4 °C, and then incubated with 3 µg/mL goat anti-human IgG Fc R-PE (Jackson ImmunoResearch Lab) for 40 min at 4 °C. Cells were analyzed using flow cytometer.

Results are shown in Tables 7-9 below.

**Table 7. Binding of the variants to OX40 antibodies**

| 1.134.9-ul-IgGlL | | | 1.214.23-ul-IgGlK | | |
|---|---|---|---|---|---|
| | MFI | PE+ % | | MFI | PE+ % |
| 293F | 29.2 | 0 | 293F | 28.6 | 0 |
| 293F+1.134.9-ul-IgG1L+ goat anti-human IgG Fc R-PE | 28.8 | 0.106 | 293F+1.214.23-ul-IgGlK +goat anti-human IgG Fc R-PE | 26.7 | 0.158 |
| hProl | 9162 | 85.2 | hProl | 8324 | 89.3 |
| mProl | 44.4 | 0.937 | mProl | 49.9 | 0.315 |
| x1 | 6442 | 75.3 | x1 | 5421 | 82.9 |
| x2 | 49.5 | 0.904 | x2 | 23300 | 92.8 |
| x3 | 3805 | 66.1 | x3 | 2251 | 70 |
| x4 | 24100 | 96.6 | x4 | 23200 | 97.7 |
| x5 | 61 | 1.73 | x5 | 21700 | 96.5 |
| x6 | 211 | 6.75 | x6 | 113 | 2.31 |
| x7 | 9157 | 80.3 | x7 | 6542 | 81.5 |
| x8 | 43.2 | 0.678 | x8 | 42.6 | 0.743 |
| x9 | 44.1 | 0.668 | x9 | 80.1 | 0.244 |
| x10 | 42.7 | 0.624 | x10 | 19600 | 94.4 |
| x11 | 9403 | 74 | x11 | 6260 | 70.8 |

**Table 8. Binding of the variants to the benchmark antibody BMK1 or BMK5**

| BMK1 | | | BMK5 | | |
|---|---|---|---|---|---|
| | MFI | PE+ % | | MFI | PE+ % |
| 293F | 28.3 | 0.024 | 293F | 28.4 | 0.023 |
| 293F+BMKl+goat anti-human IgG Fc R-PE | 25.1 | 0.063 | 293F+BMK5+goat anti-human IgG Fc R-PE | 24.4 | 0.081 |
| hProl | 8101 | 89.1 | hProl | 9641 | 89.2 |
| mProl | 33.7 | 0.189 | mProl | 31.6 | 0.04 |
| x1 | 43.7 | 1.22 | x1 | 6029 | 80.4 |
| x2 | 14800 | 94.3 | x2 | 53.9 | 1.89 |
| x3 | 3334 | 72.7 | x3 | 4412 | 73.7 |
| x4 | 22300 | 97.9 | x4 | 25900 | 98.3 |
| x5 | 103 | 2.02 | x5 | 66.1 | 2.65 |
| x6 | 12600 | 90.4 | x6 | 134 | 8.43 |
| x7 | 8118 | 85.8 | x7 | 9740 | 88.8 |
| x8 | 39.4 | 0.31 | x8 | 63.8 | 0.652 |
| x9 | 12800 | 93.2 | x9 | 38.6 | 0.198 |
| x10 | 58.2 | 0.43 | x10 | 33.5 | 0.12 |
| x11 | 105 | 4.44 | x11 | 33.5 | 0.12 |

**Table 9. Binding of the variants to the benchmark antibody BMK7 or BMK10**

| BMK7 | | | BMK10 | | |
|---|---|---|---|---|---|
| | MFI | PE+ % | | MFI | PE+ % |
| 293F | 22.5 | 0 | 293F | 22.7 | 0.045 |
| 293F+BMK7+goat anti-human IgG Fc R-PE | 21.8 | 0.147 | 293F+BMK10+goat anti-human IgG Fc R-PE | 21.9 | 0.084 |
| hProl | 18900 | 97.2 | hProl | 27600 | 98.3 |
| mProl | 157 | 21.2 | mProl | 4997 | 91.6 |
| x1 | 14900 | 91.6 | x1 | 18100 | 92.6 |
| x2 | 23900 | 92.8 | x2 | 33700 | 95.1 |
| x3 | 772 | 56.7 | x3 | 4931 | 87 |
| x4 | 18200 | 99.6 | x4 | 29500 | 99.7 |
| x5 | 26300 | 98.9 | x5 | 39700 | 99.5 |
| x6 | 7066 | 88.9 | x6 | 14000 | 94.1 |
| x7 | 775 | 49.6 | x7 | 6816 | 96.6 |
| x8 | 2629 | 95.7 | x8 | 16700 | 98.4 |
| x9 | 2975 | 82.5 | x9 | 19700 | 97.1 |
| x10 | 16400 | 97.9 | x10 | 24400 | 98.6 |
| x11 | 1144 | 37.8 | x11 | 8204 | 91.5 |
| x12 | 7969 | 96.8 | x12 | 10300 | 97.8 |
| x13 | 10300 | 95.3 | x13 | 15000 | 96.5 |
| x14 | 25.7 | 0.068 | x14 | 26.9 | 0.113 |
| x15 | 22300 | 99 | x15 | 31300 | 99.5 |
| x16 | 20000 | 89.6 | x16 | 28100 | 91.8 |
| xl7 | 24.2 | 0.086 | xl7 | 27.4 | 0.16 |
| x18 | 27.5 | 0.042 | x18 | 28 | 0.176 |
| x19 | 26.9 | 0.043 | x19 | 27.2 | 0.22 |
| x20 | 25.4 | 0.066 | x20 | 24.2 | 0.064 |
| x21 | 171 | 20.8 | x21 | 2111 | 94.5 |
| x22 | 23.8 | 0 | x22 | 32.8 | 0.806 |
| hPro40 | 60.3 | 0.291 | hPro40 | 28.8 | 0.068 |

Table 10 shows the domain of OX40 (colored in grey) involved in the antigen binding.

**Table 10. the domain of OX40 (colored in grey) involved in the antigen binding**

| Abs | CRD1 | CRD2 | | CRD3 | | CRD4 |
|---|---|---|---|---|---|---|
| 1.214.23-u1-IgG1K | | | | | | |
| 1.134.9-u1-IgG1L | | | | | | |
| BMK1 | | | | | | |
| BMK5 | | | | | | |
| BMK7 | | | | | | |
| BMK10 | | | | | | |

| | | | | | | |
|---|---|---|---|---|---|---|
| Note: CRD refers to a cysteine-rich domain, wherein "CRD1" refers to amino acids 29-65 of human OX4, "CRD2" refers to amino acids 66-107 of human OX4, "CRD3" refers to amino acids 108-146 of human OX4, and "CRD4" refers to amino acids 147-214 of human OX40. | | | | | | |

### 4.12 OX40 antibody inhibits the growth of MC38 colon carcinoma in human OX40 transgenic model

This study evaluated the *in vivo* anti-tumor efficacy of antibody 1.134.9-ul-IgGlL in MC38 colon cancer model in OX40 humanized B-hTNFRSF4 mice.

OX40 humanized B-hTNFRSF4 mice were purchased from Biocytogen Co., Ltd. The mice were kept in individual ventilation cages at constant temperature and humidity with 5 animals in each cage.

The MC38 cells were maintained *in vitro* as a monolayer culture in DMEM medium supplement with 10% fetal bovine serum, 2 mM L-glutamine, 100 U/mL penicillin and 100 µg/mL streptomycin at 37 °C in an atmosphere of 5% CO₂ in air. The tumor cells were routinely subcultured twice weekly by trypsin-EDTA treatment. The cells growing in an exponential growth phase were harvested and counted for tumor inoculation.

Each mouse was inoculated subcutaneously at the right axillary (lateral) with MC38 tumor cells (3×10⁵) in 0.1 mL of PBS for tumor development. The animals were randomly grouped when the average tumor volume reached 65 mm³, then treatment started for the efficacy study. All test antibodies and control antibodies were administered by intraperitoneal (IP) injection twice weekly (BIW) for three weeks ("BIW × 3"). Detailed information is provided in Table 11.

**Table 11.**

| **Group** | **Animal Number** | **Sex** | **Treatment** | **Dose (mg/kg body weight)** | **Dosing route** | **Schedule** |
|---|---|---|---|---|---|---|
| 1 | 8 | female | hIgG1 Isotype | 5 | IP | BIW × 3 |
| 2 | 8 | female | 1.134.9-u1-IgG1L | 5 | IP | BIW × 3 |
| 3 | 8 | female | 1.134.9-u1-IgG1L | 1 | IP | BIW × 3 |
| 4 | 8 | female | 1.134.9-u1-IgG1L | 0.2 | IP | BIW × 3 |

All the procedures related to animal handing, care and the treatment in the study were performed according to the guidelines approved by the Institutional Animal Care and Use Committee (IACUC) of WuXi Apptec following the guidance of Association for Assessment and Accreditation of Laboratory Animal Care (AAALAC). At the time of routine monitoring, the animals were daily checked for any effects of tumor growth and treatments on normal behavior such as mobility, food and water consumption (by looking only), body weight gain/loss (body weight were measured once every day), eye/hair matting and any other abnormal effect as stated in the protocol. Death and observed clinical signs were recorded on the basis of the numbers of animals within each subset.

Tumor sizes was measured three times weekly in two dimensions using a caliper, and the volume was measured in mm³ using the formula: V = 0.5a×b² where a and b are the long and short diameters of the tumor, respectively. The tumor sizes are then used for the calculations of T/C (%) values. T/C (%) of relative tumor proliferation rate was calculated using the formula: T/C % = T_{RTV}/C_{RTV}×100% (T_{RTV} means treatment group relative tumor volume; C_{RTV} means negative control relative tumor volume). The relative tumor volume was calculated based on the tumor measurements, the calculation formula was: RTV = Vₜ/V₀, V₀ is the average tumor volume on the day of treatment start, Vₜ is the average tumor volume of one time measure, T_{RTV} used data of day the same with C_{RTV}.

TGI is calculated for each group using the formula: TGI (%) = [1-(Tᵢ-T₀)/(Vᵢ-V₀)]×100; Tᵢ is the average tumor volume of a treatment group on a given day, T₀ is the average tumor volume of the treatment group on the first day of treatment, Vᵢ is the average tumor volume of the vehicle control group on the same day with Tᵢ and V₀ is the average tumor volume of the vehicle group on the first day of treatment.

Summary statistics, including mean and the standard error of the mean (SEM), are provided for the tumor volume of each group at each time point. Statistical analysis of difference in tumor volume among the groups and the analysis of drug interaction were conducted on the data obtained at the best therapeutic time point. One-way ANOVA was performed to compare tumor volume between three or more groups. When a significant F-statistics (a ratio of treatment variance to the error variance) was obtained, comparisons between groups were carried out with Games-Howell test; if not, Dunnett-t (2-sided) would be used. All data were analyzed using SPSS 17.0. A p value of less than 0.05 (p<0.05) was considered to be statistically significant.

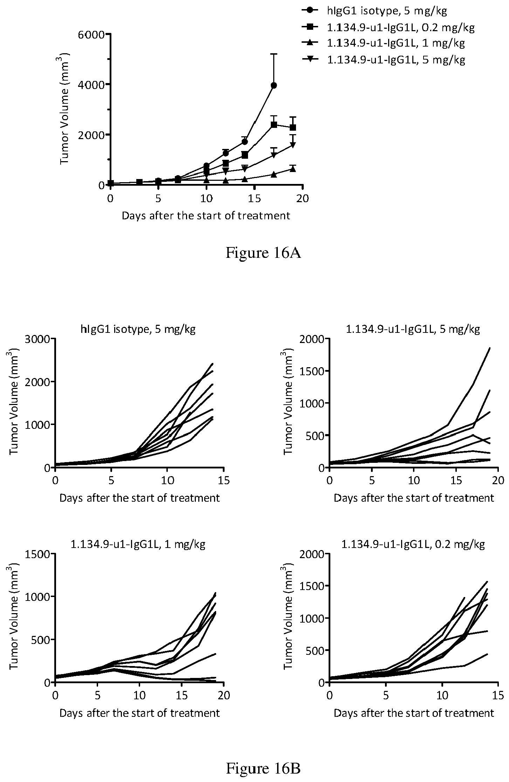

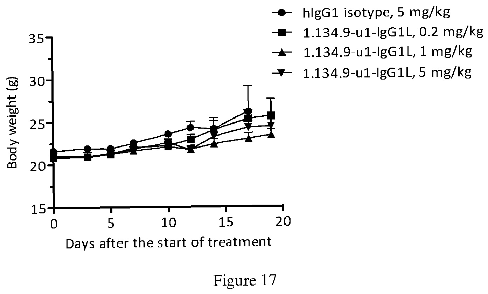

Experimental data are shown in Table 12 and 13 as well as Figures 16 and 17.

**Table 12. The mean tumor volumes over time in MC38 tumor-bearing mice post administration of 1.134.9-u1-IgG1L**

| Days after treatment | Tumor volume (mm³) | | | |
|---|---|---|---|---|
| | hIgG1 isotype (5 mg/kg) | 1.134.9-u1-IgG1L (5 mg/kg) | 1.134.9-u1-IgG1L (1 mg/kg) | 1.134.9-u1-IgG1L (0.2 mg/kg) |
| 0 | 66±4 | 65±4 | 65±3 | 65±4 |
| 3 | 103±6 | 103±7 | 104±4 | 104±9 |
| 5 | 164±10 | 131±17 | 133±8 | 137±12 |
| 7 | 260±19 | 178±31 | 191±14 | 228±29 |
| 10 | 750±97 | 376±95 | 194±32 | 539±73 |
| 12 | 1246±146 | 511±139 | 181±42 | 841±118 |
| 14 | 1709±195 | 611±165 | 227±57 | 1157±151 |
| 17 | 3955±1246 | 1168±295 | 417±101 | 2387±354 |
| 19 | - | 1574±415 | 625±150 | 2274±424 |

**Table 13. The tumor growth inhibition rate at day12 post administration of 1.134.9-u1-IgG1L**

| Group | Animal number | Tumor volume (mm³) | T/C (%) | TGI (%) | P value |
|---|---|---|---|---|---|
| hIgG1 isotype (5 mg/kg) | 8 | 1246±146 | - | - | - |
| 1.134.9-u1-IgG1L (5 mg/kg) | 8 | 511±139 | 41.29 | 62.26 | 0.019 |
| 1.134.9-u1-IgG1L (1 mg/kg) | 8 | 181±42 | 14.61 | 90.17 | 0.001 |
| 1.134.9-u1-IgG1L (0.2 mg/kg) | 8 | 841±118 | 67.98 | 34.24 | 0.255 |

It can be seen that the OX40 antibody 1.134.9-u1-IgG1L produced a significant antitumor activity against the MC38 colon carcinoma bearing B-hTNFRSF4 mice, and the antibody was well tolerated by the tumor-bearing animals.

### 4.13 Epitope mapping

In order to examine the binding epitope of OX40 antibodies, alanine scanning experiments on human OX40 were conducted and their effect to antibody binding was evaluated. Alanine residues on human OX40 were mutated to glycine codons, and all other residues (except cysteine residues and the solvent accessible surface areas< 10 of OX40 amino acid based on the OX40-OX40R complex (PDB: 2HEV) (SASA > 10 sets as surface amino acid)) were mutated to alanine codons. For each residue of the human OX40 extracellular domain, point amino acid substitutions were made using two sequential PCR steps. A pcDNA3.3-OX40-ECD.His plasmid that encodes ECD of human OX40 and a C-terminal His-tag was used as template, and a set of mutagenic primer was used for first step PCR using the QuikChange lightning multi-site-directed mutagenesis kit (Agilent technologies, Palo Alto, CA). Dpn I endonuclease was used to digest the parental template after mutant strand synthesis reaction. In the second-step PCR, linear DNA expression cassette which composed of CMV promoter, an extracellular domain of OX40, a His-tag and a herpes simplex virus thymidine kinase (TK) polyadenylation was amplifies and transiently expressed in 293F cells at 37 °C (life Technologies, Gaithersburg, MD), quantified by His-tag quantification ELISA.

Monoclonal antibody 1.134.9-u1-IgG1L (2 µg/mL) was coated in plates for ELISA binding assay. After interacting with the supernatant that contains quantified OX40 mutants or human OX40-ECD.His protein, HRP conjugated anti-His antibody (1:5000, GenScript-A00612, CHN) was added as detection antibody. Absorbance was normalized according to the average of control mutants. After setting an additional cutoff to the binding fold change (<0.75), the final determined epitope residues were identified by considering domain mapping, epitope mapping and crystal structure, which did not include the amino acids contributing to structure stability, such as a.a. belonging to CRD3 & CRD4.

The normalized fold change of OX40 point mutations on antibody binding was shown in Table 14. Hotspots were identified by considering domain mapping, alanine scanning (cutoff: binding fold change <0.75, SASA>10) and crystal structure (PDB: 2HEV), which did not include the amino acids contributing to structure stability, such as a.a. belongs to CRD3 & CRD4. As shown in Table 15, there are eight hotspot positions to 1.134.9-u1-IgG1L.

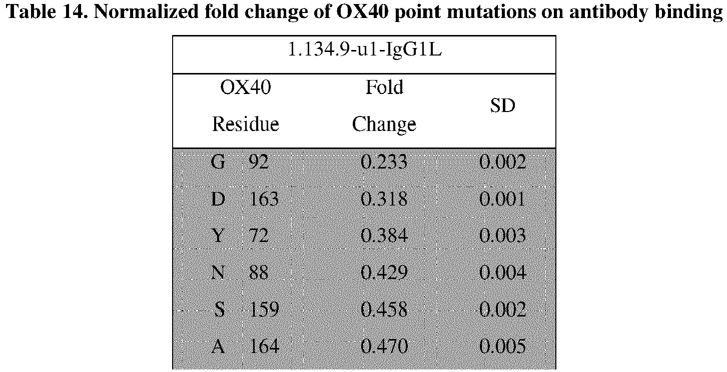

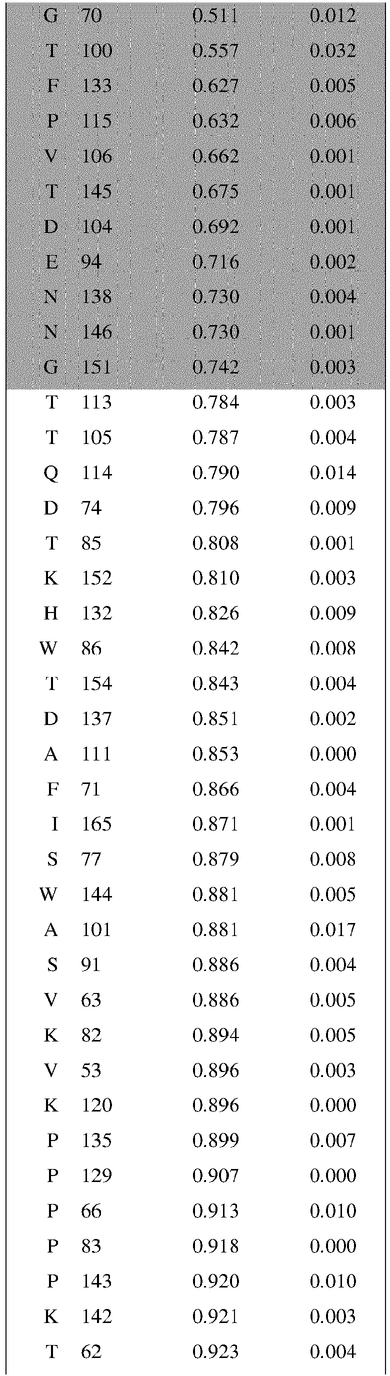

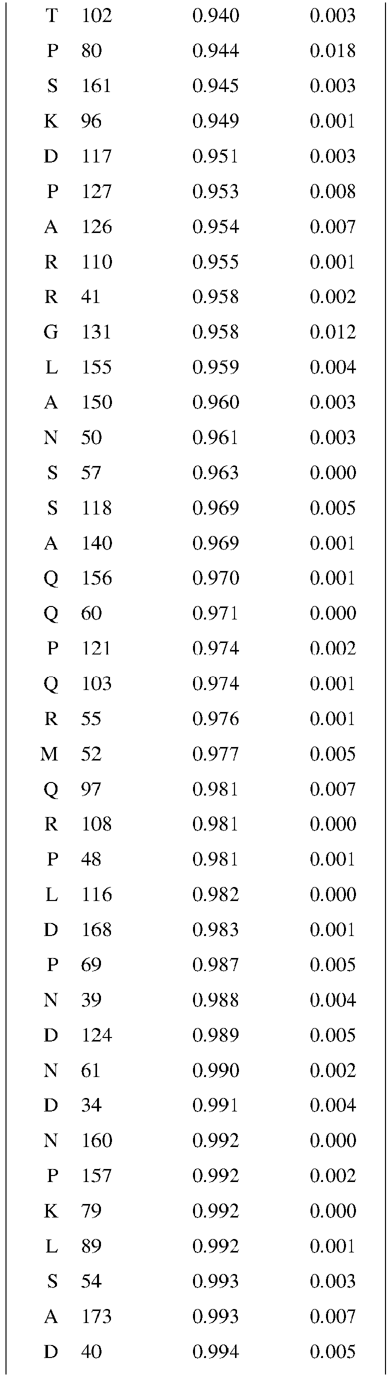

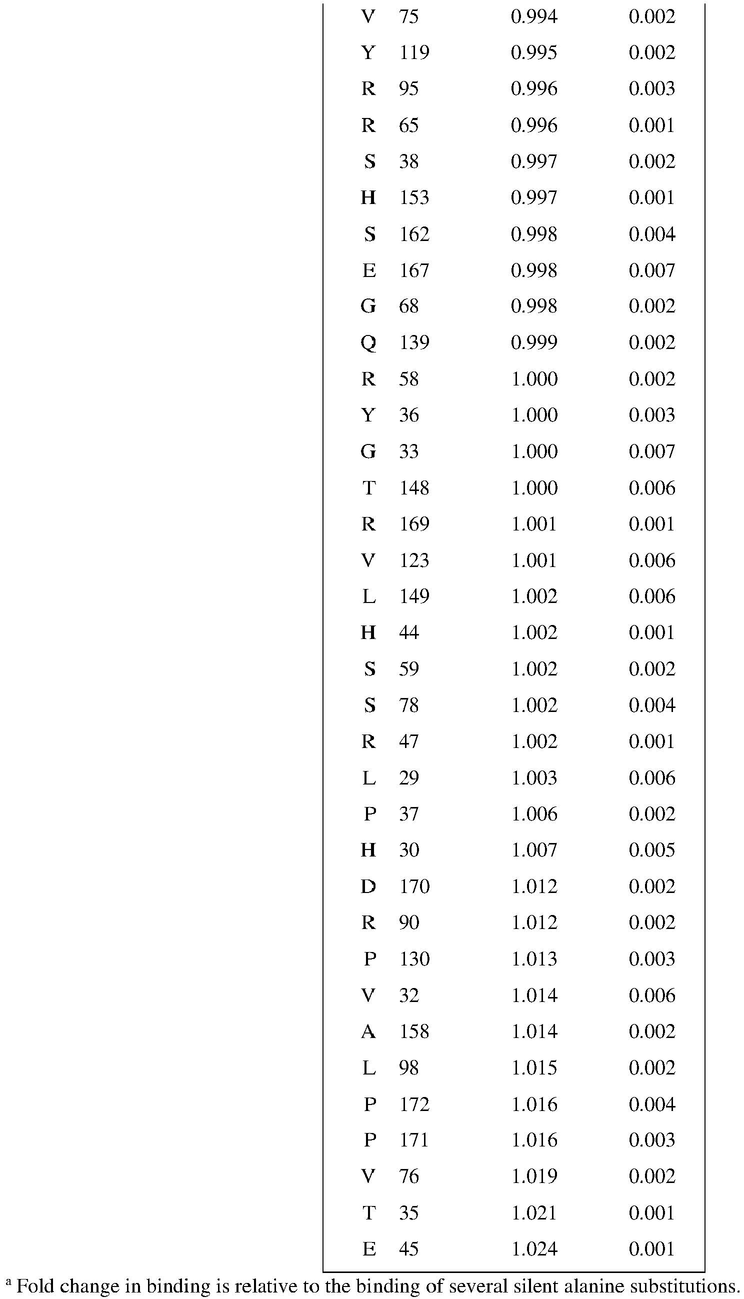

**Table 15. Eight hotspot positions to 1.134.9-u1-IgG1L**

| 1.134.9-u1-IgG1L | |
|---|---|
| Residue | Location |
| G 70 | CRD2 |
| Y 72 | CRD2 |
| N 88 | CRD2 |
| G 92 | CRD2 |
| E 94 | CRD2 |
| T 100 | CRD2 |
| D 104 | CRD2 |
| V 106 | CRD2 |
| Cutoff fold change < 0.75, SASA > 10. | |

Those skilled in the art will further appreciate that the present invention may be embodied in other specific forms without departing from the central attributes thereof. In that the foregoing description of the present invention discloses only exemplary embodiments thereof, it is to be understood that other variations are contemplated as being within the scope of the present invention. Accordingly, the present invention is not limited to the particular embodiments that have been described in detail herein. Rather, reference should be made to the appended claims as indicative of the scope and content of the invention.

## Claims

1. An isolated antibody or the antigen-binding portion thereof that binds to OX40, wherein the isolated antibody or the antigen-binding portion thereof comprises:
(a) a heavy chain variable region CDR1 comprising or consisting of SEQ ID NO: 15;
(b) a heavy chain variable region CDR2 comprising or consisting of SEQ ID NO: 17;
(c) a heavy chain variable region CDR3 comprising or consisting of SEQ ID NO: 19;
(d) a light chain variable region CDR1 comprising or consisting of SEQ ID NO: 16;
(e) a light chain variable region CDR2 comprising or consisting of SEQ ID NO: 18; and
(f) a light chain variable region CDR3 comprising or consisting of SEQ ID NO: 20.

2. The isolated antibody or the antigen-binding portion thereof of claim 1, wherein the isolated antibody or the antigen-binding portion thereof comprises:
(A) a heavy chain variable region:
(i) comprising the amino acid sequence of SEQ ID NO: 39;
(ii) comprising an amino acid sequence at least 85%, at least 90%, or at least 95% identical to the amino acid sequence of SEQ ID NO: 39; or
(iii) comprising an amino acid sequence with addition, deletion and/or substitution of one or more amino acids compared with the amino acid sequence of SEQ ID NO: 39; and
(B) a light chain variable region:
(i) comprising the amino acid sequence of SEQ ID NO: 40;
(ii) comprising an amino acid sequence at least 85%, at least 90%, or at least 95% identical to the amino acid sequence of SEQ ID NO: 40; or
(iii) comprising an amino acid sequence with addition, deletion and/or substitution of one or more amino acids compared with the amino acid sequence of SEQ ID NO: 40.

3. The isolated antibody or the antigen-binding portion thereof of claim 2, wherein the isolated antibody or the antigen-binding portion thereof comprises:
a heavy chain variable region comprising the amino acid sequence of SEQ ID NO: 39 and a light chain variable region comprising the amino acid sequence of SEQ ID NO: 40.

4. The isolated antibody or the antigen-binding portion thereof of any of the preceding claims, wherein the antibody is a monoclonal antibody, a chimeric antibody, a humanized antibody, or a fully human monoclonal antibody.

5. A fusion of the isolated antibody or the antigen-binding portion thereof of any of the preceding claims, wherein the isolated antibody or the antigen-binding portion thereof is fused to a constant region of an IgG, preferably a constant region of a human IgG, more preferably a constant region of a human IgG1 or human IgG4.

6. An isolated nucleic acid molecule, comprising a nucleic acid sequence encoding the heavy chain variable region and the light chain variable region of the isolated antibody as defined in any of claims 1-5.

7. A vector comprising the nucleic acid molecule of claim 6.

8. A host cell comprising the vector of claim 7.

9. A pharmaceutical composition comprising the isolated antibody or the antigen-binding portion thereof as defined in any of claims 1-4 and a pharmaceutically acceptable carrier.

10. A method for preparing the isolated antibody or the antigen-binding portion thereof as defined in any of claims 1-4 comprising the steps of:
- expressing the isolated antibody or antigen-binding portion thereof as defined in any of claims 1-4 in the host cell of claim 8; and
- isolating the antibody or antigen-binding portion thereof from the host cell.

11. The isolated antibody or the antigen-binding portion thereof as defined in any of claims 1-4 for use in treating or preventing proliferative disorders, such as cancers, inflammatory disease or infectious diseases.

12. A kit, comprising a container comprising the isolated antibody or the antigen-binding portion thereof as defined in any of claims 1-4.


SEQUENCE LISTING

<110>  WuXi Biologics (Shanghai) Co., LTD.
       Wuxi Biologics Ireland Limited

<120>  Fully human antibodies against OX40, method for preparing the
       same, and use thereof

<130>  IEC196027PCT

<150>  PCT/CN2018/086574
<151>  2018-05-11

<150>  CN201810529840.5
<151>  2018-05-29

<160>  56

<170>  PatentIn version 3.5

<210>  1
<211>  10
<212>  PRT
<213>  Artificial Sequence

<220>
<223>  HCDR1 of 1.7.10-u1-IgG1K

<400>  1

Gly Phe Thr Phe Ser Asp Tyr Tyr Met Ser
1               5                   10


<210>  2
<211>  11
<212>  PRT
<213>  artificial sequence

<220>
<223>  LCDR1 of 1.7.10-u1-IgG1K

<400>  2

Arg Ala Ser Gln Gly Ile Ser Ser Trp Leu Ala
1               5                   10


<210>  3
<211>  17
<212>  PRT
<213>  artificial sequence

<220>
<223>  HCDR2 of 1.7.10-u1-IgG1K

<400>  3

Tyr Ile Ser Gly Ser Gly Asn Thr Ile Tyr Tyr Ala Asp Ser Val Lys
1               5                   10                  15


Gly

<210>  4
<211>  7
<212>  PRT
<213>  artificial sequence

<220>
<223>  LCDR2 of 1.7.10-u1-IgG1K

<400>  4

Ala Ala Ser Ser Leu Gln Gly
1               5


<210>  5
<211>  12
<212>  PRT
<213>  artificial sequence

<220>
<223>  HCDR3 of 1.7.10-u1-IgG1K

<400>  5

Glu Arg Gly Ala Ala Gly Thr Gly Trp Phe Asp Pro
1               5                   10


<210>  6
<211>  9
<212>  PRT
<213>  artificial sequence

<220>
<223>  LCDR3 of 1.7.10-u1-IgG1K

<400>  6

Gln Gln Val Asn Ser Phe Pro Trp Thr
1               5


<210>  7
<211>  12
<212>  PRT
<213>  artificial sequence

<220>
<223>  HCDR1 of 1.62.3-u1-IgG1K

<400>  7

Gly Gly Ser Ile Ser Asn Gly Gly Tyr Tyr Trp Ser
1               5                   10


<210>  8
<211>  17
<212>  PRT
<213>  artificial sequence

<220>
<223>  LCDR1 of 1.62.3-u1-IgG1K

<400>  8

Lys Ser Ser Gln Ser Val Val Phe Ser Ser Asn Asn Lys Ile Cys Leu
1               5                   10                  15

Ala

<210>  9
<211>  16
<212>  PRT
<213>  artificial sequence

<220>
<223>  HCDR2 of 1.62.3-u1-IgG1K or 1.62.3-u1-3-IgG1K

<400>  9

Tyr Ile Tyr Tyr Ser Gly Ser Thr Tyr Tyr Asn Pro Ser Leu Lys Ser
1               5                   10                  15

<210>  10
<211>  7
<212>  PRT
<213>  artificial sequence

<220>
<223>  LCDR2 of 1.62.3-u1-IgG1K or 1.62.3-u1-3-IgG1K

<400>  10

Trp Ser Ser Thr Arg Glu Ser
1               5

<210>  11
<211>  10
<212>  PRT
<213>  artificial sequence

<220>
<223>  HCDR3 of 1.62.3-u1-IgG1K or 1.62.3-u1-3-IgG1K

<400>  11

Asp Glu Trp Glu Leu Arg Gly Phe Asp Tyr
1               5                   10

<210>  12
<211>  9
<212>  PRT

<213>  artificial sequence

<220>
<223>  LCDR3 of 1.62.3-u1-IgG1K or 1.62.3-u1-3-IgG1K


<400>  12

Gln Gln Tyr Tyr Ser Ser Pro Trp Thr
1               5


<210>  13
<211>  12
<212>  PRT
<213>  artificial sequence

<220>
<223>  HCDR1 of 1.62.3-u1-3-IgG1K

<400>  13

Gly Gly Ser Ile Ser Asn Ala Gly Tyr Tyr Trp Ser
1               5                   10


<210>  14
<211>  17
<212>  PRT
<213>  artificial sequence

<220>
<223>  LCDR1 of 1.62.3-u1-3-IgG1K

<400>  14

Lys Ser Ser Gln Ser Val Val Phe Ser Ser Asn Asn Lys Ile Ser Leu
1               5                   10                  15


Ala




<210>  15
<211>  11
<212>  PRT
<213>  artificial sequence

<220>
<223>  HCDR1 of 1.134.9-u1-IgG1L

<400>  15

Gly Gly Ser Ile Ser Ser Tyr Asn Trp Trp Ser
1               5                   10


<210>  16
<211>  11
<212>  PRT
<213>  artificial sequence

<220>
<223>  LCDR1 of 1.134.9-u1-IgG1L

<400>  16

Gln Gly Asp Asn Leu Arg Thr Tyr Tyr Ala Ser
1               5                   10


<210>  17
<211>  16
<212>  PRT
<213>  artificial sequence

<220>
<223>  HCDR2 of 1.134.9-u1-IgG1L

<400>  17

Glu Ile Tyr His Gly Gly Asn Thr Asn Tyr Asn Pro Ser Leu Lys Ser
1               5                   10                  15


<210>  18
<211>  7
<212>  PRT
<213>  artificial sequence

<220>
<223>  LCDR2 of 1.134.9-u1-IgG1L

<400>  18

Gly Arg Asn Lys Arg Pro Ser
1               5


<210>  19
<211>  13
<212>  PRT
<213>  artificial sequence

<220>
<223>  HCDR3 of 1.134.9-u1-IgG1L

<400>  19

Ala Pro Gly Asp Trp Gly Gly Ser Pro Tyr Phe Asp Phe
1               5                   10


<210>  20
<211>  11
<212>  PRT
<213>  artificial sequence

<220>
<223>  LCDR3 of 1.134.9-u1-IgG1L

<400>  20

Asn Ser Arg Asp Ser Ser Gly Asn Pro Val Val
1               5                   10

<210>  21
<211>  10
<212>  PRT
<213>  artificial sequence

<220>
<223>  HCDR1 of 1.186.19-u1-IgG1K

<400>  21

Gly Phe Thr Phe Ser Asp Tyr Tyr Met Gly
1               5                   10


<210>  22
<211>  11
<212>  PRT
<213>  artificial sequence

<220>
<223>  LCDR1 of 1.186.19-u1-IgG1K

<400>  22

Arg Ala Ser Gln Gly Ile Ser Ser Trp Leu Ala
1               5                   10


<210>  23
<211>  17
<212>  PRT
<213>  artificial sequence

<220>
<223>  HCDR2 of 1.186.19-u1-IgG1K

<400>  23

Tyr Ile Ser Gly Ser Gly Asn Thr Ile Tyr Tyr Ala Asp Ser Val Lys
1               5                   10                  15


Gly



<210>  24
<211>  7
<212>  PRT
<213>  artificial sequence

<220>
<223>  LCDR2 of 1.186.19-u1-IgG1K

<400>  24

Ala Ala Ser Ser Leu Gln Gly
1               5


<210>  25

<211>  12
<212>  PRT
<213>  artificial sequence

<220>
<223>  HCDR3 of 1.186.19-u1-IgG1K

<400>  25

Glu Arg Gly Ala Ala Gly Ala Gly Trp Phe Asp Pro
1               5                   10


<210>  26
<211>  9
<212>  PRT
<213>  artificial sequence

<220>
<223>  LCDR3 of 1.186.19-u1-IgG1K

<400>  26

Gln Gln Val Asn Ser Phe Pro Trp Thr
1               5


<210>  27
<211>  11
<212>  PRT
<213>  artificial sequence

<220>
<223>  HCDR1 of 1.214.23-u1-IgG1K

<400>  27

Gly Gly Ser Ile Ser Asn Arg Asn Trp Trp Ser
1               5                   10


<210>  28
<211>  11
<212>  PRT
<213>  artificial sequence

<220>
<223>  LCDR1 of 1.214.23-u1-IgG1K

<400>  28

Arg Ala Ser Gln Asp Ile Asn Ser Tyr Leu Ala
1               5                   10


<210>  29
<211>  16
<212>  PRT
<213>  artificial sequence

<220>
<223>  HCDR2 of 1.214.23-u1-IgG1K

<400>  29

Glu Ile Phe His Ser Gly Asn Thr Asn Tyr Asn Pro Ser Leu Lys Ser
1               5                   10                  15

<210>  30
<211>  7
<212>  PRT
<213>  artificial sequence

<220>
<223>  LCDR2 of 1.214.23-u1-IgG1K

<400>  30

Ala Ala Ser Ser Leu Gln Ser
1               5

<210>  31
<211>  8
<212>  PRT
<213>  artificial sequence

<220>
<223>  HCDR3 of 1.214.23-u1-IgG1K

<400>  31

Ser Phe Ala Val Ala Leu Asp Ser
1               5

<210>  32
<211>  9
<212>  PRT
<213>  artificial sequence

<220>
<223>  LCDR3 of 1.214.23-u1-IgG1K

<400>  32

Gln Gln Leu Phe Ser Tyr Pro Ile Thr
1               5

<210>  33
<211>  121
<212>  PRT
<213>  artificial sequence

<220>
<223>  VH of 1.7.10-u1-IgG1K

<400>  33

Gln Val His Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Tyr

20                  25                  30

Tyr Met Ser Trp Ile Arg Gln Ala Pro Gly Lys Gly Leu Glu Trp Val
        35                  40                  45

Ser Tyr Ile Ser Gly Ser Gly Asn Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Asp Asp Thr Ala Val Tyr Phe Cys
                85                  90                  95

Ala Arg Glu Arg Gly Ala Ala Gly Thr Gly Trp Phe Asp Pro Trp Gly
            100                 105                 110

Gln Gly Thr Leu Val Thr Val Ser Ser
        115                 120

<210>  34
<211>  107
<212>  PRT
<213>  artificial sequence

<220>
<223>  VL of 1.7.10-u1-IgG1K

<400>  34

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Gly Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Asn Ser Phe Pro Trp
                85                  90                  95

Thr Phe Gly Gln Gly Thr Lys Val Glu Ile Lys
            100                 105

<210>  35
<211>  120
<212>  PRT
<213>  artificial sequence

<220>
<223>  VH of 1.62.3-u1-IgG1K
<400>  35

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Asn Gly
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Asp Glu Trp Glu Leu Arg Gly Phe Asp Tyr Trp Gly Gln
            100                 105                 110

Gly Thr Leu Val Thr Val Ser Ser
        115                 120


<210>  36
<211>  113
<212>  PRT
<213>  artificial sequence

<220>
<223>  VL of 1.62.3-u1-IgG1K

<400>  36

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Val Phe Ser
            20                  25                  30

Ser Asn Asn Lys Ile Cys Leu Ala Trp Tyr Gln Gln Lys Pro Gly Gln
        35                  40                  45

Pro Pro Lys Leu Leu Ile Tyr Trp Ser Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Ser Pro Trp Thr Phe Gly Gln Gly Thr Lys Val Glu Ile
            100                 105                 110

Lys

<210>  37
<211>  120
<212>  PRT
<213>  artificial sequence

<220>
<223>  VH of 1.62.3-u1-3-IgG1K

<400>  37

Gln Val Gln Leu Gln Glu Ser Gly Pro Gly Leu Val Lys Pro Ser Gln
1               5                   10                  15

Thr Leu Ser Leu Thr Cys Thr Val Ser Gly Gly Ser Ile Ser Asn Ala
            20                  25                  30

Gly Tyr Tyr Trp Ser Trp Ile Arg Gln His Pro Gly Lys Gly Leu Glu
        35                  40                  45

Trp Ile Gly Tyr Ile Tyr Tyr Ser Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Asp Glu Trp Glu Leu Arg Gly Phe Asp Tyr Trp Gly Gln
            100                 105                 110

Gly Thr Leu Val Thr Val Ser Ser
        115                 120

<210>  38
<211>  113
<212>  PRT
<213>  artificial sequence

<220>
<223>  VL of 1.62.3-u1-3-IgG1K

<400>  38

Asp Ile Val Met Thr Gln Ser Pro Asp Ser Leu Ala Val Ser Leu Gly
1               5                   10                  15

Glu Arg Ala Thr Ile Asn Cys Lys Ser Ser Gln Ser Val Val Phe Ser
            20                  25                  30

Ser Asn Asn Lys Ile Ser Leu Ala Trp Tyr Gln Gln Lys Pro Gly Gln
        35                  40                  45

Pro Pro Lys Leu Leu Ile Tyr Trp Ser Ser Thr Arg Glu Ser Gly Val
    50                  55                  60

Pro Asp Arg Phe Ser Gly Ser Gly Ser Gly Thr Asp Phe Thr Leu Thr
65                  70                  75                  80

Ile Ser Ser Leu Gln Ala Glu Asp Val Ala Val Tyr Tyr Cys Gln Gln
                85                  90                  95

Tyr Tyr Ser Ser Pro Trp Thr Phe Gly Gln Gly Thr Lys Val Glu Ile
            100                 105                 110

Lys

<210>  39
<211>  122
<212>  PRT
<213>  artificial sequence

<220>
<223>  VH of 1.134.9-u1-IgG1L

<400>  39

Gln Val Gln Leu Gln Glu Ser Gly Pro Gly Leu Val Lys Pro Ser Gly
1               5                   10                  15

Thr Leu Ser Leu Thr Cys Ala Val Ser Gly Gly Ser Ile Ser Ser Tyr
            20                  25                  30

Asn Trp Trp Ser Trp Val Arg Gln Pro Pro Gly Lys Gly Leu Glu Trp
        35                  40                  45

Ile Gly Glu Ile Tyr His Gly Gly Asn Thr Asn Tyr Asn Pro Ser Leu
    50                  55                  60

Lys Ser Arg Val Thr Met Ser Val Asp Asn Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Arg Ala Pro Gly Asp Trp Gly Gly Ser Pro Tyr Phe Asp Phe Trp
            100                 105                 110

Gly Gln Gly Thr Leu Val Thr Val Ser Ser
        115                 120

<210>  40
<211>  108
<212>  PRT
<213>  artificial sequence

<220>
<223>  VL of 1.134.9-u1-IgG1L

<400>  40

Ser Ser Glu Leu Thr Gln Asp Pro Ala Val Ser Val Ala Leu Gly Gln
1               5                   10                  15

Thr Val Arg Ile Thr Cys Gln Gly Asp Asn Leu Arg Thr Tyr Tyr Ala
            20                  25                  30

Ser Trp Tyr Gln Gln Lys Pro Gly Gln Ala Pro Ile Leu Leu Ile Tyr
        35                  40                  45

Gly Arg Asn Lys Arg Pro Ser Gly Ile Pro Asp Arg Phe Ser Gly Ser
    50                  55                  60

Ser Ser Gly Asn Thr Ala Ser Leu Thr Ile Thr Gly Ala Gln Ala Glu
65                  70                  75                  80

Asp Glu Ala Ala Tyr Tyr Cys Asn Ser Arg Asp Ser Ser Gly Asn Pro
                85                  90                  95

Val Val Phe Gly Gly Gly Thr Lys Leu Thr Val Leu
            100                 105

<210>  41
<211>  121
<212>  PRT

<213>  artificial sequence

<220>
<223>  VH of 1.186.19-u1-IgG1K

<400>  41

Gln Val His Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Tyr
            20                  25                  30

Tyr Met Gly Trp Ile Arg Gln Ala Pro Gly Gln Gly Leu Glu Trp Val
        35                  40                  45

Ser Tyr Ile Ser Gly Ser Gly Asn Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Asp Asp Thr Ala Val Tyr Phe Cys
                85                  90                  95

Ala Lys Glu Arg Gly Ala Ala Gly Ala Gly Trp Phe Asp Pro Trp Gly
            100                 105                 110

Gln Gly Thr Leu Val Thr Val Ser Ser
        115                 120


<210>  42
<211>  107
<212>  PRT
<213>  artificial sequence

<220>
<223>  VL of 1.186.19-u1-IgG1K

<400>  42

Asp Ile Gln Met Thr Gln Ser Pro Ser Ser Val Ser Ala Ser Val Gly
1               5                   10                  15

Asp Arg Val Thr Ile Thr Cys Arg Ala Ser Gln Gly Ile Ser Ser Trp
            20                  25                  30

Leu Ala Trp Tyr Gln Gln Lys Pro Gly Lys Ala Pro Lys Leu Leu Ile
        35                  40                  45

Tyr Ala Ala Ser Ser Leu Gln Gly Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr His Cys Gln Gln Val Asn Ser Phe Pro Trp
                85                  90                  95

Thr Phe Gly Gln Gly Thr Lys Val Glu Ile Lys
            100                 105

<210>  43
<211>  117
<212>  PRT
<213>  artificial sequence

<220>
<223>  VH of 1.214.23-u1-IgG1K

<400>  43

Gln Val Gln Leu Gln Glu Ser Gly Pro Gly Leu Val Lys Pro Ser Gly
1               5                   10                  15

Thr Leu Ser Leu Thr Cys Val Val Ser Gly Gly Ser Ile Ser Asn Arg
            20                  25                  30

Asn Trp Trp Ser Trp Val Arg Gln Pro Pro Gly Lys Gly Leu Glu Trp
        35                  40                  45

Ile Gly Glu Ile Phe His Ser Gly Asn Thr Asn Tyr Asn Pro Ser Leu
    50                  55                  60

Lys Ser Arg Val Thr Ile Ser Val Asp Lys Ser Lys Asn Gln Phe Ser
65                  70                  75                  80

Leu Lys Val Asn Ser Val Thr Ala Ala Asp Thr Ala Val Tyr Tyr Cys
                85                  90                  95

Ala Lys Ser Phe Ala Val Ala Leu Asp Ser Trp Gly Gln Gly Thr Leu
            100                 105                 110

Val Thr Val Ser Ser
        115

<210>  44
<211>  107
<212>  PRT
<213>  artificial sequence

<220>
<223>  VL of 1.214.23-u1-IgG1K

<400>  44

Asp Ile Gln Leu Thr Gln Ser Pro Ser Phe Leu Ser Ala Ser Val Gly
1               5                   10                  15

Asp Arg Val Thr Ile Thr Cys Arg Ala Ser Gln Asp Ile Asn Ser Tyr
            20                  25                  30

Leu Ala Trp Cys Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Thr Gly Ser Gly Thr Glu Phe Thr Leu Thr Ile Ser Ser Leu Gln Pro
65                  70                  75                  80

Glu Asp Phe Ala Thr Tyr Tyr Cys Gln Gln Leu Phe Ser Tyr Pro Ile
                85                  90                  95

Thr Phe Gly Gln Gly Thr Arg Leu Glu Ile Lys
            100                 105

<210>  45
<211>  363
<212>  DNA
<213>  artificial sequence

<220>
<223>  VH of 1.7.10-u1-IgG1K

<400>  45
caggtgcacc tggtggagtc tgggggaggc ttggtcaagc ctggagggtc cctgagactc      60

tcctgtgcag cctctggatt caccttcagt gactactaca tgagctggat ccgccaggct     120

ccagggaagg ggctggagtg ggtttcatac attagtggta gtggtaacac catttactac     180

gcagactctg tgaagggccg attcaccatc tccagggaca acgccaagaa ctcactgtat     240

ctgcaaatga acagcctgag agccgacgac acggccgtat atttctgtgc gagagagaga     300

ggagcagctg gtacagggtg gttcgacccc tggggccagg gaaccctggt caccgtctcc     360

tca                                                                   363

<210>  46
<211>  321
<212>  DNA
<213>  artificial sequence

<220>
<223>  VL of 1.7.10-u1-IgG1K

<400>  46
gacatccaga tgacccagtc tccatcttcc gtgtctgcat ctgtaggaga cagagtcacc      60

atcacttgtc gggcgagtca gggtattagc agctggttag cctggtatca gcagaaacca     120

gggaaagccc ctaagctcct gatctatgct gcatccagtt tgcaaggtgg ggtcccatca     180

aggttcagcg gcagtggatc tgggacagat ttcactctca ccatcagcag cctgcagcct     240

gaagattttg caacttacta ttgtcaacag gttaacagtt tcccgtggac gttcggccaa     300

gggaccaagg tggaaatcaa a                                               321


<210>  47
<211>  360
<212>  DNA
<213>  artificial sequence

<220>
<223>  VH of 1.62.3-u1-IgG1K

<400>  47
caggtgcagc tgcaggagtc gggcccagga ctggtgaagc cttcacagac cctgtccctc      60

acctgcactg tctctggtgg ctccatcagt aatggtggtt actactggag ctggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaaac tgagctctgt gactgccgcg gacacggccg tgtattactg tgcgagagat     300

gagtgggagc tacgggggtt tgactactgg ggccagggaa ccctggtcac cgtctcctca     360


<210>  48
<211>  339
<212>  DNA
<213>  artificial sequence

<220>
<223>  VL of 1.62.3-u1-IgG1K

<400>  48
gacatcgtga tgacccagtc tccagactcc ctggctgtgt ctctgggcga gagggccacc      60

atcaactgca agtccagcca gagtgttgta ttcagctcca acaataagat ctgcttagct     120

tggtaccagc agaaaccagg acagcctcct aagctgctca tttactggtc atctacccgg     180

gaatccgggg tccctgaccg attcagtggc agcgggtctg ggacagattt cactctcacc     240

atcagcagcc tgcaggctga agatgtggca gtttattact gtcagcaata ttatagttct     300

ccgtggacgt tcggccaagg gaccaaggtg gaaatcaaa                            339


<210>  49
<211>  360
<212>  DNA
<213>  artificial sequence

<220>
<223>  VH of 1.62.3-u1-3-IgG1K

<400>  49
caggtgcagc tgcaggagtc gggcccagga ctggtgaagc cttcacagac cctgtccctc      60

acctgcactg tctctggtgg ctccatcagt aatgccggtt actactggag ctggatccgc     120

cagcacccag ggaagggcct ggagtggatt gggtacatct attacagtgg gagcacctac     180

tacaacccgt ccctcaagag tcgagttacc atatcagtag acacgtctaa gaaccagttc     240

tccctgaaac tgagctctgt gactgccgcg gacacggccg tgtattactg tgcgagagat     300

gagtgggagc tacgggggtt tgactactgg ggccagggaa ccctggtcac cgtctcctca     360


<210>  50
<211>  339
<212>  DNA
<213>  artificial sequence

<220>
<223>  VL of 1.62.3-u1-3-IgG1K

<400>  50
gacatcgtga tgacccagtc tccagactcc ctggctgtgt ctctgggcga gagggccacc      60

atcaactgca agtccagcca gagtgttgta ttcagctcca acaataagat cagcttagct     120

tggtaccagc agaaaccagg acagcctcct aagctgctca tttactggtc atctacccgg     180

gaatccgggg tccctgaccg attcagtggc agcgggtctg ggacagattt cactctcacc     240

atcagcagcc tgcaggctga agatgtggca gtttattact gtcagcaata ttatagttct     300

ccgtggacgt tcggccaagg gaccaaggtg gaaatcaaa                            339


<210>  51
<211>  366
<212>  DNA
<213>  artificial sequence

<220>
<223>  VH of 1.134.9-u1-IgG1L

<400>  51
caggtgcagc tgcaggagtc gggcccagga ctggtgaagc cttcggggac cctgtccctc      60

acctgcgctg tctctggtgg ctccatcagt agttataact ggtggagttg ggtccgccag     120

cccccaggga agggactgga gtggattggg gaaatctatc atggtgggaa caccaactac     180

aacccgtccc tcaagagtcg agtcaccatg tcagtagaca actccaagaa ccagttctcc     240

ctgaagctga gctctgtgac cgccgcggac acggccgtat attactgtgc gagagccccc     300

ggggactggg gaggttcccc ctattttgac ttctggggcc agggaaccct ggtcaccgtc     360

tcctca                                                                366

<210> 52
<211> 336
<212> DNA
<213> artificial sequence

<220>
<223> VL of 1.134.9-u1-IgG1L

<400> 52
ggttctgtgg tttcttctga actgactcag gaccctgctg tgtctgtggc cttgggacag 60

acagtcagga tcacatgcca gggagacaac ctcagaacct attatgcaag ctggtaccag 120

cagaagccag gacaggcccc tatacttctc atctatggta gaaacaagcg gccctcaggg 180

atcccagacc gattctctgg ctccagctcg ggaaacacag cttccttgac catcactggg 240

gctcaggcgg aagatgaggc tgcgtactac tgtaactccc gggacagcag tggtaatcct 300

gtggtattcg gcggagggac caagctgacc gtccta 336


<210> 53
<211> 363
<212> DNA
<213> artificial sequence

<220>
<223> VH of 1.186.19-u1-IgG1K

<400> 53
caggtgcacc tggtggagtc tgggggaggc ttggtcaagc ctggagggtc cctgagactc 60

tcctgtgcag cctctggatt caccttcagt gactactaca tgggctggat ccgccaggct 120

ccagggcagg ggctggagtg ggtttcatac attagtggta gtggtaacac catttactac 180

gcagactctg tgaagggccg attcaccatc tccagggaca acgccaagaa ctcactgtat 240

ctgcaaatga acagcctgag agccgacgac acggccgttt atttctgtgc gaaagagaga 300

ggagcagctg gtgcaggtgg gttcgacccc tggggccagg gaaccctggt caccgtctcc 360

tca 363


<210> 54
<211> 321
<212> DNA
<213> artificial sequence

<220>
<223> VL of 1.186.19-u1-IgG1K

<400> 54
gacatccaga tgacccagtc tccatcttcc gtgtctgcat ctgtaggaga cagagtcacc 60

atcacttgtc gggcgagtca gggtattagc agctggttag cctggtatca gcagaaacca 120

gggaaagccc ctaagctcct gatctatgct gcatccagtt tgcaaggtgg ggtcccatca 180

aggttcagcg gcagtggatc tgggacagat ttcactctca ccatcagcag cctgcagcct 240

gaagattttg caacttacca ttgtcaacag gttaacagtt tcccgtggac gttcggccaa 300

gggaccaagg tggaaatcaa a 321

<210> 55
<211> 351
<212> DNA
<213> artificial sequence

<220>
<223> VH of 1.214.23-u1-IgG1K
<400> 55
caggtgcagc tgcaggagtc gggcccagga ctggtgaagc cttcggggac cctgtccctc 60

acctgtgttg tctccggtgg ctccatcagc aatagaaact ggtggagttg ggtccgccag 120

cccccaggga aggggctgga gtggattggg gaaatctttc atagtgggaa caccaactac 180

aacccgtccc tcaagagtcg cgtcaccata tcagtagaca agtccaagaa ccagttctcc 240

ctgaaggtga actctgtgac cgccgcggac acggccgtgt attactgtgc gaaatccttt 300

gcagtggccc ttgactcctg gggccaggga accctggtca ccgtctcctc a 351

<210> 56
<211> 321
<212> DNA
<213> artificial sequence

<220>
<223> VL of 1.214.23-u1-IgG1K

<400> 56
gacatccagt tgacccagtc tccatccttc ctgtctgcat ctgtaggaga cagagtcacc 60

atcacttgcc gggccagtca ggacattaac agttatttag cctggtgtca gcaaaaacca 120

gggaaagccc ctaagctcct gatctatgct gcatcctctt tgcaaagtgg ggtcccatca 180

aggttcagcg gcactggatc tgggacagag ttcactctca caatcagcag cctgcagcct 240

gaagattttg caacttatta ctgtcaacag ctttttagtt acccgatcac cttcggccaa 300

gggacacgac tggagattaa a 321